(19) 

**Europäisches Patentamt**
**European Patent Office**
**Office européen des brevets**

(11) **EP 4 439 065 A1**

(12) **EUROPEAN PATENT APPLICATION**
published in accordance with Art. 153(4) EPC

(43) Date of publication:
02.10.2024 Bulletin 2024/40

(21) Application number: 22898625.3

(22) Date of filing: 24.11.2022

(51) International Patent Classification (IPC):
*G01N 33/543* (2006.01)    *G01N 33/569* (2006.01)

(52) Cooperative Patent Classification (CPC):
G01N 33/569; G01N 33/543; G01N 33/54388;
G01N 33/56911

(86) International application number:
PCT/JP2022/043411

(87) International publication number:
WO 2023/095843 (01.06.2023 Gazette 2023/22)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 24.11.2021  JP 2021190601
24.11.2021  JP 2021190597
06.12.2021  JP 2021198120

(71) Applicant: Asahi Kasei Kabushiki Kaisha
Tokyo 1000006 (JP)

(72) Inventors:
• TAKAHASHI, Katsuyoshi
Tokyo 100-0006 (JP)
• MAEHANA, Koji
Tokyo 100-0006 (JP)
• ODA, Kotaro
Tokyo 100-0006 (JP)
• ANDO, Yu
Tokyo 100-0006 (JP)
• SUGATA, Mika
Tokyo 100-0006 (JP)

(74) Representative: Forstmeyer, Dietmar et al
Boeters & Lieck
Oberanger 32
80331 München (DE)

(54) **IMMUNOCHROMATOGRAPHY DEVICE AND PRODUCTION METHOD THEREOF, AND METHOD FOR DETECTING TARGET BACTERIUM USING SAME**

(57) An immunochromatography device 10 that holds a labeling antibody-carrying member, which elutably carries an antibody for labeling, disposed thereon in such a manner that at least a part of the labeling antibody-carrying member is immersed in a specimen solution. Owing to this arrangement, the labeling antibody elutes and diffuses into the specimen solution when the immunochromatography device 10 is immersed in the specimen solution. By diffusing the labeling antibody into the specimen solution, an antigen-antibody reaction occurs in the specimen solution so that the reaction time can be prolonged compared with the case of using conventional techniques, thereby improving the detection sensitivity.

**EP 4 439 065 A1**

Fig. 1

**Description**

**TECHNICAL FIELD**

The present invention relates to an immunochromatographic device for detecting a target bacterium in a sample liquid based on an antigen-antibody reaction detection, a method for producing the immunochromatographic device, and a method for detecting a target bacterium using the immunochromatographic device.

**BACKGROUND ART**

Conventional methods for detecting the presence or absence of various bacteria in various samples include the culture method, in which samples are cultured to confirm bacterial growth, and the ATP method, which detects intracellular ATP (adenosine triphosphate) of bacteria in samples. However, the former, culture method has the problem that it requires a long time for cultivation and takes a lot of work. The latter, ATP method has the problem that since ATP is present not only in bacterial cells but also in eukaryotic cells (animal and plant cells), it is difficult to identify bacteria from eukaryotic cells derived from living organisms, food and drink, and the environment.

Therefore, in order to test for bacterial contamination in samples quickly and with high sensitivity, simple measuring devices using chromatography (chromatographic devices) have become popular. Such a device can quickly and easily detect a target substance to be detected in a sample by using a specific reaction with a specific binding substance, which specifically binds to the target substance. Various substances are used as target substances to be detected. Among them, immunochromatographic systems, which use antibodies as specific binding substances, can detect various target substances and are used in various fields such as research, medicine, and food.

A typical mode of immunochromatographic systems widely used is the lateral-flow mode. Immunochromatographic systems of lateral-flow mode are generally classified into two main types: dipstick type and cassette type. In the food or livestock industry, where simple operation and low cost are required, dipstick-type immunochromatographic systems are used, which do not require a housing and can be used for testing simply by inserting a strip into a sample solution (Patent Literature 1: JP6325895 B). There are also known techniques for extracting the target substance to be detected by lysing bacteria with a bacteriolysis agent or enzyme, with the aim of increasing the sensitivity of bacterial detection (Patent Literature 2: JP6280567 B; Patent Literature 3: JP6314156 B; Patent Literature 4: JP6370807 B). Also known is the technique of incorporating a bacteriolysis enzyme into an immunochromatographic strip for highly sensitive detection of the target substance to be detected (Patent Literature 5: JP6081457 B). Such immunochromatographic devices are usually manufactured by fixing a capture reagent containing a specific binding substance, which specifically binds to the target substance to be detected, to the detection region of a membrane carrier for chromatographic development.

Examples of target samples to be measured using such an immunochromatographic systems include milk samples from livestock and other animals. Livestock milk is not sterile and may be contaminated with some microorganisms due to diseases or environments. Diseases caused by microbial infections of the udder often result in a large number of microorganisms being excreted in the milk. An example of typical livestock diseases caused by microbial infection is mastitis, which is an inflammation of the milk duct system and mammary tissue caused primarily by the invasion, establishment, and growth of microorganisms in the udder. While mastitis affects many animals, bovine mastitis, especially in dairy cows, is one of the most important diseases for dairy farmers as it is estimated to affect 15-40% of all dairy cows. When a dairy cow contracts mastitis, milk synthesis is impaired, resulting in decreased milk production and possibly cessation of lactation. In addition, it causes significant economic losses to the dairy farmer in terms of treatment costs and penalties on milk price due to decreased milk quality. On top of that, the labor burden on the dairy farmer is increased by, e.g., milking each mastitisaffected quarter separately to prevent infection.

Mastitis is caused by a variety of microorganisms. Examples of the causative organisms include coliforms such as Escherichia coli and Klebsiella coli, which cause systemic symptoms such as fever and local symptoms such as heat sensation, swelling, and induration of the udder. The culture method is widely used to detect coliforms in milk. The culture method is not suitable for rapid identification of causative organisms because it takes several days to obtain results. On the other hand, identification based on an antigen-antibody reaction using antibodies against specific components of the causative organisms, especially immunochromatography, is widely used in homes and examination rooms as a quick and simple testing method because the results can be determined in several ten minutes.

There are various detection methods for milk samples using immunochromatography as described above. However, milk samples have their unique problem in that they contain fat globules in large quantities, which may inhibit chromatographic membrane development and therefore need to be removed (Patent Literature 6: JP5693938 B).

## LIST OF CITATIONS

### Patent Literature

[Patent Literature 1] JP6325895 B
[Patent Literature 2] JP6280567 B
[Patent Literature 3] JP63 14156 B
[Patent Literature 4] JP6370807 B
[Patent Literature 5] JP6081457 B
[Patent Literature 6] JP5693938 B

## SUMMARY OF INVENTION

### Problem to Be Solved by the Invention

Conventional immunochromatographic systems are required to improve the detection sensitivity and/or detection efficiency of the target substance to be detected. Accordingly, an objective of the present invention is to increase the detection sensitivity and/or detection efficiency of the target substance to be detected by an immunochromatographic device.

Conventional immunochromatographic devices are manufactured by fixing a capture reagent containing a specific binding substance, which specifically binds to the target substance, to the detection region of the membrane carrier for chromatographic development. Upon investigation, the present inventors have found that the sensitivity of the chromatographic devices manufactured in this manner increases over time during the storage period after manufacture. Accordingly, an objective of the present invention is to reduce the change in sensitivity of immunochromatographic devices over time during the storage period from the viewpoint of quality control during the manufacture of immunochromatographic devices.

When a conventional immunochromatographic system is used for milk samples, there is a need to improve the poor development caused by members for removing fat globules (hereinafter also referred to as "filter members"). Accordingly, an objective of the present invention is to improve the poor development caused by the fat globule removers when the immunochromatographic system is used for milk samples.

### Means to Solve the Problem

As a result of diligent research, the present applicants have found that when an immunochromatographic device is used, the labeling antibody can be diffused into the sample liquid to cause the antigen-antibody reaction in the sample liquid, whereby a longer reaction time can be ensured than with conventional methods, resulting in improved detection sensitivity and/or detection efficiency. The present applicants have also found that when the specific binding substance is fixed to the detection region of a membrane carrier for chromatographic development, the membrane carrier be dried at a higher temperature and for a longer time than the drying temperature and time conventionally used, whereby the change in the sensitivity over time during the storage period of the resulting immunochromatographic system can be reduced. The present applicants have further found that in an immunochromatographic system with a member for removing fat globules for milk samples, the volume of the liquid to be developed with the chromatographic membrane (hereinafter also referred to as "total water absorption volume Y") and the overlap of multiple fat globule removers have a significant impact on the performance of the development with the chromatographic membrane during use, and that optimizing these parameters can help improve the poor development caused by the fat globule removal removers. As a result of further diligent research based on these findings, the present applicants have completed the present invention.

Specifically, some aspects of the present invention include the following.

[Aspect 1] A method for detecting a target bacterium in a sample liquid based on an antigen-antibody reaction using an immunochromatographic device, comprising the steps of:

1) immersing at least a portion of a labeling antibody-attached member, to which a labeling antibody is attached, in the sample liquid to thereby cause at least a portion of an antigen-antibody reaction between an antigen derived from the target bacterium and the labeling antibody in the sample liquid to form a first complex; and
2) causing an antigen-antibody reaction between a capture antibody immobilized on the immunochromatographic device and the first complex to form a second complex, thereby detecting the bacterium based on the label of the labeling antibody.

[Aspect 2] The method according to Aspect 1, wherein together with the at least portion of the labeling antibody-attached member, at least a portion of a bacteriolysis agent-attached member, to which a bacteriolysis agent is attached, is immersed in the sample liquid.

[Aspect 3] The method according to Aspect 2, wherein the at least portion of the labeling antibody-attached member and the at least portion of the bacteriolysis agent-attached member are immersed in the sample liquid at the same time.

[Aspect 4] The method according to any one of Aspects 1 to 3, further comprising agitating the sample liquid after the immersion and before the detection.

[Aspect 5] The method according to any one of Aspects 1 to 4, wherein the antigen derived from the target bacterium is an intracellular antigen.

[Aspect 6] The method according to Aspect 5, wherein the intracellular antigen is a ribosome protein.

[Aspect 7] The method according to Aspect 6, wherein the ribosome protein is a L7/L12 protein.

[Aspect 8] The method according to any one of Aspects 1 to 7, wherein after the immersion, at least a portion of the labeling antibody attached to the labeling antibody-attached member and the bacteriolysis agent attached to the bacteriolysis agent-attached member dissolves in the sample liquid.

[Aspect 9] An immunochromatographic device for detecting a target bacterium in a sample liquid based on an antigen-antibody reaction detection, comprising:

(a) a labeling antibody-attached member, to which is attached a labeling antibody, which forms a first complex with an antigen derived from the target bacterium based on an antigen-antibody reaction; and
(b) a chromatographic development member, which contains an immersing region, which is to be immersed in the sample liquid, and a detection region, on which is immobilized a capture antibody, which forms a second complex with the first complex based on an antigen-antibody reaction,

wherein the immunochromatographic device is configured such that when it is used, the at least portion of the labeling antibody-attached member and the immersing region of the chromatographic development member are both immersed in the sample liquid, and
wherein the detection of the target bacterium in the sample liquid by the immunochromatographic device is carried out by detecting the second complex captured in the detection region of the chromatographic development member based on the label of the labeling antibody detection.

[Aspect 10] The immunochromatographic device according to Aspect 9, which is configured such that the at least portion of the labeling antibody-attached member is included in the immersing region.

[Aspect 11] The immunochromatographic device according to Aspect 9 or 10, further comprising:
(c) a bacteriolysis agent-attached member, to which a bacteriolysis agent is attached,
wherein the immunochromatographic device is configured such that when it is used, at least a portion of the bacteriolysis agent-attached member is immersed in the sample liquid.

[Aspect 12] The immunochromatographic device according to Aspect 11, wherein the bacteriolysis agent-attached member and the labeling antibody-attached member are formed as separate members.

[Aspect 13] The immunochromatographic device according to Aspect 11, wherein the bacteriolysis agent-attached member and the labeling antibody-attached member are formed as a single member.

[Aspect 14] The immunochromatographic device according to any one of Aspects 11 to 13, which is configured such that at least a portion of the bacteriolysis agent-attached member is included in the immersing region.

[Aspect 15] The immunochromatographic device according to any one of Aspects 11 to 14, wherein either or both of the bacteriolysis agent-attached member and the labeling antibody-attached member are provided separately from the chromatographic development member.

[Aspect 16] The immunochromatographic device according to any one of Aspects 11 to 15, further comprising:
(d) a sample contact member,
wherein the immunochromatographic device is configured such that when it is used, at least a portion of the sample contact member is immersed in the sample liquid.

**Effect of the Invention**

An embodiment of the present invention provides an excellent immunochromatographic device with improved detection sensitivity and/or detection efficiency of the target substance to be detected.

Another embodiment of the present invention provides an excellent immunochromatographic device with reduced sensitivity changes over time during storage.

Still another embodiment of the present invention provides an excellent immunochromatographic device to be used for milk samples, in which the development failure caused by the fat globule removal member has been improved.

## BRIEF EXPLANATION OF FIGURES

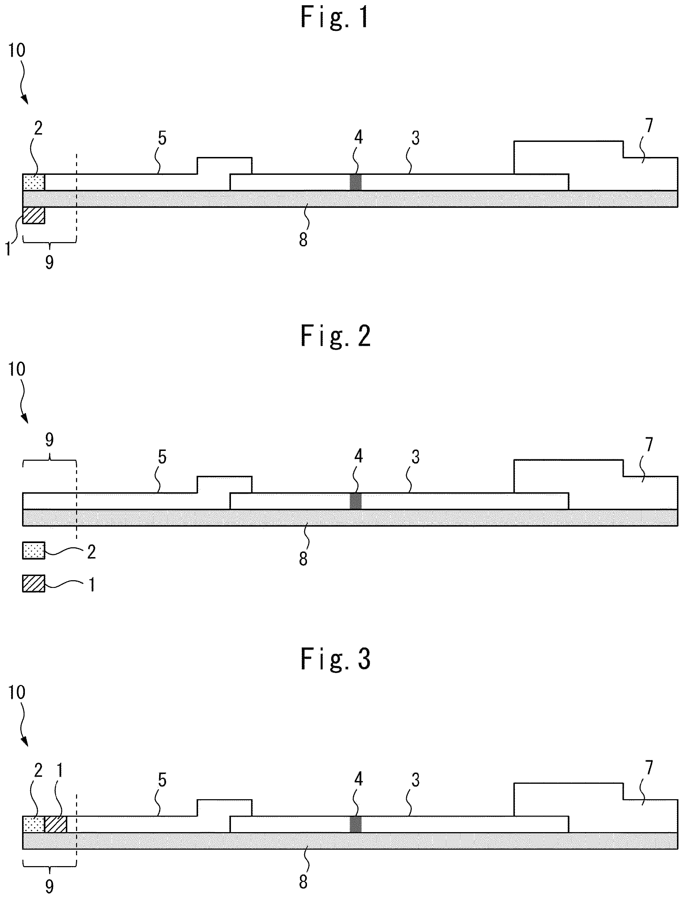

[Figure 1] Figure 1 is a cross-sectional view schematically showing the configuration of an immunochromatographic device according to an embodiment of the present invention (Example I-1, I-7, and 1-13). A labeling antibody-attached member 1 is arranged on a base material 8 on the back side of the immunochromatographic device 10, while a bacteriolysis agent-attached member 2 is arranged on the base material 8 on the front side of the immunochromatographic device 10. The labeling antibody-attached member 1 and the bacteriolysis agent-attached member 2 are exposed to the outside and arranged within an immersing region 9.

[Figure 2] Figure 2 is a cross-sectional view schematically showing the configuration of an immunochromatographic device according to an embodiment of the present invention (Example I-2, I-8, and 1-14). A labeling antibody-attached member 1 and a bacteriolysis agent-attached member 2 are provided separately of the immunochromatographic device 10, and to be immersed in the sample liquid.

[Figure 3] Figure 3 is a cross-sectional view schematically showing the configuration of an immunochromatographic device according to an embodiment of the present invention (Example 1-3, 1-9, and 1-15). A labeling antibody-attached member 1 and a bacteriolysis agent-attached member 2 are arranged on a base material 8 on the front side of the immunochromatographic device 10. The labeling antibody-attached member 1 and the bacteriolysis agent-attached member 2 are exposed to the outside and arranged within an immersing region 9.

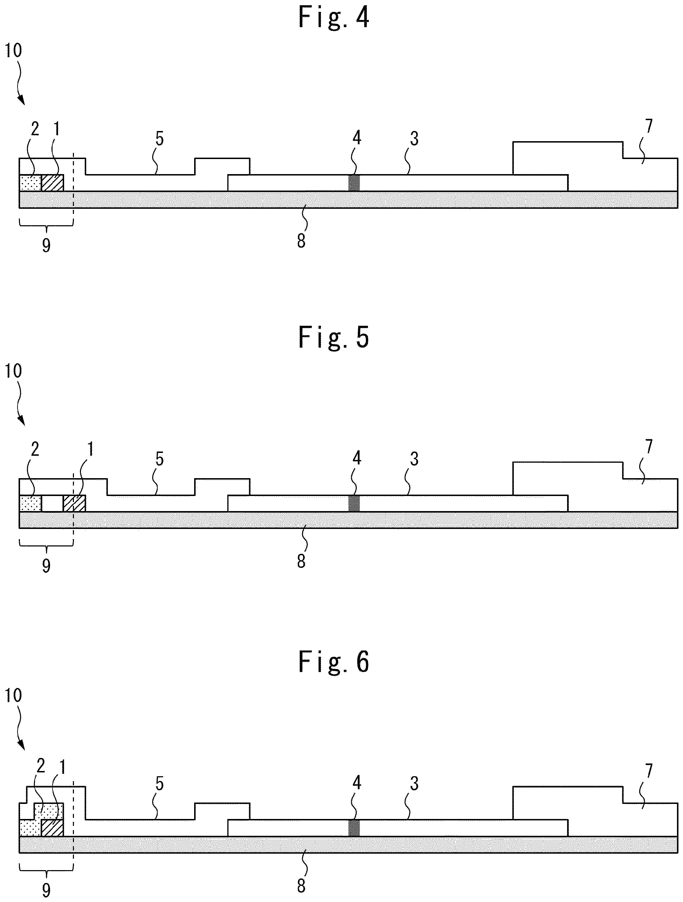

[Figure 4] Figure 4 is a cross-sectional view schematically showing the configuration of an immunochromatographic device according to an embodiment of the present invention (Example 1-4, I-10, and I-16). A labeling antibody-attached member 1 and a bacteriolysis agent-attached member 2 are arranged in contact with each other on a base material 8 on the front side of the immunochromatographic device 10. A sample contact member 5 is arranged so as to cover the labeling antibody-attached member 1 and the bacteriolysis agent-attached member 2. The labeling antibody-attached member 1 and the bacteriolysis agent-attached member 2 are arranged within an immersing region 9.

[Figure 5] Figure 5 is a cross-sectional view schematically showing the configuration of an immunochromatographic device according to an embodiment of the present invention (Example I-5, I-11, and I-17). the labeling antibody-attached member 1 and the bacteriolysis agent-attached member 2 are arranged apart from each other on a base material 8 on the front side of the immunochromatographic device 10. A sample contact member 5 is arranged so as to cover the labeling antibody-attached member 1 and the bacteriolysis agent-attached member 2. At least a portion of the labeling antibody-attached member 1 and the bacteriolysis agent-attached member 2 are arranged within an immersing region 9.

[Figure 6] Figure 6 is a cross-sectional view schematically showing the configuration of an immunochromatographic device according to an embodiment of the present invention (Example I-6, I-12, and I-18). A labeling antibody-attached member 1 and a bacteriolysis agent-attached member 2 are arranged in contact with each other on a base material 8 on the front side of the immunochromatographic device 10. The bacteriolysis agent-attached member 2 is arranged over the base material 8 and the labeling antibody-attached member 1 so as to completely cover the labeling antibody-attached member 1. A sample contact member 5 is arranged so as to cover the bacteriolysis agent-attached member 2. The labeling antibody-attached member 1 and the bacteriolysis agent-attached member 2 are arranged within an immersing region 9.

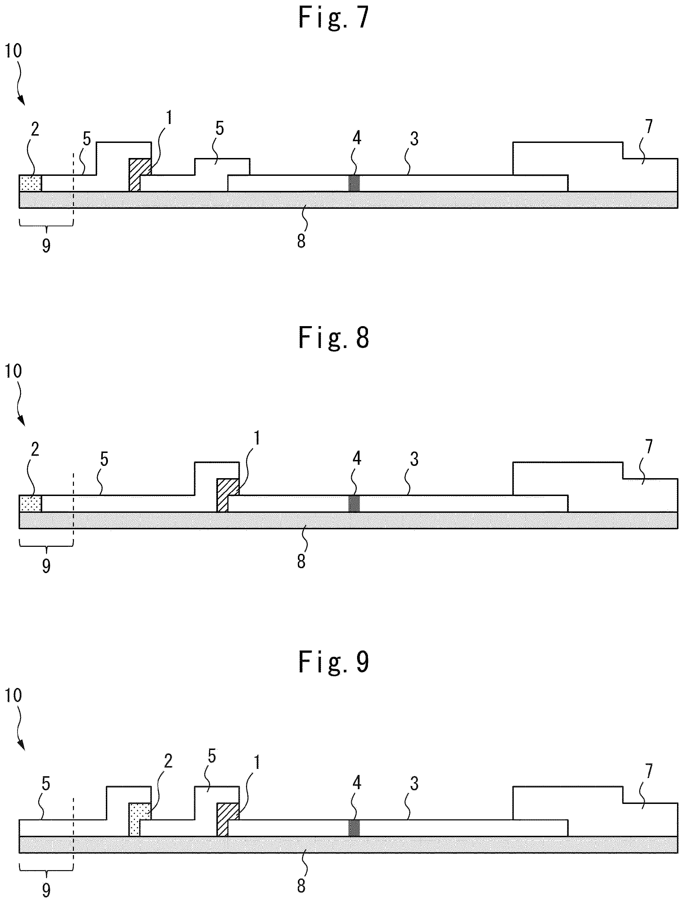

[Figure 7] Figure 7 is a cross-sectional view schematically showing the configuration of an immunochromatographic device according to Comparative Example I-1, I-5, and I-9. the labeling antibody-attached member 1 and the bacteriolysis agent-attached member 2 are arranged apart from each other on a base material 8 on the front side of the immunochromatographic device 10. Two separate sample contact members 5 are provided and arranged so as to partially overlap with each other. The labeling antibody-attached member 1 is arranged so as to overlap with a portion of the sample contact member on the downstream side 5 and the base material 8, and the sample contact member on the upstream side is arranged so as to cover the labeling antibody-attached member 1. The bacteriolysis agent-attached member 2 is arranged so as to be exposed to the outside. The labeling antibody-attached member 1 is arranged outside an immersing region 9, while the bacteriolysis agent-attached member 2 is arranged within the immersing region 9.

[Figure 8] Figure 8 is a cross-sectional view schematically showing the configuration of an immunochromatographic device according to Comparative Example I-2, I-6, and I-10. A labeling antibody-attached member 1 and a bacteriolysis agent-attached member 2 are arranged apart from each other on a base material 8 on the front side of the immunochromatographic device 10. The labeling antibody-attached member 1 is arranged so as to overlap with a portion of a membrane carrier for chromatographic development 3 and the base material 8, and a sample contact member 5 is arranged so as to cover the labeling antibody-attached member 1. The bacteriolysis agent-attached member 2 is exposed to the outside. The labeling antibody-attached member 1 is arranged outside an immersing region 9, while the bacteriolysis agent-attached member 2 is arranged within the immersing region 9.

[Figure 9] Figure 9 is a cross-sectional view schematically showing the configuration of an immunochromatographic device according to Comparative Example I-3, I-7, and I-11. A labeling antibody-attached member 1 and a bacteriolysis agent-attached member 2 are arranged apart from each other on a base material 8 on the front side of the immunochromatographic device 10. Two separate sample contact members 5 are provided and arranged so as to partially overlap with each other. A labeling antibody-attached member 1 is arranged so as to overlap with a portion of a membrane carrier for chromatographic development 3 and the base material 8, and the sample contact member on the downstream side 5 is arranged so as to cover the labeling antibody-attached member 1. The bacteriolysis agent-attached member 2 is arranged so as to overlap with a portion of the sample contact member on the downstream side 5 and the base material 8, and the sample contact member on the upstream side 5 is arranged so as to cover the bacteriolysis agent-attached member 2. The labeling antibody-attached member 1 and the bacteriolysis agent-attached member 2 are both arranged outside an immersing region 9.

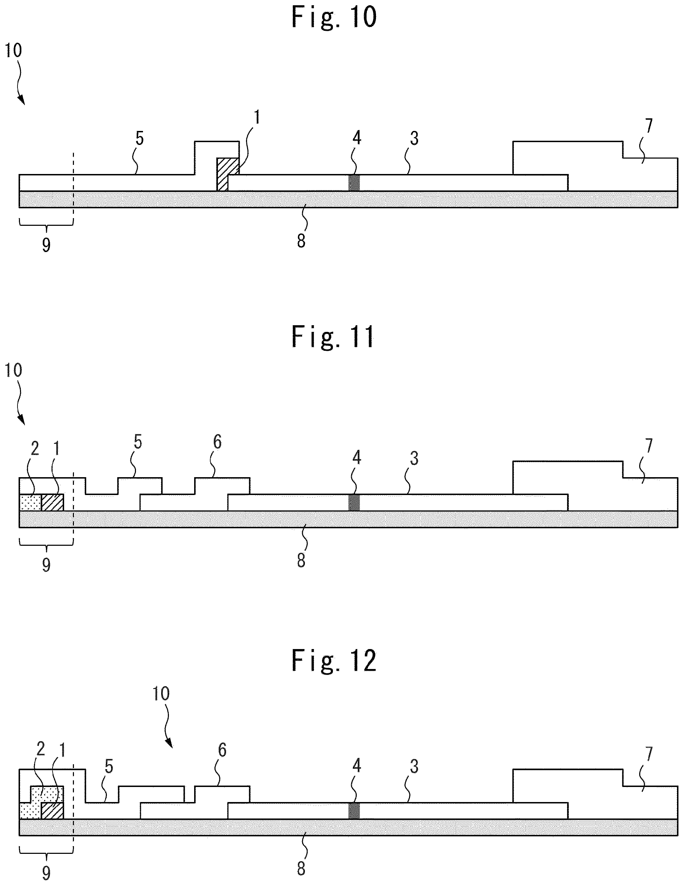

[Figure 10] Figure 10 is a cross-sectional view schematically showing the configuration of an immunochromatographic device according to Comparative Example I-4 and I-8. A membrane carrier for chromatographic development 3 is arranged on a base material 8 on the front side of the immunochromatographic device 10, and the labeling antibody-attached member 1 is arranged so as to overlap with a portion on the upstream side of the membrane carrier for chromatographic development 3 and the base material 8, and a sample contact member 5 is arranged so as to overlap with a portion on the upstream side of the labeling antibody-attached member 1 and the base material 8. The labeling antibody-attached member 1 is arranged outside an immersing region 9, while no bacteriolysis agent-attached member 2 is provided.

[Figure 11] Figure 11 is a cross-sectional view schematically showing the configuration of an immunochromatographic device according to an embodiment of the present invention (Example I-19, I-21, and I-24). A labeling antibody-attached member 1 and a bacteriolysis agent-attached member 2 are arranged in contact with each other on a base material 8 on the front side of the immunochromatographic device 10. A filter member 6 is arranged so as to overlap with a portion of a membrane carrier for chromatographic development 3 and the base material 8, and a sample contact member 5 is arranged so as to overlap with a portion of the filter member 6 and the base material 8. The sample contact member 5 is arranged so as to cover the labeling antibody-attached member 1 and the bacteriolysis agent-attached member 2. The labeling antibody-attached member 1 and the bacteriolysis agent-attached member 2 are arranged within an immersing region 9.

[Figure 12] Figure 12 is a cross-sectional view schematically showing the configuration of an immunochromatographic device according to an embodiment of the present invention (Example I-20 and I-25). A labeling antibody-attached member 1 and a bacteriolysis agent-attached member 2 are arranged in contact with each other on a base material 8 on the front side of the immunochromatographic device 10. The bacteriolysis agent-attached member 2 is arranged so as to overlap with the base material 8 and the labeling antibody-attached member 1, and also to completely cover the labeling antibody-attached member 1. A sample contact member 5 is arranged so as to cover the bacteriolysis agent-attached member 2. The filter member 6 is arranged so as to overlap with a portion on the downstream side of the sample contact member 5 and the base material 8. The filter member 6 is also arranged so as to overlap with a portion on the upstream side of the membrane carrier for chromatographic development 3 and the base material 8. The labeling antibody-attached member 1 and the bacteriolysis agent-attached member 2 are arranged within an immersing region 9.

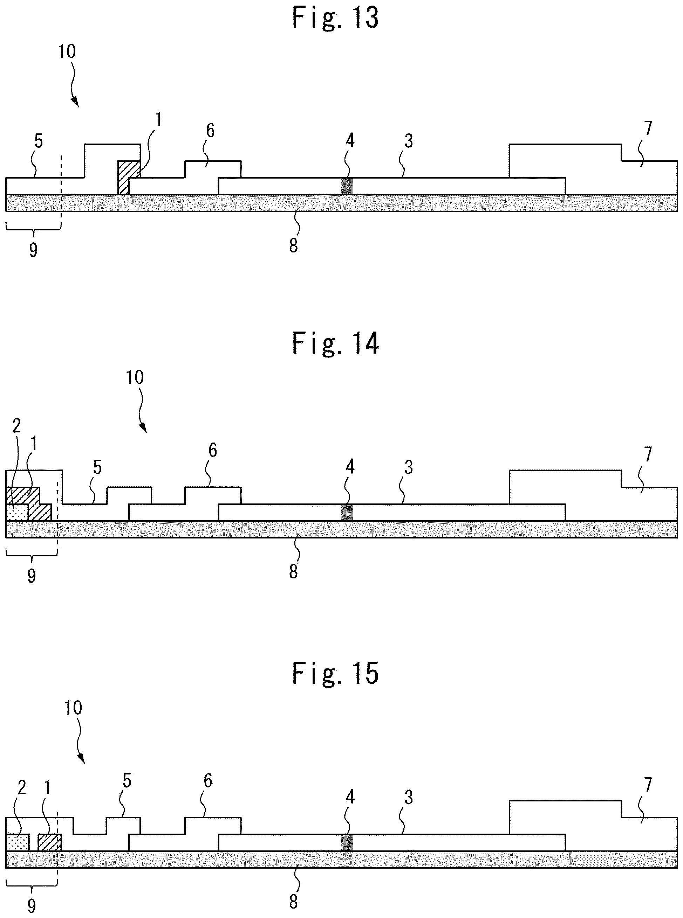

[Figure 13] Figure 13 is a cross-sectional view schematically showing the configuration of an immunochromatographic device according to Comparative Example I-12 and I-13. A membrane carrier for chromatographic development 3 is arranged on a base material 8 on the front side of the immunochromatographic device 10, and a filter member 6 is arranged so as to overlap with a portion on the upstream side of the membrane carrier for chromatographic development 3 and the base material 8. The labeling antibody-attached member 1 is arranged so as to overlap with a portion on the upstream side of the filter member 6 and the base material 8, and the sample contact member 5 is arranged so as to overlap with a portion on the upstream side of the labeling antibody-attached member 1 and the base material 8. The labeling antibody-attached member 1 is arranged outside an immersing region 9, while no bacteriolysis agent-attached member 2 is provided.

[Figure 14] Figure 14 is a cross-sectional view schematically showing the configuration of an immunochromatographic device according to Example I-22. A membrane carrier for chromatographic development 3 is arranged on a base material 8 on the front side of the immunochromatographic device 10, and the labeling antibody-attached member 1 and the bacteriolysis agent-attached member 2 are arranged in contact with each other. The labeling antibody-attached member 1 is arranged so as to overlap with the base material 8 and the bacteriolysis agent-attached member 2, and also to completely cover the bacteriolysis agent-attached member 2. A sample contact member 5 is also arranged so as to cover the labeling antibody-attached member 1. A filter member 6 is arranged so as to overlap with the base material 8 and a portion on the upstream side of the membrane carrier for chromatographic development 3, and a portion on the downstream side of the sample contact member 5 is arranged so as to overlap with a portion on the upstream side of the filter member 6. The labeling antibody-attached member 1 and the bacteriolysis agent-attached member 2 are arranged within an immersing region 9.

[Figure 15] Figure 15 is a cross-sectional view schematically showing the configuration of an immunochromatographic device according to Example I-23 and I-29. A membrane carrier for chromatographic development 3 is arranged on a base material 8 on the front side of the immunochromatographic device 10, and a labeling antibody-attached member 1 and a bacteriolysis agent-attached member 2 are arranged so as to be apart from each other. A sample contact member 5 is arranged so as to completely cover the labeling antibody-attached member 1 and the bacteriolysis agent-attached member 2. A filter member 6 is arranged so as to overlap with the base material 8 and a portion on the upstream side of the membrane carrier for chromatographic development 3, and a portion on the downstream side of the sample contact member 5 is arranged so as to overlap with a portion on the upstream side of the filter member 6. The labeling antibody-attached member 1 and the bacteriolysis agent-attached member 2 are arranged within an immersing region 9.

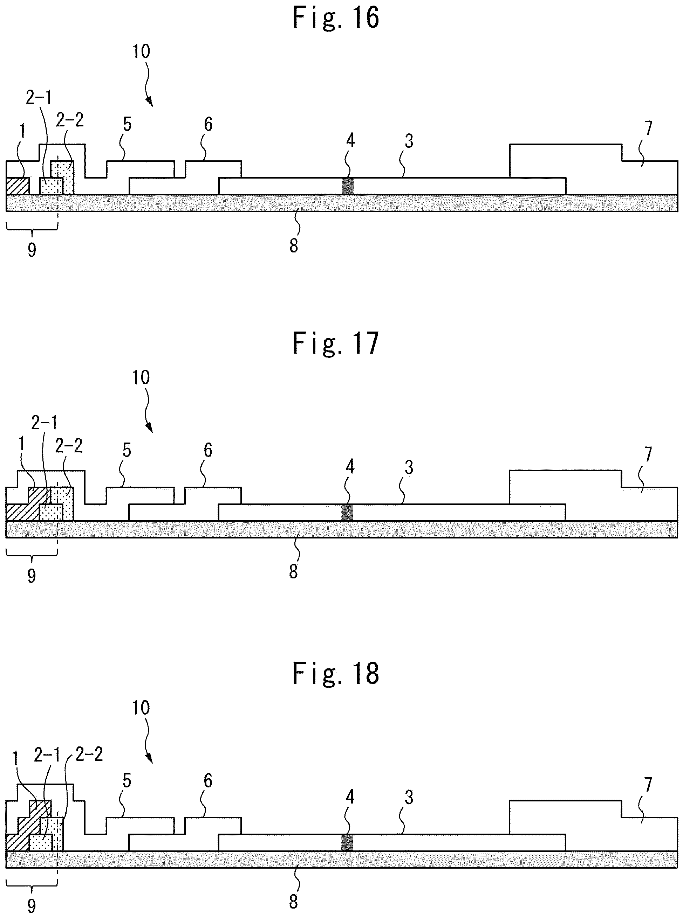

[Figure 16] Figure 16 is a cross-sectional view schematically showing the configuration of an immunochromatographic device according to Example I-26. A membrane carrier for chromatographic development 3 is arranged on a base material 8 on the front side of the immunochromatographic device 10, and a labeling antibody-attached member 1 and two bacteriolysis agent-attached members 2-1 and 2-2 are provided. The two bacteriolysis agent-attached members 2-1 and 2-2 are arranged such that a portion of the bacteriolysis agent-attached member 2-1 is covered by the bacteriolysis agent-attached member 2-2. The labeling antibody-attached member 1 is arranged so as to be apart from the two bacteriolysis agent-attached members 2-1 and 2-2. A sample contact member 5 is arranged so as to completely cover the labeling antibody-attached member 1 and the bacteriolysis agent-attached members 2-1 and 2-2. A filter member 6 is arranged so as to overlap with the base material 8 and a portion on the upstream side of the membrane carrier for chromatographic development 3, and a portion on the downstream side of the sample contact member 5 is arranged so as to overlap with a portion on the upstream side of the filter member 6. The labeling antibody-attached member 1 and bacteriolysis agent-attached members 2-1 and 2-2 are arranged within an immersing region 9.

[Figure 17] Figure 17 is a cross-sectional view schematically showing the configuration of an immunochromatographic device according to Example I-27. A membrane carrier for chromatographic development 3 is arranged on a base material 8 on the front side of the immunochromatographic device 10, and a labeling antibody-attached member 1 and two bacteriolysis agent-attached members 2-1 and 2-2 are provided. The two bacteriolysis agent-attached members 2-1 and 2-2 are arranged such that a portion of the bacteriolysis agent-attached member 2-1 is covered by the bacteriolysis agent-attached member 2-2. The labeling antibody-attached member 1 is arranged so as to completely cover the bacteriolysis agent-attached member 2-1, and also to be in contact with the bacteriolysis agent-attached member 2-2. A sample contact member 5 is arranged so as to completely cover the labeling antibody-attached member 1 and the bacteriolysis agent-attached member 2-2. A filter member 6 is arranged so as to overlap with the base material 8 and a portion on the upstream side of the membrane carrier for chromatographic development 3, and a portion on the downstream side of the sample contact member 5 is arranged so as to overlap with a portion on the upstream side of the filter member 6. The labeling antibody-attached member 1 and bacteriolysis agent-attached members 2-1 and 2-2 are arranged within an immersing region 9.

[Figure 18] Figure 18 is a cross-sectional view schematically showing the configuration of an immunochromatographic device according to Example I-28. A membrane carrier for chromatographic development 3 is arranged on a base material 8 on the front side of the immunochromatographic device 10, and a labeling antibody-attached member 1 and two bacteriolysis agent-attached members 2-1 and 2-2 are provided. The two bacteriolysis agent-attached members 2-1 and 2-2 are arranged such that a portion of the bacteriolysis agent-attached member 2-1 is covered by the bacteriolysis agent-attached member 2-2. The labeling antibody-attached member 1 is arranged so as to completely cover the bacteriolysis agent-attached member 2-1, and also to cover a portion of the bacteriolysis agent-attached member 2-2. A sample contact member 5 is arranged so as to completely cover the labeling antibody-attached member 1 and the bacteriolysis agent-attached member 2-2. A filter member 6 is arranged so as to overlap with the base material 8 and a portion on the upstream side of the membrane carrier for chromatographic development 3, and a portion on the downstream side of the sample contact member 5 is arranged so as to overlap with a portion on the upstream side of the filter member 6. The labeling antibody-attached member 1 and bacteriolysis agent-attached members 2-1 and 2-2 are arranged within an immersing region 9.

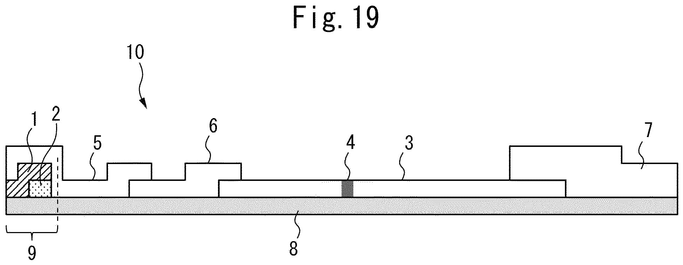

[Figure 19] Figure 19 is a cross-sectional view schematically showing the configuration of an immunochromatographic device according to Example I-30. A membrane carrier for chromatographic development 3 is arranged on a base material 8 on the front side of the immunochromatographic device 10, and the labeling antibody-attached member 1 and the bacteriolysis agent-attached member 2 are arranged in contact with each other. The labeling antibody-attached member 1 is arranged so as to overlap with the base material 8 and the bacteriolysis agent-attached member 2, and also to completely cover the bacteriolysis agent-attached member 2. A filter member 6 is arranged so as to span from the base material 8 to a portion on the upstream side of the membrane carrier for chromatographic development 3, and a sample contact member 5 is arranged so as to cover the labeling antibody-attached member 1 and span from a portion on the upstream side of the filter member 6 to the base material 8. The labeling antibody-attached member 1 and the bacteriolysis agent-attached member 2 are arranged within an immersing region 9.

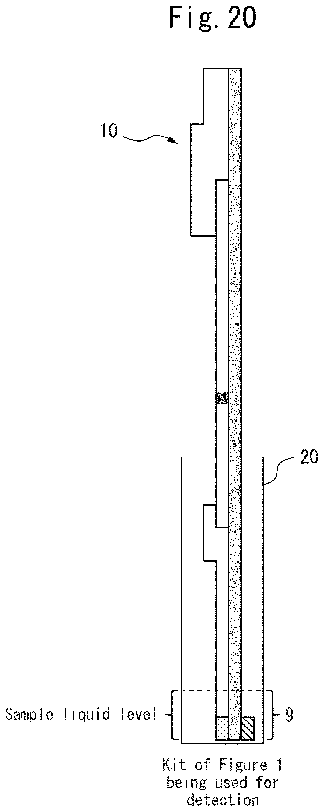

[Figure 20] Figure 20 schematically illustrates an embodiment in which the immunochromatographic device of Figure 1 is immersed in a sample liquid for use.

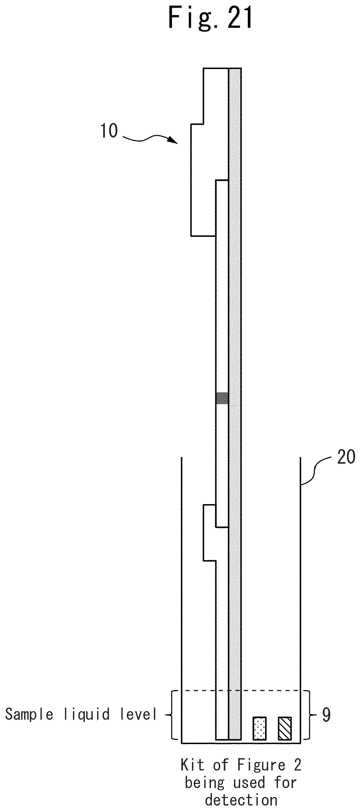

[Figure 21] Figure 21 schematically illustrates an embodiment in which the immunochromatographic device, the labeling antibody-attached member 1, and the bacteriolysis agent-attached member 2 of Figure 2 are immersed in a sample liquid for use.

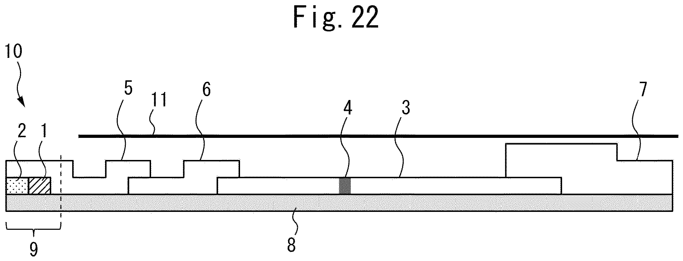

[Figure 22] Figure 22 shows a cross-sectional view of an example of a chromatographic device produced by a production method according to an embodiment of the present invention.

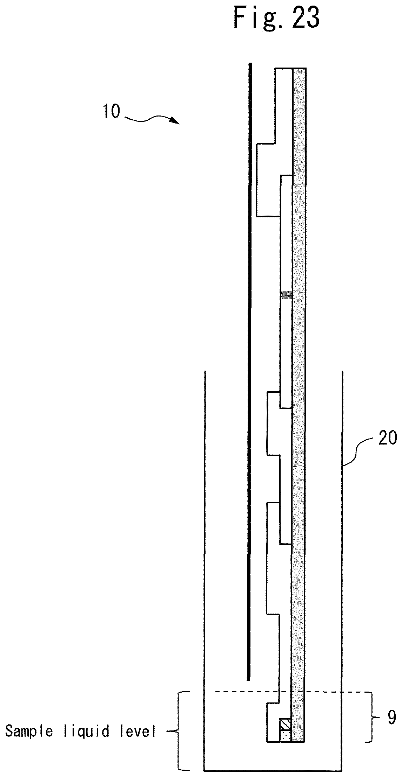

[Figure 23] Figure 23 schematically illustrates an embodiment in which the immunochromatographic device of Figure 22 is immersed in a sample liquid for use.

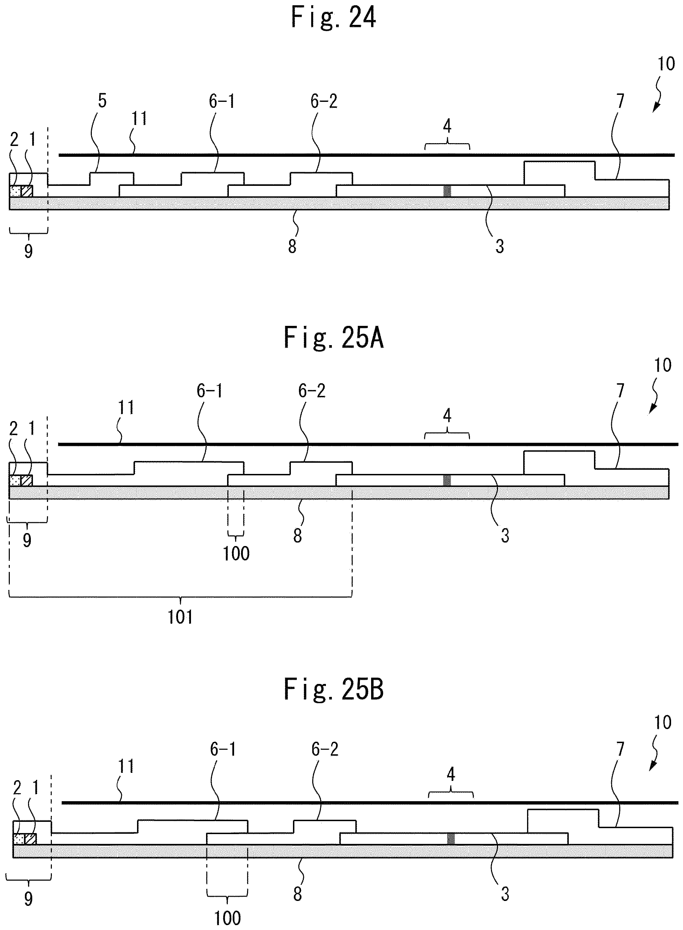

[Figure 24] Figure 24 shows a cross-sectional view of an immunochromatographic device 10 according to an embodiment of the present invention.

[Figure 25] Figure 25 shows a cross-sectional view of the immunochromatographic device 10 according to an embodiment of the present invention, with the sample contact member 5 removed. (A) and (B) show figures with different overlapping lengths (denoted by 100) between the first filter member 6-1 and the second filter member 6-2 (hereinafter also referred to as the overlap distance X).

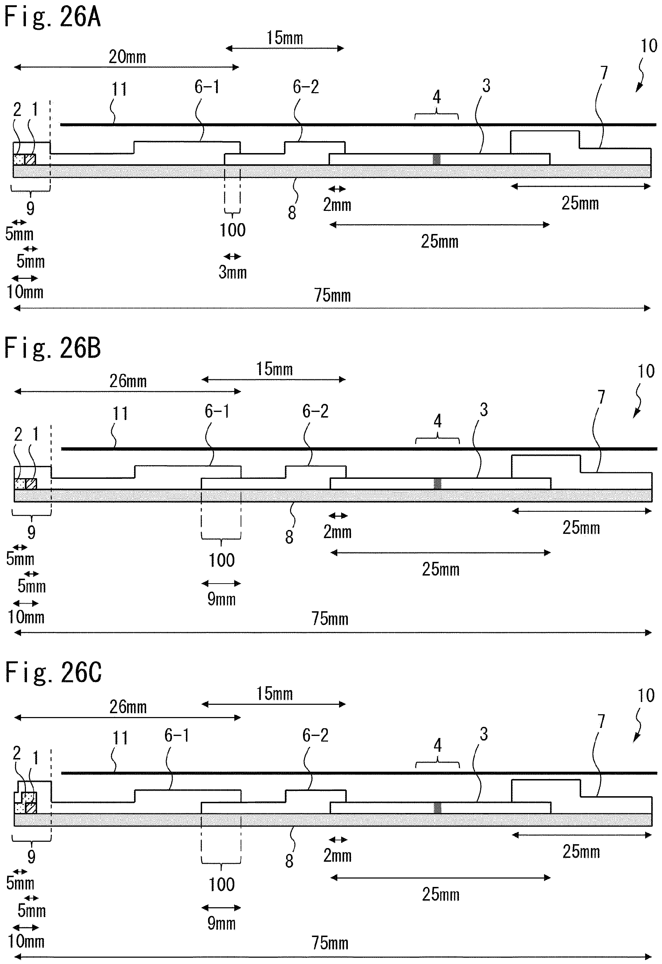

[Figure 26] Figure 26 schematically illustrates immunochromatographic devices used in the Examples of the present invention with their dimensions. (A) illustrates the immunochromatographic device used in Example III-1, (B) illustrates the immunochromatographic device used in Example III-5, and (C) illustrates the immunochromatographic device used in Example III-6.

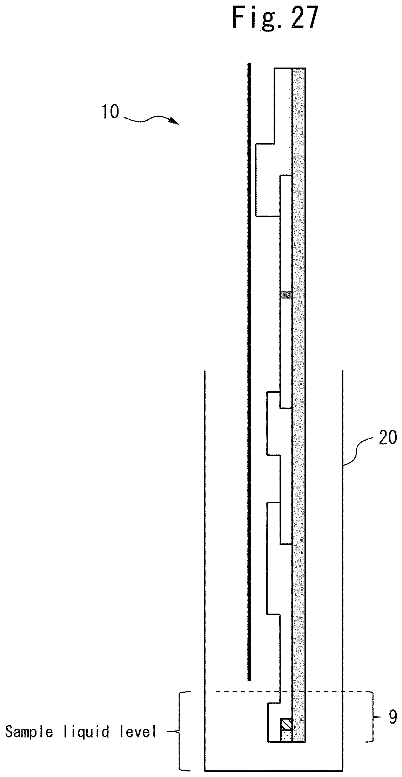

[Figure 27] Figure 27 schematically illustrates an embodiment in which the immunochromatographic device 10 according to an embodiment of the present invention is immersed in a milk sample for use.

## DESCRIPTION OF EMBODIMENTS

The present invention will be described in detail by referring to the specific embodiments below. However, the present invention should not be limited to the following embodiments, but can be implemented in any form to the extent that it does not depart from the gist of the present invention.

### <<FIRST EMBODIMENT>>

The first embodiment of the present invention relates to an immunochromatographic device for detecting a target bacterium in a sample liquid based on an antigen-antibody reaction. It also relates to a method for detecting a target bacterium in a sample liquid based on an antigen-antibody reaction using the immunochromatographic device.

[Immunochromatographic Device]

The term "immunochromatographic device" as used herein may refer to a kit containing an immunochromatographic device for detecting a subject to be detected (target bacterium) in a sample liquid based on an antigen-antibody reaction detection. When the immunochromatographic device is used, an immersing region 9 of the immunochromatographic device 10 is immersed in a sample liquid contained in a sample liquid retention container 20, whereby the sample liquid is developed from a sample contact member 5 via a membrane carrier for chromatographic development 3 to an absorption member (absorption pad) 7 using the capillary principle. Once a complex formed with a labeling antibody and an antigen contained in the sample liquid is developed, the developed complex is captured by a capture antibody in a detection region of the membrane carrier for chromatographic development 3, the label of the labeling antibody is detected, whereby the presence or absence of the target bacterium is determined. In order to prevent the sample liquid from developing from areas outside the immersing region 9, all or part of the areas outside the immersing region 9 may be covered by a water-impermeable cover member. If the labeling antibody and/or the bacteriolysis agent can elute into the sample liquid, a portion of the immersing region 9 may be covered by a water-impermeable cover member. The immunochromatographic device may consist solely of the immunochromatographic device 10, or may further include a sample liquid retention container 20 for retaining the sample liquid, or may further include an instruction manual.

The immunochromatographic device according to this embodiment includes (a) a labeling antibody-attached member 1, to which a labeling antibody is attached, and (b) the immunochromatographic device 10, which has the immersing region 9 and the detection region, on which a capture antibody is immobilized. When the immunochromatographic device is used, at least a portion of the labeling antibody-attached member 1 and the immersing region 9 are both immersed in the sample liquid. The labeling antibody-attached member 1 may be arranged in the immersing region of the immunochromatographic device 10, or may be arranged to be apart from the immunochromatographic device 10. When the labeling antibody-attached member 1 is immersed in the sample liquid, the labeling antibody, which is attached in an elutable manner, elutes and diffuses into the sample liquid. This causes a reaction between the antigen and the labeling antibody in the sample liquid, allowing for a sufficiently long reaction time. The sufficiently long reaction time increases the amount of complex formed from the antigen and the labeling antibody. The thus-formed complex is captured by the immobilized capture antibody in the detection region to achieve high sensitivity. The presence of a bacteriolysis agent in the sample liquid causes a bacteriolysis reaction prior to the antigen-labeling antibody reaction. This allows the bacteriolysis reaction and the antigen-labeling antibody reaction to occur in the sample liquid, ensuring a longer reaction time for each reaction than with conventional technology, thereby further improving detection sensitivity.

The bacteriolysis agent in the sample liquid may be added to the sample liquid externally or may elute from the bacteriolysis agent-attached member 2 included in the immunochromatographic device. When it is added externally, the immunochromatographic device may include the bacteriolysis agent separately from the immunochromatographic device 10. The immunochromatographic device according to this embodiment may further include (c) a bacteriolysis agent-attached member, to which a bacteriolysis agent is attached 2. The bacteriolysis agent-attached member 2 may be provided in the sample liquid retention container 20 in advance or provided in the immunochromatographic device 10. When the immunochromatographic device is used, at least a portion of the bacteriolysis agent-attached member 2 may be immersed in the sample liquid. The bacteriolysis agent-attached member 2 may be arranged in the immersing region of the immunochromatographic device 10, or may be arranged to be apart from the immunochromatographic device 10. When the bacteriolysis agent-attached member 2 is immersed in the sample liquid, the bacteriolysis agent elutes and diffuses into the sample liquid, thereby causing a bacteriolysis reaction in the sample liquid. When the bacteriolysis agent-attached member 2 is arranged in the immunochromatographic device 10, immersing the immunochromatographic device 10 into the sample liquid makes both the bacteriolysis agent and the detection antibody elute into the sample liquid. This gives rise to a bacteriolysis reaction in the sample liquid, followed by an antigen-labeling antibody reaction in the sample liquid. The reaction time for both reactions can be kept long enough to increase the amount of the complex formed from the antigen and the labeling antibody and to thereby achieve high sensitivity. In addition, arranging the bacteriolysis agent-attached member 2 in the immunochromatographic device 10 enables the immunochromatographic device 10 to detect bacteria independently, thereby further simplifying the configuration of the immunochromatographic device.

The labeling antibody-attached member 1 of item (a) may be made of any material as long as it allows for attachment of the labeling antibody. The labeling antibody may preferably be attached to the labeling antibody-attached member 1 so as to be elutable. The labeling antibody-attached member may be manufactured by impregnating a water-absorbent member with a solution containing the labeling antibody, optionally followed by drying. Examples of water-absorbent members include: fiber aggregates such as paper, woven and non-woven fabrics, and fiberglass; and porous materials such as sponges. According to another embodiment, the labeling antibody-attached member 1 may be prepared by applying or adhering a solution containing the labeling antibody directly to a component member of the immunochromatographic device 10. When adhered, the labeling antibody-attached member 1 may be mixed with a thickener to prepare a water-soluble adhesive material. When the labeling antibody-attached member 1 is provided in the immunochromatographic device 10, it may be arranged at any member of the immunochromatographic device 10 as long as at least a portion of the labeling antibody-attached member 1 is immersed in the sample liquid when the immunochromatographic device 10 is used.

The amount of the labeling antibody attached may be selected as appropriate depending on various conditions, such as the volume of the sample liquid and the labeling antibody's ability to form a complex with the target substance. As an example, the amount may be from 0.01 to 1000 μg. At least a portion, e.g., 10% or more, preferably 50% or more, more preferably 90% or more, of the attached labeling antibody can diffuse into the sample liquid.

The immunochromatographic device 10 of item (b) can include any configuration that a normal immunochromatographic device 10 used in an immunochromatographic system may have. The immunochromatographic device 10 is configured such that the immersing region 9 is arranged upstream and the detection region downstream. The detection region of the immunochromatographic device 10 has a capture site 4, on which the capture antibody is immobilized. It is also possible to form a control site downstream of the capture site 4 by immobilizing a substance can bind to the labeling antibody. The capture site 4 and the control site can be formed on a membrane carrier for chromatographic development 3 in the form of, e.g., lines. The immunochromatographic device 10 has, on the base material 8, a membrane carrier for chromatographic development 3, an absorption member (absorption pad) 7, and a sample contact member 5, and may also have one or more filter members 6. In the immersing region 9 of the immunochromatographic device 10, the labeling antibody-attached member 1 is arranged, and the bacteriolysis agent-attached member 2 may be arranged. The labeling antibody-attached member 1 and/or the bacteriolysis agent-attached member 2 may be arranged on any member as long as a portion thereof is arranged in the immersing region 9, which is immersed in the sample liquid when the device is used. The immunochromatographic device 10 may be usually formed by arranging its components on the base material 8, but may not include the base material 8. Detection can be carried out by immersing the immersing region of the immunochromatographic device 10 into the sample liquid contained in the sample liquid retention container 20.

The bacteriolysis agent-attached member 2 of item (c) may be made of any material as long as it allows for attachment of the bacteriolysis agent in an elutable manner. The bacteriolysis agent-attached member 2 may be formed by impregnating a water-absorbent member with a solution containing the bacteriolysis agent, optionally followed by drying. Examples of water-absorbent members include: fiber aggregates such as paper, woven and non-woven fabrics, and fiberglass; and porous materials such as sponges. According to another embodiment, the bacteriolysis agent-attached member 2 may be prepared by applying or adhering a solution containing the bacteriolysis agent directly to a component member of the immunochromatographic device 10. When adhered, the bacteriolysis agent-attached member 2 may be mixed with a thickener to prepare a water-soluble adhesive material. When the bacteriolysis agent-attached member 2 is provided in the immunochromatographic device 10, it may be arranged at any member of the immunochromatographic device 10 as long as at least a portion of the bacteriolysis agent-attached member 2 is immersed in the sample liquid when the immunochromatographic device 10 is used. The labeling antibody-attached member 1 and the bacteriolysis agent-attached member 2 may be formed as a single member, by impregnating a water-absorbent member with a solution containing the labeling antibody and the bacteriolysis agent.

The types of the bacteriolysis agents (will be explained later) may be selected according to the types of bacteria to be detected, and include, although are not limited to, bacteriolysis enzymes and surfactants.

The amount of the bacteriolysis agent attached may be selected as appropriate, depending on various conditions such as the amount of the sample liquid, the bacteriolysis ability, and the types of the bacteria to be detected. As an example, it may be used in an amount of 0.01 to 100000μg in the case of the bacteriolysis enzyme, and in an amount of 0.1 to 100 mg in the case of the surfactant. At least a portion, e.g., 10% or more, preferably 50% or more, more preferably 90% or more, of the attached bacteriolysis agent can diffuse into the sample liquid.

[Arrangement of Components of Immunochromatographic Device 10]

The arrangement of each component of the immunochromatographic device 10 is explained with reference to Figures 1 to 21. The components of the immunochromatographic device 10 are arranged in layers on the base material 8 and/or on other components. The side on which each component, especially the membrane carrier for chromatographic development 3, is placed is referred to as the front side of the base material 8. The components of the immunochromatographic device 10 may be adhered to each other using an adhesive substance. The adhesive substance may be placed on the base material 8. The base material 8 may be removed after each component is arranged on the base material 8. As an example, the membrane carrier 3 for chromatographic development may be placed on the base material 8, and the absorption member (absorption pad) 7 may be placed to span at least a portion of the downstream end of the membrane carrier for chromatographic development 3 and the base material 8 (Figures 1 to 19). The sample contact member 5 may be arranged to span at least part of the upstream end of the membrane carrier for chromatographic development 3 and on the base material 8 (Figures 1 to 10). With this basic configuration, when the immersing region 9 of the immunochromatographic device 10 is immersed in the sample liquid, the sample liquid is developed on the membrane carrier for chromatographic development 3 via the sample contact member 5, and finally absorbed by the absorption member (absorption pad) 7. The sample contact member 5 may be divided into two or more parts (Figures 7 and 9), and labeling antibody-attached member 1 and/or the bacteriolysis agent-attached member 2 may be placed between the two sample contact members. Another component may also be present between the sample contact member 5 and the membrane carrier for chromatographic development 3. In such cases, the sample contact member 5 and the membrane carrier for chromatographic development 3 do not need to be in contact with each other, since the sample liquid may be developed onto the membrane carrier for chromatographic development 3 via the other member. For example, the filter member 6 may be placed to span at least a portion of the membrane carrier for chromatographic development 3 and the base material 8, and the sample contact member 5 may be disposed over at least a portion of the filter member 6 and the base material 8 (Figures 11 to 19). With this configuration, the sample liquid may be developed from the sample contact member 5 in the immersing region onto the membrane carrier for chromatographic development 3 via the filter member 6.

The labeling antibody-attached member 1 may be arranged at any position as long as at least a portion of it can be immersed in the sample liquid when the device is used. The labeling antibody-attached member 1 may be placed on the immunochromatographic device 10 (Figures 1 and 3 to 19), or it may be placed apart from the immunochromatographic device 10 (Figures 2). When the labeling antibody-attached member 1 is arranged on the immunochromatographic device 10, it may be arranged n any component as long as a portion thereof is arranged in the immersing region.

The labeling antibody-attached member 1 may be arranged either on the back side of base material 8 (Figures 1) or on the front side of base material 8 (Figures 3 to 19), or as long as a portion thereof is arranged in the immersing region, it may be placed on the sample contact member 5, the filter member 6, the bacteriolysis agent-attached member 2, or the membrane carrier for chromatographic development 3. Alternatively, the labeling antibody-attached member 1 may be arranged to cover the base material and/or any of these components. A portion or all of labeling antibody-attached member 1 may be covered by another component, such as the sample contact member 5 (Figures 4 to 19), or the labeling antibody-attached member 1 may be exposed to the outside (Figures 1 to 3). Among these, from the viewpoint of allowing the bacteriolysis and antigen-antibody reactions to proceed in close proximity, both the labeling antibody-attached member 1 and the bacteriolysis agent-attached member 2 may preferably be covered by the sample contact member 5 (Figures 4 to 6, 11, 12, and 14 to 19). From the viewpoint of the arrangement of the labeling antibody-attached member 1, the immunochromatographic device 10 shown in each of Figures 7 to 10 and 13 does not correspond to this embodiment because while the labeling antibody-attached member 1 is arranged on the immunochromatographic device 10, it is not arranged in the immersing region 9.

The bacteriolysis agent-attached member 2 may be arranged at any position as long as at least a portion thereof is immersed in the sample liquid. The bacteriolysis agent-attached member 2 may be placed on the immunochromatographic device 10 (Figures 1, 3 to 12, and 14 to 19), or may be arranged to be apart from the immunochromatographic device 10 (Figure 2). The bacteriolysis agent-attached member 2 may be arranged either on the back side or on the front side of the base material 8 (Figures 1 and 3 to 9, 11, 12, and 14 to 19), or as long as a portion thereof is arranged in the immersing region 9, it may be arranged on the sample contact member 5, the filter member 6, the labeling antibody-attached member 1, or the membrane carrier for chromatographic development 3. The bacteriolysis agent-attached member 2 may be arranged to cover the base material and/or any other component. The bacteriolysis agent-attached member 2 and the labeling antibody-attached member 1 are both arranged in the immersing region 9 so as to be either in contact with each other (Figures 3, 4, 6, 11, 12, 14, 17, 18, and 19) or apart from each other (Figures 2, 5, 7 to 10, 15, and 16). When the labeling antibody-attached member 1 and the bacteriolysis agent-attached member 2 ae in contact with each other, their wall surfaces may be in contact (Figures 3, 4, and 11), or they may overlap in whole or in part (Figures 6, 12, 14, 17, 18, and 19). As an example, the bacteriolysis agent-attached member 2 may cover the base material 8 and the labeling antibody-attached member 1 (Figures 6 and 12). In this case, the bacteriolysis agent-attached member 2 may cover only a portion of the labeling antibody-attached member 1, or as shown in Figures 6 and 12, it may completely cover the labeling antibody-attached member 1. A portion or all of the bacteriolysis agent-attached member 2 may further be covered by another member such as the sample contact member 5 (Figures 4, 5, 6, 9, 11, 12, and 15 to 19), or may be exposed to the outside (Figures 1 to 3, 7, and 8). As another example, the labeling antibody-attached member 1 may cover the base material 8 and the bacteriolysis agent-attached member 2. The labeling antibody-attached member 1 may cover only a portion of the bacteriolysis agent-attached member 2 (Figures 16 to 18), or may completely cover the bacteriolysis agent-attached member 2. From the viewpoint of the arrangement of the bacteriolysis agent-attached member 2, the immunochromatographic device 10 shown in Figure 9 does not correspond to this embodiment because while the bacteriolysis agent-attached member 2 is arranged on the immunochromatographic device 10, it is not arranged in the immersing region 9.

The membrane carrier for chromatographic development 3 is a component for carrying out chromatographic development on the membrane using the capillary principle. The membrane carrier for chromatographic development 3 may be made of any material as long as it allows for immunochromatography. Preferable examples include nitrocellulose, mixed nitrocellulose esters, polyvinylidene fluoride, and nylon. An example is a nitrocellulose membrane. The capture antibody may be immobilized on the membrane carrier for chromatographic development 3 to form a capture site 4. A substance that can bind to the labeling antibody may be immobilized on the membrane downstream of the capture site 4 to form a control site. The capture region and the control region can be formed on the membrane carrier for chromatographic development 3 in the form of, e.g., lines, which constitute a detection region.

The sample contact member 5 is a member that is in contact with the sample, and may be any member as long as a solution can pass through it. Examples of members that can be used as the sample contact member 5 include fiber aggregates, such as filter paper, cloth, or non-woven fabric. Examples of fibers that can be used for the sample contact member 5 include: chemical fibers such as cellulose fiber and glass fiber; and natural fibers such as cotton. The sample contact member 5 may also serve as a filter member 6 if it is adjusted to have an appropriate exclusion size due to fiber aggregates.

The filter member 6 is a member for removing a portion of the substances contained in the sample liquid. Specifically, while the sample liquid contains substances that can clog the membrane carrier for chromatographic development 3, in order to remove such substances, the filter member 6 may be arranged upstream of the membrane carrier for chromatographic development 3. The filter member 6 may be made of fiber aggregates having a predetermined retention particle size. Examples of fibers that can be used for the filter member 6 include chemical fibers, especially glass fiber. As an example, when milk is used as the sample, the filter member can be a member for removing fat globules in the milk. Since the diameter of fat globules in milk are about 1 to 10 $\mu$m or more, the filter member may be a filter with a retention particle size that can remove milk fat globules, for example 1 to 10 $\mu$m, preferably 1 to 5 $\mu$m. The filter member 6 may be or may not be provided depending on the type of the sample liquid, and one or more filter members 6 may be provided.

The absorption member 7 is a water-absorbent member located at the downstream end for absorbing the chromatographically developed solution downstream to carry out chromatographic development continuously. The absorption member 7 may be any water-absorbent material, such as cotton, cloth, paper, etc. It may cover a portion of the downstream end of the membrane carrier for chromatographic development 3 and the base material 8.

The base material 8 is a thin layer of water-impermeable material on which each component is arranged in the manufacture of the immunochromatographic device 10. It may be made of, e.g., polystyrene, and may be coated with an adhesive substance to secure other members.

[Detection Method of Target Bacteria]

The method for detecting a target bacterium in a sample liquid based on an antigen-antibody reaction using the immunochromatographic device includes the steps of:

immersing the immersing region 9 of the immunochromatographic device 10 in the sample liquid; and
detecting a second complex captured at the detection region of the immunochromatographic device 10 based on the label of the labeling antibody detection.

When the immersing region 9 of the immunochromatographic device 10 is immersed in the sample liquid, at least a portion of the labeling antibody-attached member 1 is immersed in the sample liquid, whereby the labeling antibody elutes into the sample liquid. This allows for the antigen-antibody reaction to occur in the sample liquid, thereby ensuring a longer reaction time and thus improving detection sensitivity. In addition, when the immunochromatographic device includes the bacteriolysis agent-attached member 2, not only the labeling antibody-attached member 1 but also at least a portion of the bacteriolysis agent-attached member 2 is immersed in the sample liquid, whereby the labeling antibody and the bacteriolysis agent elute into the sample liquid. In this case, the timing of immersion of the labeling antibody-attached member 1 and the bacteriolysis agent-attached member 2 in the sample liquid is not limited, and they may be immersed either simultaneously or sequentially (i.e., one of the labeling antibody-attached member 1 and the bacteriolysis agent-attached member 2 is immersed before the other) at a slight time interval (e.g., within 5 minutes, 1 minute, or 30 seconds). The presence of the labeling antibody and the bacteriolysis agent in the sample liquid allows for the bacteriolysis reaction and antigen-antibody reaction to occur in the sample liquid, thereby prolonging the reaction time for both reactions and thus improving detection sensitivity. The immersion time may be long enough for the second complex to be captured by the capture antibody immobilized on the immunochromatographic device 10. The immersion time may be until the labeling antibody binds to the control region and the label is detected. In the detection region where the capture antibody is immobilized, a second complex is formed and can be detected based on the label of the labeling antibody.

From the above viewpoint, in order to obtain higher detection sensitivity, at least a portion of the labeling antibody-attached member 1 and at least a portion of the bacteriolysis agent-attached member 2 may preferably be arranged in close proximity. More specifically, when the side of the immersing region or the sample contact member of the chromatographic device is referred to as upstream and the side of the detection region as downstream, and when the direction from upstream to downstream is referred to as the chromatographic development direction, then the labeling antibody-attached member 1 and the bacteriolysis agent-attached member 2 may preferably be arranged so that their positions when projected in the vertical direction relative to the chromatographic development direction are in contact or overlap with each other (i.e., the labeling antibody-attached member 1 and the bacteriolysis agent-attached member 2 include the same position at least partially in the chromatographic development direction). The "overlap" arrangement herein is not limited to cases where the labeling antibody-attached member 1 and the bacteriolysis agent-attached member 2 are in contact with or overlap each other on the same side of the chromatographic system, but also includes cases where, for example, one of these members is placed on the back side of the chromatographic device so that they are separated from each other. With this arrangement, when the immersing region 9 of the immunochromatographic device 10 is immersed in the sample liquid, at least a portion of the labeling antibody-attached member 1 and at least a portion of the bacteriolysis agent-attached member 2 are immersed in close proximity in the sample liquid, whereby the labeling antibody and the bacteriolysis agent elute into the sample liquid in close proximity and co-exist in the sample liquid. This allows the bacteriolysis reaction and the antigen-antibody reaction to occur in the sample liquid, thereby ensuring a longer reaction time for both reactions and thus improving the detection sensitivity.

The detection method according to this embodiment may also include the step of agitating the detection solution (sample liquid) using the immunochromatographic device 10. Agitation can promote elution of the labeling antibody and the bacteriolysis into the sample liquid and promote bacteriolysis reaction and/or antibody-antigen reaction. After agitation, the immunochromatographic device 10 may be left to stand still, whereby the first complex formed via the antibody-antigen reaction may be, along with the sample liquid, developed on the membrane carrier for chromatographic development 3 via the sample contact member 5 of the immunochromatographic device 10 (and optionally the filter member 6, depending on the device configuration).

When the immunochromatographic device does not include the bacteriolysis agent-attached member 2, the detection method of target bacteria according to this embodiment may also include the step of adding a bacteriolysis agent to the sample liquid. The bacteriolysis agent may be arranged in the sample liquid retention container 20 of the immunochromatographic device in advance. In this case, the step of placing the sample liquid in the retention container also allows for the bacteriolysis agent to disperse in the sample liquid.

The step of detecting the second complex captured by the detection region of the immunochromatographic device 10 based on the label of the labeling antibody detection can be carried out by any appropriate means depending on the type of the label. When gold colloid is used as the label, the coloration in the detection region can be confirmed with the naked eye. The coloration of the control area where the substance that can bind to the labeling antibody is immobilized indicates that the labeling antibody has been properly developed on the membrane carrier for chromatographic development 3.

[Labeling antibody]

The labeling antibody is an antibody that is labeled for detection and forms a complex with the antigen derived from the target bacteria released from the lysed target bacteria via an antigen-antibody reaction. The complex thus formed may also be referred to as a first complex. The type of detection label used for the labeling antibody is not restricted and may be selected as appropriate in accordance with the detection method. Specific examples include: metal colloids such as gold colloids, platinum colloids, and palladium colloids; non-metal colloids such as selenium colloids, alumina colloids, and silica colloids; insoluble granular materials such as colored resin particles, dye colloids, colored liposomes, etc.; enzymes that catalyze chromogenic reactions such as alkaline phosphatase, peroxidase, luciferase, etc.; fluorescent dyes, radioisotopes; and chemiluminescent labels, bioluminescent labels, electrochemiluminescent labels, etc. The method for attaching the label to the antibody is also not restricted, but specific examples of methods that can be used include physical adsorption using the hydrophobicity of the antibody, chemisorption using a functional group of the antibody, etc.

The first complex formed of the antigen derived from the target bacteria and the labeling antibody based on an antigen-antibody reaction is developed from the immersing region to the detection region of the immunochromatographic device 10.

[Capture antibody]

The capture antibody is an antibody that is immobilized in a detection region of the immunochromatographic device 10 and forms a complex with the first complex based on an antigen-antibody reaction. The complex thus formed may also be referred to as a second complex. When used in immunochromatography, the capture antibody may more specifically be immobilized on the membrane carrier for chromatographic development 3, and the location where it is immobilized may be referred to as a capture site 4 of the detection region. There are no restrictions on the method used to immobilize the antibody on the solid-phase material. Examples of specific methods include immobilization by physisorption using the hydrophobicity of the antibody and immobilization by chemical bonding using the functional groups of the antibody.

The second complex is held at the position where the capture antibody is immobilized via an antigen-antibody reaction with the immobilized capture antibody, and where the labeling of the labeling antibody is detected. If the label is detected at the capture site 4, where the capture antibody is immobilized, then the target bacteria are detected in the sample solution.

[Antibodies]

The term "antibody" used herein refers to a protein that recognizes and binds to a specific antigen or substance, which may also be referred to as an immunoglobulin (Ig). Common antibodies typically have two light chains (light chains) and two heavy chains (heavy chains) that are interconnected by disulfide bonds. There are two classes of light chains, called λ and κ chains, and five classes of heavy chains, called γ, μ, α, δ, and ε chains. Depending on the class of their heavy chains, antibodies are classified into five isotypes: IgG, IgM, IgA, IgD and IgE, respectively.

Heavy chains each include a heavy chain constant (CH) region and a heavy chain variable (VH) region. Light chains each include a light chain constant (CL) region and a light chain variable (VL) region. The light chain constant (CL) region consists of a single domain. The heavy chain constant (CH) region consists of three domains, namely CH1, CH2, and CH3. The light chain variable (VL) region and the heavy chain variable (VH) region each consist of four highly conserved regions called framework regions (FRs; FR-1, FR-2, FR-3, and FR-4) and three hypervariable regions called complementaritydetermining regions (CDRs; CDR-1, CDR-2, and CDR-3). The heavy chain constant (CH) region consists of three CDRs (CDR-H1, CDR-H2, and CDR-H3) and four FRs (FR-H1, FR-H2, FR-H3, and FR-H4), which are arranged from the amino terminus to the carboxy terminus in the order of FR-H1, CDR-H1, FR-H2, CDR-H2, FR-H3, CDR-H3, and FR-H4. The light chain constant (CL) region has three CDRs (CDR-L1, CDR-L2, CDR-L3) and four FRs (FR-L1, FR-L2, FR L3, and FR-L4), which are arranged from the amino terminus to the carboxy terminus in the order of FR-L1, CDR-L1, FR-L2, CDR-L2, FR-L3, CDR-L3, and FR-L4. The variable regions of the heavy and light chains contain binding domains that interact with the antigen.

The antibody of the present invention may be either a polyclonal antibody or a monoclonal antibody, but a monoclonal antibody be preferred. A polyclonal antibody is usually prepared from the serum of an animal immunized with an antigen and is a mixture of various antibody molecules with different structures. A monoclonal antibody, on the other hand, is an antibody composed of a single type of molecules containing a combination of light chain variable (VL) and heavy chain variable (VH) regions having determined amino acid sequences. Monoclonal antibodies can be produced from clones derived from antibody-producing cells, or they can be produced using genetic engineering technique, by obtaining nucleic acid molecules having gene sequences encoding amino acids of antibody proteins. It is also a well-known technique to those skilled in the art to improve the binding and specificity of antibodies using genetic information of their heavy chains and light chains or their variable regions and CDRs.

The antibody of the present invention may be a fragment and/or derivative of an antibody. Fragments of antibodies include $F(ab')_2$, Fab, Fv, etc. Antibody derivatives include antibodies in which amino acid mutations have been artificially introduced into the constant region(s) of the light and/or heavy chains, antibodies in which the domain configuration of the constant region(s) of the light and/or heavy chains has been modified, antibodies with two or more Fc regions per molecule, glycosylated antibodies, bispecific antibodies, antibody conjugates in which an antibody or antibody fragment is bound to a protein other than the antibody, antibody enzymes, antibody conjugates in which an antibody or antibody fragment is bound to a protein other than an antibody, etc. Antibody conjugates, antibody enzymes, tandem scFv, bispecific tandem scFv, diabody, etc. Furthermore, when the aforementioned antibodies or their fragments or derivatives are derived from non-human animals, chimeric or humanized antibodies in which some or all of the sequences other than the CDRs thereof are replaced with the corresponding sequences of human antibodies are also included in the scope of the antibody of the present invention. When the simple term "antibodies" is used herein, it is intended to also encompass fragments and/or derivatives of antibodies, unless otherwise specified.

When the antibody of the present invention causes antigen-antibody reactions with certain bacteria, it means that they bind specifically to some components of the bacteria as antigens. The components of bacteria that serve as antigens for the antibody of the present invention are not limited. It may be a component contained in the cell walls or cell membranes that are exposed outside the bacterial cells, or it may be a component contained in the cytoplasm, cell organelles, or nucleus and not exposed outside the bacterial cells, i.e., an intracellular antigen.

According to an embodiment, the antibody of the present invention may preferably cause antigen-antibody reactions with intracellular antigens of bacteria, more preferably with ribosome proteins of bacteria, still more preferably with ribosome proteins L7/L12 of bacteria. As used herein, the term "ribosome protein L7/L12," or simply "L7/L12," refers to a type of ribosomal protein essential for microbial protein synthesis and commonly possessed by various bacteria. For antibodies that cause antigen-antibody reactions against the bacterial ribosomal proteins L7/L12 and methods for producing the same, reference may be made to, e.g., to WO2000/006603A, the publication of an earlier patent application filed by the present inventors.

The degree of antigen-antibody reaction between the antibody of the present invention and the bacteria to be detected is not particularly limited, but the antigen-antibody reaction may occur at least to the extent that it can be detected by some known detection method.

[Bacteria as Detection Targets]

The bacteria as the target to be detected may be any bacteria. Example include bacteria belonging to the genus Escherichia, the genus Staphylococcus, the genus Klebsiella, the genus Proteus, the genus Enterococcus, the genus Citrobacter, the genus Pseudomonas, the genus Bacillus, the genus Serratia, the genus Rahnella, the genus Listeria, the genus Enterobacter, and the genus Salmonella, the genus Moraxella, the genus Aeromonas, the genus Lactobacillus, the genus Micrococcus, the genus Acinetobacter, the genus Campylobacter, the genus Vibrio, the genus Streptococcus, the genus Haemophilus, the genus Mycoplasma, the genus Legionella, the genus Bordetella, the genus Chlamydia, and the genus Neisseria, the genus Yersinia, the genus Erwinia, the genus Hafnia, the genus Morganella, the genus Providencia, the genus Shigella, and the genus Pectobacterium.

Bacteria belonging to the genus Escherichia include Escherichia coli (E. coli, EC) and Escherichia albertii.

Bacteria belonging to the genus Staphylococcus include Staphylococcus aureus (Staphylococcus aureus, S.

aureus, SA), S. epidermidis, and S. argenteus.

Bacteria belonging to the genus Streptococcus include Streptococcus uberis (S. uberis, SU), S. agalactiae, S. dysgalactiae, and S. pneumoniae.

Bacteria belonging to the genus Klebsiella include Klebsiella pneumoniae (K. pneumoniae, KP), Klebsiella oxytoca (K. oxytoca), and Klebsiella aerogenes (K. aerogenes).

Bacteria belonging to the genus Proteus include Proteus milabilis (P.milabilis, PM), Proteus morganii (P. morganii), Proteus vulgaris (P. vulgaris), and Proteus rettgeri (P. rettgeri).

Bacteria belonging to the genus Enterococcus include Enterococcus faecalis and Enterococcus faecium.

Bacteria belonging to the genus Citrobacter include Citrobacter freundii (C. freundii, CF), Citrobacter amalonaticus (C. amalonaticus), and Citrobacter diversus (C. diversus).

Bacteria belonging to the genus Pseudomonas include Pseudomonas aeruginosa (P. aeruginosa, PA), P. fluorescens, P. phosphorescence, and P. putida.

Bacteria belonging to the genus Bacillus include Bacillus subtilis (B. subtilis, BS), Bacillus cereus (B. cereus), Bacillus licheniformis (B. licheniformis), and Bacillus megaterium (B. megaterium).

Bacteria belonging to the genus Serratia include Serratia liquefaciens (S. liquefaciens, SL), Serratia marcescens (S. marcescens), and Serratia fonticola (S. fonticola).

Bacteria belonging to the genus Rahnella include Rahnella aquatilis (R. aquatilis, RA), Rahnella victoriana (R.victoriana), and Rahnella bruchi (R. bruchi).

Bacteria belonging to the genus Listeria include Listeria monocytogenes (L. monocytogenes, LM), Listeria innocua (L. innocua) and Listeria ivanovii (L. ivanovii).

Bacteria belonging to the genus Enterobacter include Enterobacter cloacae (E. cloacae, ECL), Enterobacter aerogenes (E. aerogenes), and Enterobacter sakazakii (E. sakazakii).

Bacteria belonging to the genus Salmonella include Salmonella enteritidis (S. enteritidis, SE), Salmonella infantis (S. infantis), and Salmonella typhimurium (S. typhimurium).

[Bacteriolysis Agents]

The bacteriolysis agent may be any substance conventionally used in the art as long as it has the ability to lyse the target bacteria. Examples of bacteriolysis agents include bacteriolysis enzymes and surfactants. Any one bacteriolysis enzyme and/or surfactant may be used alone, or two or more surfactants and/or two or more bacteriolysis enzymes may be used in combination.

Bacteriolysis enzymes may be selected according to the type of target bacteria. Examples include lysozyme, lysostaphin, labiase, pepsin, glucosidase, galactosidase, achromopeptidase, $\beta$-N-acetylglucosaminidase, lysostaphin, acetylglucotaminidase, and achromopeptidase. These enzymes may be naturally-occurring enzymes or genetically engineered enzymes. The amount of the bacteriolysis enzyme attached may be determined depending on the desired final concentration and the amount of the sample liquid. As an example, since the bacteriolysis enzyme may be used at a final concentration of from 0.01 $\mu$g/mL to 100 mg/mL, the attached amount may be 0.01 to 100000$\mu$g, preferably 0.1 to 10000$\mu$g, more preferably 1 to 1000$\mu$g.

Surfactants may be selected according to the type of target bacteria. Examples of surfactants include nonionic surfactants, cationic surfactants, anionic surfactants, and zwitterionic surfactants, which may be used either singly or in combination of any two or more. The amount of the surfactant attached may be determined depending on the desired final concentration and the amount of the sample liquid. As an example, since the surfactant may be used at a final concentration of from 0.01% to 10%, the attached amount may be 0.1 to 100 mg, preferably 0.1 to 10 mg, more preferably 1 to 10 mg.

Examples of nonionic surfactants include ester-ether type, ester type, and ether type surfactants, all of which may preferably be used. More specific examples include, although are not limited to, polyoxyethylene alkyl ether, polyoxyethylene alkyl phenyl ether, fatty acid sorbitan ester, alkyl polyglucoside, fatty acid diethanol amide, alkyl monoglyceryl ether, and polysorbates (fatty acid sorbitan esters condensed with tens of molecules of ethylene oxide). Especially preferred examples are polysorbates, more specifically polyoxyethylene (20) sorbitan monolaurate, polyoxyethylene (60) sorbitan monostearate, polyoxyethylene (65) sorbitan tristearate, and polyoxyethylene (80) sorbitan monooleate, etc., and polyoxyethylene alkyl phenyl ethers, more specifically polyoxyethylene (10) octyl phenyl ether, etc.

Examples of anionic surfactants include carboxylates, sulfonates, sulfates, and others, all of which may preferably be used. Specific examples include alkyl ether carboxylates, straight chain alkyl benzene sulfonates (LAS), $\alpha$-olefin sulfonates (AOS), dialkyl sulfosuccinates, formaldehyde condensation products of naphthalene sulfonates, alkyl sulfates (AS), polyoxyethylene alkyl sulfates sulfated by adding ethylene oxide to higher alcohols (AES), and phosphates of higher alcohols or their ethylene oxide adducts. More specific examples include: sodium alkyl sulfates such as sodium dodecyl sulfate and sodium myristyl sulfate; sodium N-acyl sarcosinates such as sodium N-lauroyl sarcosinate and sodium N-myristoyl sarcosinate; N-acyl glutamates such as sodium dodecylbenzene sulfonate, sodium hydrogenated coconut fatty acid monoglyceride monosulfate, sodium lauryl sulfoacetate, and sodium N-palmitoyl glutamate; sodium N-methyl-N-acyl alanine, and sodium $\alpha$-olefin sulfonate.

Examples of cationic surfactants include amine salts and quaternary ammonium salts, both of which may preferably be used. Specific examples include distearyldimethyl ammonium chloride, benzalkonium chloride, hexadecyltrimethyl ammonium bromide, hexadecyltrimethyl ammonium bromide, and myristyltrimethyl ammonium bromide.

Examples of zwitterionic surfactants include, although are not limited to, amino acids (e.g., alkyl amino fatty acid salts), betaines (e.g., alkyl betaines), and amine oxides (e.g., alkylamine oxides). More specific examples include dimethyl ammoniopropane sulfonate, dodecyl dimethyl ammoniobutyrate, betaine laurylate, and amide propyl betaine.

## <<SECOND EMBODIMENT>>

The second embodiment of the present invention relates to a method of producing a chromatographic device including a membrane carrier for chromatographic development having a detection region on which a capture reagent containing a specific binding substance, which specifically binds to a target substance to be detected (capture antibody), is immobilized.

This embodiment relates to a method of producing a chromatographic device including a membrane carrier for chromatographic development having a detection region on which a capture reagent containing a specific binding substance, which specifically binds to a target substance to be detected (capture antibody), is immobilized, the method comprising the steps of:

applying a capture reagent solution containing a specific binding substance (capture antibody) to the detection region of the membrane carrier for chromatographic development; and

drying the membrane carrier for chromatographic development having the detection region to which the capture reagent solution has been applied,

wherein the step of drying is carried out at a temperature of 55°C or more for a period of 3 hours or more but 30 hours or less.

Thus, the step of drying is carried out at a temperature of 55°C or more for a period of 3 hours or more but 30 hours or less, whereby the changes in sensitivity of the resulting chromatographic device over time can be suppressed during the storage period.

The step of applying refers to applying the capture reagent solution to the detection region of the membrane carrier for chromatographic development. The step of applying may be carried out by applying the capture reagent solution using a brush, stamp, roller, etc., or by dripping using an inkjet or other dispensing device. The applying may be carried out in any shape, although it may usually be applied in lines in the detection regions.

The capture reagent solution to be used in the step of applying includes a specific binding substance (capture antibody) in an amount of 0.1 mg/mL or more but 20 mg/mL or less. From the viewpoint of achieving sufficient sensitivity for detection, it may preferably be 0.3 mg/mL or more, more preferably 1 mg/mL or more. On the other hand, from the viewpoint of controlling false positives, it may preferably be 10 mg/mL or less, more preferably 5 mg/mL or less. The capture reagent solution may further contain buffer, sugar, and/or protein. When the capture reagent solution containing at least one of sugar, buffer solution, and protein is applied to the membrane carrier for chromatographic development and then subjected to the drying step, the production method according to this embodiment can inhibit nonspecific binding without including additional blocking and washing steps. This reduces the steps required in the production of the chromatographic device.

Examples of buffers include: inorganic salts such as phosphate, acetate, and borate; good buffers such as CAPSO, MOPSO, TAPS, and ACES; and Tris hydrochloride, which may be selected according to according to the desired pH. The concentration may be 1 mM or more but 500 mM or less. When the capture reagent solution contains buffer, it may become possible to increase the stability of the specific binding substance, and also to improve the detection performance of the specific binding substance. From the viewpoint of achieving buffering ability, it may preferably be 3 mM or more, more preferably 5 mM or more. However, if the ion intensity is too high, the specific binding substance may be destabilized. Accordingly, it may preferably be 300 mM or less, more preferably 100 mM or less.

Examples of sugars include monosaccharides, disaccharides, and polysaccharides. Specific examples include sucrose, maltose, and trehalose. Its concentration may be 0.1 mass % or more but 10 mass % or less. When the capture reagent solution contains sugar, it may become possible to prevent the specific binding substance from deteriorating during storage. From the viewpoint of controlling deterioration, it may preferably be 0.3 mass % or more, more preferably 1 mass % or more. On the other hand, from the viewpoint of controlling moisture absorption after production, it may preferably be 7.5 mass % or less, more preferably 5 mass % or less.

Examples of proteins include casein, albumin, and fat-free milk. Its concentration may be 0.01 mass % or more but 1.00 mass % or less. When the capture reagent solution contains protein, it may become possible to achieve the effect of inhibiting non-specific reactions (blocking effect). From the viewpoint of achieving blocking effect, it may preferably be 0.05 mass % or more, more preferably 0.1 mass % or more. On the other hand, from the viewpoint of not interfering with the binding of the specific binding substance to the membrane carrier for chromatographic development, it may preferably be 0.5 mass % or less, more preferably 0.25 mass % or less.

In the production method according to this embodiment, the step of applying may further include applying an additional reagent solution to the detection region of the membrane carrier for chromatographic development. Such an additional reagent solution may be a capture reagent solution containing another specific binding substance, or may be a control reagent solution. When an additional reagent solution containing a different specific binding substance is applied, it may become possible to provide a chromatographic device that can detect two or more target substances in the sample liquid. When a control reagent solution is applied, the control reagent solution may contain a substance that can bind to the labeling antibody. When the control reagent solution is applied to the membrane carrier for chromatographic development and then subjected to the drying step, the substance that can bind to the labeling antibody is immobilized in the detection region of the membrane carrier for chromatographic development to form a control site. Since the labeling antibody is captured in the control site, observation of the label in the control site indicates that the chromatographic development has properly been carried out. The control site is usually arranged at a position downstream of the capture site on the membrane carrier for chromatographic development.

The drying step refers to the step of drying the detection region to which the capture reagent solution has been applied. The temperature during the drying step may be 55 °C or more, preferably 57 °C or more, more preferably 59 °C or more. From the viewpoint of preventing the specific binding substance contained in the capture reagent from deteriorating, the temperature during the drying step may be 80 °C or less, preferably 70 °C or less, more preferably 65 °C or less. The drying step may be carried out for a period of 3 hours or more but 30 hours or less. From the viewpoint of suppressing sensitivity change, the drying time may preferably be 6 hours or more, more preferably 10 hours or more. On the other hand, from the viewpoint of suppressing the degradation of detection performance, it may preferably be 24 hours or less, more preferably 18 hours or less. As an example, the drying process can be performed by using a constant-temperature dryer. In the conventional technology, the drying step is avoided at high temperatures exceeding, e.g., 50°C to avoid denaturation of the specific binding substance contained in the capture reagent.

The drying step allows the specific binding substance to be immobilized onto a membrane support for chromatographic development via physical adsorption by utilizing its hydrophobic nature. The immobilization herein can be achieved by applying the capture reagent solution containing the specific binding substance to the detection region of the chromatographic development membrane carrier and then drying it. When the sample solution is chromatographically developed on the membrane carrier for chromatographic development during use of the chromatographic device, the immobilized specific binding substance remains on the membrane carrier for chromatographic development, and can capture the complex of the substance to be detected in the sample solution with the labeling antibody. This makes it possible to detect the labeling of the complex and thereby determine the presence or absence of the substance to be detected.

The production method of the chromatographic device includes the step of assembling the chromatographic device using the membrane carrier for chromatographic development that has undergone the immobilization step and the drying step. As an example, the chromatographic device can be assembled by layering, on the base material, the membrane carrier for chromatographic development to which the specific binding substance has been attached is arranged, as well as other members constituting the chromatographic device, such as the sample contact member, absorption member, filter member, and cover member. An adhesive material may be applied over the base material for fixing other members, and it may also be applied to between other members. In order for the sample liquid to be chromatographically developed from the sample contact member through the filter member and the membrane carrier for chromatographic development to the absorption member, these members are arranged successively in this order. The state of being "arranged successively in this order" herein does not only refer to the state where parts of two adjacent members are overlapped and in contact with each other, but also refers to the state where two adjacent members are arranged side by side so that the facing wall surfaces of these members are in contact without overlapping. Each member may be in the form of a sheet with a generally rectangular plain shape, and its thickness may vary from members to members, but may be from 50 to 2000 μm. The side of the sample contact member of the chromatographic device may be referred to as upstream, and the side of the detection area may be referred to as downstream. The direction from upstream to downstream may also be referred to as the chromatographic development direction.

[Chromatographic Device]

The chromatographic device to be produced in this embodiment is a device (also referred to as a strip) for detecting a target substance in a sample solution by developing the sample solution on the membrane carrier for chromatographic development based on capillary action. An example of the chromatographic device 10 of this embodiment is shown in Figure 22. The chromatographic device 10 includes the sample contact member 5, the membrane carrier for chromatographic development 3, and the absorption member 7 (absorption pad), in this order along the direction from upstream to downstream. The chromatographic device 10 may also include other optional members, such as the base material 8, the filter member 6, and the cover member 11. These members are arranged with at least parts of two adjacent members in contact with each other such that when the sample liquid is in contact with the sample contact member 5, it is developed from the sample contact member 5 through the membrane member for chromatographic development 3 to the absorption member 7 based on capillary action. The adjacent members may be made into contact with each other either by arranging their facing side surfaces without space or by at least partially overlapping these members. Depending on the type of the sample liquid, one or more filter members 6 may be arranged between the sample contact member 5 and the membrane member for chromatographic development 3. The target substance which may be contained in the developed sample liquid is captured by the specific binding substance immobilized on the membrane member for chromatographic development 3 in the capture site 4 of the detection region. Since the captured target substance forms a complex with the labeling antibody, the presence or absence of the target substance can be determined by detecting the labeling. The labeling antibody which binds to the target substance may be added to the sample liquid in advance. Alternatively, the chromatographic device 10 may further include a labeling antibody-attached member 1, to which a labeling antibody is attached, such that an antigen-antibody reaction between the target substance and the labeling antibody occurs when the chromatographic device 10 is used. When the target substance is a bacterium, then the chromatographic device 10 may further include a bacteriolysis agent-attached member 2 such that a bacteriolysis reaction occurs when the chromatographic device 10 is used. All or part of the chromatographic device may be covered by a water-impermeable cover member 11 to eliminate the development of the sample liquid from points other than the sample contact member 5. The filter member 6 can remove foreign substances in the sample liquid and prevent clogging of the membrane carrier for chromatographic development. The chromatographic device 10 is manufactured by stacking each component on the base material 8.

The sensitivity of a chromatographic device changes over a storage period after production. In the case of a chromatographic device manufactured using conventional technology (i.e., a production method that includes a drying step at 50°C or lower), the sensitivity is low immediately after production (hereinafter referred to as initial sensitivity), and increases after several months of storage. Accelerated heating tests are conducted to test for changes in sensitivity over storage periods. The temperature for accelerated heating tests may be a predetermined temperature selected from 45 °C to 55 °C and can be performed for a predetermined period of time ranging from 1 day to 4 weeks. An example of the condition for such accelerated warming tests is heating at 50°C for one week. Storage at 50°C for one week is usually considered equivalent to storage at room temperature for 3 to 6 months. To evaluate the sensitivity variation from the initial sensitivity, the sensitivity change can be calculated as the rate of change in sensitivity after an accelerated heating test from the initial sensitivity (hereinafter referred to as "post-accelerated test sensitivity "), which can be calculated as follows:

[Formula 1]

$$\text{Rate of change} = \frac{(\text{Post-accelerated test sensitivity}) - (\text{Initial sensitivity})}{\text{Initial sensitivity}} \times 100$$

From the viewpoint of quality control, the change in sensitivity may preferably be as small as possible. Specifically, the rate of change between the post-accelerated test sensitivity and the initial sensitivity may preferably be 50 % or less, more preferably 40 % or less, more preferably 30 % or less.

The membrane carrier for chromatographic development 3 is not restricted as long as it may be used for chromatographic development. Preferable examples include nitrocellulose, mixed nitrocellulose esters, polyvinylidene fluoride, and nylon. As an example, nitrocellulose membrane may be used. A capture reagent solution containing the specific binding substance may be applied to the detection region of the membrane carrier for chromatographic development 3 and then subjected to drying to immobilize the substance, whereby the capture site can be formed. In addition, the substance that can bind to the labeling antibody may be immobilized at a position downstream of the capture site, whereby the control site can be formed. The capture site 4 and the control site may be formed in the shape of, e.g., lines, in the detection region of the membrane carrier for chromatographic development.

The sample contact member 5 is a member to be in contact with the sample (sample liquid), and may be any member through which the sample liquid can pass. Examples of materials for the sample contact member 5 include fiber aggregates such as filter paper, cloth, or non-woven fabric. Examples of fibers that can be used for the sample contact member 5 include chemical fibers such as cellulose fiber and glass fiber, and natural fibers such as cotton. It is also possible to make the sample contact member 5 also serve as the filter member by adjusting the fiber aggregates to have an exclusion size. The sample contact member may have a partial region that serves as a sample contact region (immersing region 9), which is in contact with the sample liquid.

The filter member 6 is a member for removing some of the substances contained in the sample liquid. Specifically, the sample liquid contains substances that can clog the membrane carrier for chromatographic development 3. In order to remove such substances, the filter member 6 can be provided upstream of the membrane carrier for chromatographic development 3. Examples of materials for the filter member 6 include fiber aggregates having a predetermined retention particle size. Examples of fibers that can be used for the filter member include chemical fibers, especially glass fiber. As an example, when milk is used as the sample, the filter member 6 can be a member for removing fat globules in the milk. Since the diameter of fat globules in milk are about 1 to 10 $\mu$m or more, the filter member may be a filter with a retention particle size that can remove milk fat globules, for example 1 to 10 $\mu$m, preferably 1 to 5 $\mu$m. The filter member 6 may be or may not be provided depending on the type of the sample liquid, and one or more filter members 6 may be provided.

The absorption member 7 is a water-absorbent member located at the downstream end for absorbing the chromatographically developed solution downstream to carry out chromatographic development continuously. The absorption member 7 may be any water-absorbent material, such as cotton, cloth, paper, etc. It may cover a portion of the downstream end of the membrane carrier for chromatographic development 3 and the base material 8.

The base material 8 is a thin layer of water-impermeable material on which each component is arranged in the manufacture of the immunochromatographic device 10. It may be made of, e.g., polyester or polystyrene, and may be coated with an adhesive substance to secure other members.

The labeling antibody-attached member 1 is a member to which the labeling antibody is attached. The labeling antibody-attached member 1 may be arranged at any position as long as at least a portion thereof is immersed in the sample liquid. The labeling antibody-attached member 1 may be arranged in the immersing region 9 of the chromatographic device 10, whereby when the chromatographic device 10 is used, at least a portion of the labeling antibody-attached member 1 is immersed in the sample liquid, a portion of the labeling antibody, which is attached in an elutable manner, elutes and diffuses into the sample liquid. This causes an antigen-antibody reaction in the sample liquid, thereby ensuring a sufficiently long reaction time to increase the amount of complex formed from the substance to be detected and the labeling antibody.

The labeling antibody-attached member 1 may be made of any material as long as it allows for attachment of the labeling antibody. The labeling antibody may preferably be attached to the labeling antibody-attached member 1 so as to be elutable. The labeling antibody-attached member 1 may be produced by impregnating a water-absorbent member with a solution containing the labeling antibody, optionally followed by drying. Examples of water-absorbent members include fiber aggregates such as paper, woven fabric, and non-woven fabric, and porous materials such as sponge. According to another embodiment, the labeling antibody-attached member 1 may be produced by applying or adhering a solution containing the labeling antibody directly to a member of the immunochromatographic device 10. When adhered, the labeling antibody-attached member 1 may be mixed with a thickener to prepare a water-soluble adhesive material. From the viewpoint of improving storage stability, the labeling antibody-attached member may also contain sugar such as sucrose or trehalose.

The amount of the labeling antibody attached may be selected as appropriate depending on various conditions, such as the volume of the sample liquid and the labeling antibody's ability to form a complex with the target substance. As an example, it is possible to use an antibody solution with an antibody concentration prepared so that when the antibody solution is diluted 21-fold, the absorbance of the resulting diluted solution at a wavelength of 540 nm is 0.1 to 2.0.

The bacteriolysis agent-attached member 2 is a member to which the bacteriolysis agent is attached. The bacteriolysis agent-attached member 2 may be arranged at any position as long as at least a portion thereof is immersed in the sample liquid. The bacteriolysis agent-attached member 2 may be arranged between the base material 8 and another member, or as long as a portion thereof is arranged in the immersing region 9, it may be arranged on the sample contact member 5, on the filter member 6, on the labeling antibody-attached member 1, or on the cover member 11, or may be arranged on the back side of the base material 8. The labeling antibody-attached member 1 and the bacteriolysis agent-attached member 2 are both arranged in the immersing region 9. They may be arranged to be in contact with each other or apart from each other. When the labeling antibody-attached member 1 and the bacteriolysis agent-attached member 2 are in contact with each other, their wall surfaces may be in contact, or they may overlap in whole or in part. As an example, the bacteriolysis agent-attached member 2 may be arranged so as to cover the base material 8 and the labeling antibody-attached member 1. In this case, the bacteriolysis agent-attached member 2 may cover only a portion of the labeling antibody-attached member 1, or may completely cover the labeling antibody-attached member 1. On the bacteriolysis agent-attached member 2, the sample contact member 5 and/or the filter member 6 may be arranged. In order to detect bacteria in the sample liquid with high sensitivity, it is preferable to lysis the bacteria to extract the target substance to be detected.

The bacteriolysis agent-attached member 2 may be made of any material as long as it allows for the bacteriolysis agent to be attached so as to be elutable. The bacteriolysis agent-attached member 2 may be produced by impregnating a water-absorbent member with a solution containing the bacteriolysis agent, optionally followed by drying. Examples of water-absorbent members include fiber aggregates such as paper, woven fabric, and non-woven fabric, and porous materials such as sponge. According to another embodiment, the bacteriolysis agent-attached member 2 may be produced by applying or adhering a solution containing the bacteriolysis agent directly to a member of the immunochromatographic device 10. When adhered, the bacteriolysis agent-attached member 2 may be mixed with a thickener to prepare a water-soluble adhesive material. From the viewpoint of improving storage stability, the labeling antibody-attached member may also contain sugar such as sucrose or trehalose. When the bacteriolysis agent-attached member 2 is provided in the immunochromatographic device 10, may be arranged at any position on the immunochromatographic device 10 as long as when the immunochromatographic device is used, at least a portion of the bacteriolysis agent-attached member 2 is immersed in the sample liquid. The labeling antibody-attached member 1 and the bacteriolysis agent-attached member 2 may be formed as a single member, by impregnating a water-absorbent member with a solution containing the labeling antibody and the bacteriolysis agent.

[Bacteriolysis Agents]

The bacteriolysis agent may be any substance conventionally used in the art as long as it has the ability to lyse the target bacteria. Examples of bacteriolysis agents include bacteriolysis enzymes and surfactants. Any one bacteriolysis enzyme and/or surfactant may be used alone, or two or more surfactants and/or two or more bacteriolysis enzymes may be used in combination.

[Bacteriolysis Enzymes]

Bacteriolysis enzymes may be any bacteriolysis enzymes used in the art as long as they can lyse the target bacteria. Bacteriolysis enzymes may be selected according to the type of target bacteria. Examples include lysozyme, lysostaphin, pepsin, glucosidase, galactosidase, achromopeptidase, $\beta$-N-acetylglucosaminidase, acetylglucotaminidase, and achromopeptidase. These enzymes may be naturally-occurring enzymes or genetically engineered enzymes. The amount of the bacteriolysis enzyme attached may be determined depending on the desired final concentration and the amount of the sample liquid. As an example, since the bacteriolysis enzyme may be used at a final concentration of 0.01 $\mu$g/mL or more but 100 mg/mL or less, the attached amount may be 0.01 $\mu$g or more but 100 mg or less, preferably 0.1 $\mu$g or more but 10 mg or less, more preferably 1 $\mu$g or more but 3 mg or less.

[Surfactants]

Surfactants may be selected according to the type of target bacteria. Examples of surfactants include nonionic surfactants, cationic surfactants, anionic surfactants, and zwitterionic surfactants, which may be used either singly or in combination of any two or more. The amount of the surfactant attached may be determined depending on the desired final concentration and the amount of the sample liquid. As an example, since the surfactant may be used at a final concentration of from 0.01 mass % to 10 mass %, the attached amount may be 0.1 mg or more but 100 mg or less, preferably 0.1 mg or more but 10 mg or less, more preferably 1 mg or more but 10 mg or less.
Examples of nonionic surfactants include ester-ether type, ester type, and ether type surfactants, all of which may preferably be used. More specific examples include, although are not limited to, polyoxyethylene alkyl ether, polyoxyethylene alkyl phenyl ether, fatty acid sorbitan ester, alkyl polyglucoside, fatty acid diethanol amide, alkyl monoglyceryl ether, and polysorbates (fatty acid sorbitan esters condensed with tens of molecules of ethylene oxide). Especially preferred examples are polysorbates, more specifically polyoxyethylene (20) sorbitan monolaurate, polyoxyethylene (60) sorbitan monostearate, polyoxyethylene (65) sorbitan tristearate, and polyoxyethylene (80) sorbitan monooleate, etc., and polyoxyethylene alkyl phenyl ethers, more specifically polyoxyethylene (10) octyl phenyl ether, etc.
Examples of anionic surfactants include carboxylates, sulfonates, sulfates, and others, all of which may preferably be used. Specific examples include alkyl ether carboxylates, straight chain alkyl benzene sulfonates (LAS), $\alpha$-olefin sulfonates (AOS), dialkyl sulfosuccinates, formaldehyde condensation products of naphthalene sulfonates, alkyl sulfates (AS), polyoxyethylene alkyl sulfates sulfated by adding ethylene oxide to higher alcohols (AES), and phosphates of higher alcohols or their ethylene oxide adducts. More specific examples include: sodium alkyl sulfates such as sodium dodecyl sulfate and sodium myristyl sulfate; sodium N-acyl sarcosinates such as sodium N-lauroyl sarcosinate and sodium N-myristoyl sarcosinate; N-acyl glutamates such as sodium dodecylbenzene sulfonate, sodium hydrogenated coconut fatty acid monoglyceride monosulfate, sodium lauryl sulfoacetate, and sodium N-palmitoyl glutamate; sodium N-methyl-N-acyl alanine, and sodium $\alpha$-olefin sulfonate.
Examples of cationic surfactants include amine salts and quaternary ammonium salts, both of which may preferably be used. Specific examples include distearyldimethyl ammonium chloride, benzalkonium chloride, hexadecyltrimethyl ammonium bromide, hexadecyltrimethyl ammonium bromide, and myristyltrimethyl ammonium bromide.
Examples of zwitterionic surfactants include, although are not limited to, amino acids (e.g., alkyl amino fatty acid salts), betaines (e.g., alkyl betaines), and amine oxides (e.g., alkylamine oxides). More specific examples include dimethyl ammoniopropane sulfonate, dodecyl dimethyl ammoniobutyrate, betaine laurylate, and amide propyl betaine.

The specific binding substance refers to a substance that can specifically bind to a target substance to be detected. Examples of specific binding substances include: bioactive substances with interactions, such as ligands and receptors, or their analogs; substrates in enzymatic reactions or their analogs; and antigens in antigen-antibody reactions or their analogs. There are no restrictions to the specific binding substances as long as they fall under these categories. For example, proteins, peptides, amino acids, sugar chains, lipids, or complexes thereof (glycoproteins, glycolipids, fatty acid-modified proteins, and nucleic acids such as DNA and RNA) can be the substance to be detected. From the viewpoint of binding to any substance to be detected, antibodies may preferably be used as specific binding substances. When an antibody is used as a specific binding substance, the chromatographic device can be called an immunochromatographic device.

The target substance to be detected may be any substance, as long as there is a specific binding substance that specifically binds to that target substance. Examples of target substance to be detected include: bioactive substances with interactions, such as ligands and receptors, or their analogs; substrates in enzymatic reactions or their analogs; and antigens in antigen-antibody reactions or their analogs. There are no restrictions to the specific binding substances as long as they fall under these categories. For example, proteins, peptides, amino acids, sugar chains, lipids, or complexes thereof (glycoproteins, glycolipids, fatty acid-modified proteins, and nucleic acids such as DNA and RNA) can be the substance to be detected. Other substances can be artificial, such as low molecular weight compounds or portions of antibodies (e.g., Fab' fragments) that are intended to be therapeutic agents.

The sample liquid may be any liquid as long as it contains a substance to be detected. As an example, it may preferably be a food or environmental sample liquid. The food sample liquid refers to any sample liquid obtained from any food or beverage. For example, the food sample may be food or beverage itself, or may be a sample liquid obtained by diluting, grinding, swabbing off the surface of, and/or suspending food or beverage. The environmental sample refers to any sample liquid obtained from the environment. An example is a sample liquid obtained by wiping off the surface of an environmental substance and dispersing it in a solution. When bacteria are to be detected as the target substances, milk can be used as the sample liquid, among other things.

When an antibody is used as the specific binding substance, the target substance to be detected may be proteins, peptides, sugars, lipids, nucleic acids, or complexes thereof, as well as any other substances that can be recognized by the antibody, such as metals or compounds. In particular, from the viewpoint of use in the food and livestock industry, bacteria may be used as the target substance to be detected, and antibodies that bind to proteins derived from the bacteria may be used as the specific binding substances. When such antibodies bind specifically to some component of a bacterium as an antigen, the antibody can be said to be capable of detecting the bacterium. There are no restrictions on the bacterial components that can be used as antigens for the antibodies of the present invention. It can be a component contained in the cell wall, cell membrane, etc. that is exposed outside the bacterial cell, or it can be a component contained in the cytoplasm, cell organelles, nucleus, etc. that is not exposed outside the bacterial cell, i.e., intracellular antigen. According to an embodiment, the antibodies of the present invention may produce antigen-antibody reactions with bacterial intracellular antigens, more preferably with bacterial ribosomal proteins, and especially with ribosomal proteins L7/L12.

The target substance to be detected may be pre-complexed with the labeling antibody such that when the specific binding substance captures the target substance to be detected at the capture site, the label can be detected at the capture site. The labeling antibody that has not formed a complex with the target substance to be detected may be captured at the control site such that when the labeling is detected at the control site, it is understood that chromatographic development has been properly performed. The label may be selected according to the detection method. specific examples include: metallic colloids such as gold colloids, platinum colloids, palladium colloids, etc.; non-metallic colloids such as selenium colloids, alumina colloids, silica colloids, etc.; insoluble granular materials such as colored resin particles, dye colloids, colored liposomes, etc.; enzymes that catalyze chromogenic reactions, such as alkaline phosphatase, peroxidase, and luciferase; fluorescent dyes and radioisotopes; chemiluminescent, bioluminescent, and electrochemiluminescent labels. Methods for labeling antibodies to make them into labeling antibodies are not limited, specific examples including physical adsorption using the hydrophobic properties of antibodies and chemisorption using the functional groups of antibodies.

## <<THIRD EMBODIMENT>>

The third embodiment of the present invention relates to an immunochromatographic device for detecting a target substance to be detected in a milk sample based on an antigen-antibody reaction, and a method for detecting a target substance to be detected in a milk sample based on an antigen-antibody reaction using the immunochromatographic device.

[Immunochromatographic Device]

The immunochromatographic device is a device (also referred to as a strip) for detecting a target substance to be detected based on an antigen-antibody reaction. According to this embodiment, the immunochromatographic device is related to a dipstick-type immunochromatographic device. The dipstick-type immunochromatographic device refers to an immunochromatographic device for inducing chromatographic membrane development by immersing the immersing region 9 of the immunochromatographic device 10 into a solution. When the immunochromatographic device of this embodiment is used, the immersing region 9 of the immunochromatographic device 10 is immersed in a milk sample contained in a milk sample retention container 20, whereby milk sample solution is developed based on the principle of chromatography. the complex formed with the labeling antibody and the antigen contained in the developed milk sample solution is captured by the capture antibody immobilized in the detection region 4 of the membrane member for chromatographic development 3, and the label of the labeling antibody is detected, whereby the presence or absence of the target substance is detected. The dipstick-type immunochromatographic device may consist solely of the immunochromatographic device 10, but may also contain a sample liquid retention container 20 for containing the milk sample, as well as other components included in a usual immunochromatographic device, such as an instruction manual.

The immunochromatographic device according to this embodiment 10 has an immersing region 9 and a detection region 4, and also includes:

a labeling antibody-attached member 1, to which a labeling antibody is attached, wherein the labeling antibody binds to a target substance to be detected in a milk sample based on an antigen-antibody reaction to form a first complex;
a first filter member 6-1, which has a retention particle size that can remove milk fat globules in milk;
a second filter member 6-2, which has a retention particle size that is smaller than the retention particle size of the first filter member 6-1; and
a membrane member for chromatographic development 3, which has a detection region in which a capture antibody against the target substance to be detected is immobilized.

The immunochromatographic device 10 may further include other components included in a usual immunochromatographic device, such as a bacteriolysis agent-attached member 2, to which a bacteriolysis agent is attached, a sample contact member 5, absorption member 7, a base material 8, and a cover member 11.

When the immunochromatographic device 10 according to this embodiment is used, in order for the milk sample to be chromatographically developed from the first filter member, then the second filter member, and to the membrane member for chromatographic development from upstream to downstream of the immunochromatographic device 10, these members are arranged successively in this order. The state of being "arranged successively in this order" herein does not only refer to the state where parts of two adjacent members are overlapped and in contact with each other, but also refers to the state where two adjacent members are arranged side by side so that the facing wall surfaces of these members are in contact without overlapping. Each member may be in the form of a sheet with a generally rectangular plain shape, and its thickness may vary from members to members, but may be from 50 to 2000 $\mu$m. According to this embodiment, from the viewpoint of promoting chromatographic development, the first filter member 6-1 and the second filter member 6-2 are arranged in contact with partially overlapping with each other. The overlapping parts are represented by 100, and the overlapping distance is represented by X. When the absorption member 7 is arranged, the solution is finally absorbed by the absorption member 7. The side of the immersing region 9 of the immunochromatographic device 10 may be referred to as upstream, and the side of the detection region 4 as downstream. The direction from upstream to downstream may also be referred to as the chromatographic development direction.

The arrangement of each component of the immunochromatographic device 10 is explained with reference to the figures. Each component of the immunochromatographic device 10 is stacked and arranged on the base material 8 and/or on other components. The side of the base material 8 on which each component, especially the membrane member for chromatographic development 3, is placed is referred to as the front side of the base material 8. Each component of the immunochromatographic device 10 may be adhered to other components with an adhesive material. An adhesive material may be applied on the base material 8. The base material 8 may be removed after the components are arranged on the base material 8. As an example, the membrane member for chromatographic development 3 is arranged on the base material 8, and the absorption member 7 (absorption pad) is arranged so as to cover at least a portion on the downstream end of the membrane member for chromatographic development 3 and the base material 8 (Figures 24). The second filter member 6-2 is arranged so as to cover at least a portion on the upstream end of the membrane member for chromatographic development 3 and the base material 8, and the first filter member 6-1 is arranged so as to cover at least a portion on the upstream end of the second filter member 6-2 and the base material 8. The sample contact member 5 may be arranged so as to cover at least a portion of the first filter member 6-1 and the base material 8 (Figures 24). The sample contact member 5 may be omitted (Figures 25). When the sample contact member 5 is omitted, the first filter member also substitutes for the sample contact member 5 and is made into contact with the sample. Except for the sample contact member 5 or the portion of the first filter member 6-1 formed on the base material 8, the outer surface is covered by a cover member 11. The cover member 11 is a water-impermeable member that prevents the members below the cover member 11 from coming into contact with the solution. Figures 24 to 27 indicate as if the cover member 11 is not in contact with other members for the sake of facilitating the understanding. However, the cover member 11 is actually in contact with, and adhered to, other members to form the immunochromatographic device 10. Instead, the cover member 11 may be omitted, and the region exposed to outside may be formed as a water immersing region 9. With the basic configuration described above, when the immersing region 9 of the immunochromatographic device 10 is immersed in the sample liquid, the sample liquid is developed from the sample contact member 5 through the first filter member 6-1, then through the second filter member 6-2 onto the membrane member for chromatographic development 3. The labeling antibody-attached member 1 and/or the bacteriolysis agent-attached member 2 may be arranged in the immersing region. With this arrangement, when the immunochromatographic device 10 is immersed in the milk sample, the labeling antibody and/or the bacteriolysis agent may elute from the immersing region and disperse into the milk sample to cause a bacteriolysis reaction and/or an antigen-antibody reaction in the milk sample to form a complex, which may be developed through the first filter member 6-1 and the second filter member 6-2 onto the membrane member for chromatographic development 3.

The immunochromatographic device 10 may be an elongated sheet having a generally rectangular, especially oblong, plain shape with the chromatographic development direction as the long axis. As an example, the length of the long side may be 40 mm to 100 mm, and the length of the short side may be 3 mm to 20 mm. From the viewpoint of making the distance of chromatographic development as long as possible, the long side of the immunochromatographic device may preferably be 50 mm or more, more preferably 60 mm or more. From the viewpoint of facilitating the operation of the device, the long side of the immunochromatographic device may preferably be 90 mm or less, more preferably 80 mm or less. The thickness of the thickest part of the immunochromatographic device can vary depending on the members to be stacked, but may be from 0.5 mm to 3 mm.

[Filter Member]

The filter member refers to a member for removing fat globules in milk, and may also be referred to a fat globules removal member. This embodiment involves the use of two or more filter members, including a first filter member 6-1 and a second filter member 6-2. The first filter member 6-1 is arranged on the upstream side of the second filter member. These filter members may be any members as long as they can remove fat globules in milk. As an example, a filter made of glass fiber may be used. Fat globules in milk have a diameter of approximately from 1 $\mu$m to 10 $\mu$m or more. According, the filter members may preferably have a retention particle size suitable for removing milk fat globules of such sizes. Although a single filter has been conventionally used, there is a limit to the number of fat globules that can be removed by a single filter, so that clogging can occur. Therefore, two or more filters are used in this embodiment. The second filter member 6-2 may have a retention particle size that is smaller than the retention particle size of the first filter member 6-1. The total length of the filter members used may be selected as appropriate, but from the viewpoint of downsizing the device, it may preferably not exceed half of the length of the immunochromatographic device 10. As an example, the first filter member 6-1 and the second filter member 6-2 may be selected such that when the total length of these filter members in the state of being overlapped with each other (also referred to as the total length of the filter members) is 70 mm or less, preferably 55 mm or less, or 40 mm or less. From the viewpoint of removing fat globules, the total length may be typically 5 mm or more, preferably 10 mm or more, more preferably 15 mm or more, still more preferably 25 mm or more. The total length of the first filter member 6-1 and the second filter member 6-2 overlapped with each other may account for 10% to 70% of the total length of the immunochromatographic device. From the viewpoint of sufficiently removing fat globules, the ratio of the total length of the filter members to the total length of the immunochromatographic device may preferably be 20 % or more, more preferably 25 % or more, still more preferably 30 % or more. From the viewpoint of reducing dead volume and improving sensitivity, the ratio of the total length of the filter members to the total length of the immunochromatographic device may preferably be 60% or less, more preferably 50% or less. The retention particle size of a filter member can be measured by a method for measuring the size of particles in the liquid 98% or more of which can be retained by the filter member.

The retention particle size of the first filter member 6-1 may be selected as appropriate as long as it can remove milk fat globules. As an example, a filter member having a retention particle size of 3 to 10 $\mu$m, preferably 3 to 8 $\mu$m, more preferably 3 to 4 $\mu$m, may be used as the first filter member 6-1. The retention particle size of the second filter member 6-2 may be selected as appropriate as long as it is smaller than the retention particle size of the first filter member 6-1. As an example, a filter member having a retention particle size of 0.5 to 3 $\mu$m, preferably 1 to 2.5 $\mu$m, may be used as the second filter member 6-2. The first filter can remove fat globules with relatively large sizes (3 $\mu$m or more), and then the second filter can remove fat globules with smaller size (less than 3 $\mu$m).

From the viewpoint of achieving fat globule removal effect, the length of the first filter member 6-1 in the upstream to downstream direction may preferably be 5 mm or more, more preferably 10 mm or more, even more preferably 15 mm or more, still more preferably 21 mm or more. On the other hand, from the viewpoint of being able to control the loss of sample liquid volume for testing, the length may preferably be 40 mm or less, more preferably 35 mm or less, even more preferably 32 mm or less, still more preferably 29 mm or less.

From the viewpoint of achieving fat globule removal effect, the length of the second filter member 6-2 in the upstream to downstream direction may preferably be 5 mm or more, more preferably 10 mm or more, even more preferably 14 mm or more. On the other hand, from the viewpoint of being able to control the loss of sample liquid volume for testing, the length may preferably be 40 mm or less, more preferably 30 mm or less, even more preferably 20 mm or less.

It has been found that in this embodiment, if the distance of the overlap part (represented by 100) between the first filter member 6-1 and the second filter member 6-2 (overlap distance X) is small, chromatographic development will be poor (Figures 25(A)). In order to obtain good chromatographic development, the distance X of the direct overlap between the first and second filter members may preferably be sufficient for the amount of liquid to be chromatographically developed. The amount of liquid to be chromatographically developed herein refers to the amount of liquid that can be absorbed by the membrane member for chromatographic development 3 and the absorption member 7. The amount of liquid to be chromatographically developed can be determined by subtracting the total weight of the membrane member for chromatographic development 3 and the absorption member 7 before chromatographic development from the total weight of the membrane member for chromatographic development 3 and the absorption member 7 after chromatographic development. A member that does not absorb liquid, e.g., the base material 8, may be included in the weighing. More specifically, the ratio of the overlap distance X to the total the amount of liquid that can be absorbed by the membrane member for chromatographic development 3 and the absorption member 7 (X/Y (mm/g)) may preferably be 30 or more, more preferably 70 or more, even more preferably 100 or more. On the other hand, if the overlap distance X is too long, the loss of the volume of the sample liquid for the testing will increase, and the sensitivity will decrease. Therefore, X/Y may preferably be 500 or less, more preferably 350 or less, even more preferably 150 or less.

[Membrane member for chromatographic development]

The membrane member for chromatographic development 3 is a component for carrying out chromatographic development on the membrane using the principle of chromatography. The membrane member for chromatographic development 3 may be made of any material as long as it allows for immunochromatography. Preferable examples include nitrocellulose, mixed nitrocellulose esters, polyvinylidene fluoride, and nylon. An example is a nitrocellulose membrane. The capture antibody may be immobilized on the membrane member for chromatographic development 3 to form a capture region 4. A substance that can bind to the labeling antibody may be immobilized on the membrane downstream of the capture region 4 to form a control site. The capture region and the control region can be formed on the membrane member for chromatographic development 3 in the form of, e.g., lines, which constitute a detection region 4.

[Labeling antibody-attached member]

The labeling antibody-attached member 1 is a member to which the labeling antibody is attached. The labeling antibody-attached member 1 may be arranged at any position as long as at least a portion thereof is immersed in the milk sample. The labeling antibody-attached member 1 may be arranged between the base material 8 and another member (e.g., the sample contact member 5 or the first filter member 6-1) (Figures 24 and 25). Alternatively, as long as a portion thereof is arranged in the immersing region, it may be arranged on the sample contact member 5, on the first filter member 6-1, on the bacteriolysis agent-attached member 2, or on the cover member 11, or may be arranged on the back side of the base material 8.

The labeling antibody-attached member 1 may be arranged in the immersing region 9 of the chromatographic device 10, whereby when the chromatographic device 10 is used, at least a portion of the labeling antibody-attached member 1 is immersed in the milk sample, a portion of the labeling antibody, which is attached in an elutable manner, elutes and diffuses into the milk sample. This causes an antigen-labelling antibody reaction in the milk sample, thereby ensuring a sufficiently long reaction time to increase the amount of complex formed from the substance to be detected and the labeling antibody. The sufficiently long reaction time increases the amount of complex formed from the antigen and the labeling antibody. In the dipstick-type immunochromatographic device, the labeling antibody forms a complex in the test liquid, and the complex is subjected to chromatographic development. This may result in clogging of the membrane member for chromatographic development 3 due to fat globules contained in the milk sample, making adjustment of the filter members 6 particularly important.

The labeling antibody-attached member 1 may be made of any material as long as it allows for attachment of the labeling antibody. The labeling antibody may preferably be attached to the labeling antibody-attached member 1 so as to be elutable. The labeling antibody-attached member 1 may be produced by impregnating a water-absorbent member with a solution containing the labeling antibody, optionally followed by drying. Examples of water-absorbent members include fiber aggregates such as paper, woven fabric, and non-woven fabric, and porous materials such as sponge. According to another embodiment, the labeling antibody-attached member 1 may be produced by applying or adhering a solution containing the labeling antibody directly to a member of the immunochromatographic device 10. When adhered, the labeling antibody-attached member 1 may be mixed with a thickener to prepare a water-soluble adhesive material. From the viewpoint of improving storage stability, the labeling antibody-attached member may also contain sugar such as sucrose or trehalose.

The amount of the labeling antibody attached may be selected as appropriate depending on various conditions, such as the volume of the sample liquid and the labeling antibody's ability to form a complex with the target substance. As an example, it is possible to use an antibody solution with an antibody concentration prepared so that when the antibody solution is diluted 21-fold, the absorbance of the resulting diluted solution at a wavelength of 540 nm is 0.1 to 2.0.

[Bacteriolysis agent-attached member]

The bacteriolysis agent-attached member 2 is a member to which the bacteriolysis agent is attached. The bacteriolysis agent-attached member 2 may be arranged at any position as long as at least a portion thereof is immersed in the milk sample. The bacteriolysis agent-attached member 2 may be arranged between the base material 8 and another member (e.g., the sample contact member 5 or the first filter member 6-1) (Figures 24, 25(A), 25(B), 26(A), and 26(B)). Alternatively, as long as a portion thereof is arranged in the immersing region 9, it may be arranged on the sample contact member 5, on the first filter member 6-1, on the labeling antibody-attached member 1, or on the cover member 11, or may be arranged on the back side of the base material 8. The labeling antibody-attached member 1 and the bacteriolysis agent-attached member 2 are both arranged in the immersing region 9. They may be arranged to be in contact with each other or apart from each other. When the labeling antibody-attached member 1 and the bacteriolysis agent-attached member 2 are in contact with each other, their wall surfaces may be in contact, or they may overlap in whole or in part. As an example, the bacteriolysis agent-attached member 2 may be arranged so as to span over the base material 8 and the labeling antibody-attached member 1 (Figure 26(C)). In this case, the bacteriolysis agent-attached member 2 may cover only a portion of the labeling antibody-attached member 1, or as shown in Figure 26(C), it may completely cover the labeling antibody-attached member 1. On the bacteriolysis agent-attached member 2, the sample contact member 5 may be arranged (Figures 24), or the first filter member 6-1 may be arranged (Figures 25(A), 25(B), 26(A), 26(B), and 26(C)). In order to detect bacteria in milk with high sensitivity, it is preferable to lysis the bacteria to extract the target substance to be detected. When a bacteriolysis enzyme is used as the bacteriolysis agent, a bacteriolysis reaction by the enzyme takes tens of seconds to several minutes. Therefore, if the bacteriolysis reaction and the detection operation with the immunochromatographic device are performed simultaneously, the detection signal will rise after several minutes because it takes tens of seconds to several minutes from the start of the detection operation until the substance to be detected is extracted. Therefore, it is important for highly sensitive detection of bacteria that the sample liquid in the immunochromatographic device is deployed smoothly, even after several minutes, without causing blockage.

The bacteriolysis agent-attached member 2 may be made of any material as long as it allows for the bacteriolysis agent to be attached so as to be elutable. The bacteriolysis agent-attached member 2 may be produced by impregnating a water-absorbent member with a solution containing the bacteriolysis agent, optionally followed by drying. Examples of water-absorbent members include fiber aggregates such as paper, woven fabric, and non-woven fabric, and porous materials such as sponge. According to another embodiment, the bacteriolysis agent-attached member 2 may be produced by applying or adhering a solution containing the bacteriolysis agent directly to a member of the immunochromatographic device 10. When adhered, the bacteriolysis agent-attached member 2 may be mixed with a thickener to prepare a water-soluble adhesive material. When the bacteriolysis agent-attached member 2 is provided in the immunochromatographic device 10, may be arranged at any position on the immunochromatographic device 10 as long as when the immunochromatographic device is used, at least a portion of the bacteriolysis agent-attached member 2 is immersed in the milk sample. The labeling antibody-attached member 1 and the bacteriolysis agent-attached member 2 may be formed as a single member, by impregnating a water-absorbent member with a solution containing the labeling antibody and the bacteriolysis agent.

[Sample contact member]

The sample contact member 5 is a member to be in contact with the milk sample, and may be any member through which the solution can pass. Examples of materials for the sample contact member 5 include fiber aggregates such as filter paper, cloth, or non-woven fabric. Examples of fibers that can be used for the sample contact member 5 include chemical fibers such as fiber of cellulose derivatives, glass fiber, and polyester fiber, as well as natural fibers such as cotton. The sample contact member 5 may be omitted. In this case, the first filter member 6-1 may be exposed at its immersing region, and substitutes for the sample contact member 5 (Figures 25(A), 25(B), 26(A), 26(B), and 26(C)).

[Absorption member]

The absorption member 7 is a water-absorbent member located at the downstream end for absorbing the chromatographically developed solution downstream to carry out chromatographic development continuously. The absorption member 7 may be any water-absorbent material, such as cotton, cloth, paper, etc. It may span over a portion of the downstream end of the membrane member for chromatographic development 3 and the base material 8.

[Base material]

The base material 8 is a thin layer of water-impermeable material on which each component is arranged in the manufacture of the immunochromatographic device 10. It may be made of, e.g., polystyrene, and may be coated with an adhesive substance to secure other members.

[Labeling antibody]

The labeling antibody is an antibody that is labeled for detection and forms a complex with any antigen contained in the milk sample via an antigen-antibody reaction. Such antigens may be proteins contained in milk or antigens derived from target bacteria that are released by lysis from bacteria contained in milk. The complex formed with the labeling antibody may also be referred to as a first complex. The type of detection label used for the labeling antibody is not restricted and may be selected as appropriate in accordance with the detection method. Specific examples include: metal colloids such as gold colloids, platinum colloids, and palladium colloids; non-metal colloids such as selenium colloids, alumina colloids, and silica colloids; insoluble granular materials such as colored resin particles, dye colloids, colored liposomes, etc.; enzymes that catalyze chromogenic reactions such as alkaline phosphatase, peroxidase, luciferase, etc.; fluorescent dyes, radioisotopes; and chemiluminescent labels, bioluminescent labels, electrochemiluminescent labels, etc. The method for attaching the label to the antibody is also not restricted, but specific examples of methods that can be used include physical adsorption using the hydrophobicity of the antibody, chemisorption using a functional group of the antibody, etc.

The first complex formed of the antigen as the target substance to be detected and the labeling antibody based on an antigen-antibody reaction is developed from the immersing region 9 to the detection region 4 of the immunochromatographic device 10.

[Capture antibody]

The capture antibody is an antibody that is immobilized in the detection region 4 of the immunochromatographic device 10 and forms a complex with the first complex based on an antigen-antibody reaction. The complex thus formed may also be referred to as a second complex. When used in immunochromatography, the capture antibody may more specifically be immobilized on the membrane member for chromatographic development 3, which is located in the detection area 4 of the immunochromatographic system 10. There are no restrictions on the method used to immobilize the antibody on the solid-phase material. Examples of specific methods include immobilization by physisorption using the hydrophobicity of the antibody and immobilization by chemical bonding using the functional groups of the antibody. From the viewpoint of suppressing sample-derived nonspecific reactions, when the capture antibody is immobilized on the membrane member for chromatographic development 3, a protein such as casein or BSA may also be immobilized as a blocking agent.

The second complex is held at the position where the capture antibody is immobilized via an antigen-antibody reaction with the immobilized capture antibody, and where the labeling of the labeling antibody is detected. If the label is detected at the site where the capture antibody is immobilized, then the target bacteria are detected in the sample solution.

[Bacteria as Detection Targets]

The bacteria as the target to be detected in this embodiment may be any bacteria. Example include bacteria belonging to the genus Escherichia, the genus Staphylococcus, the genus Klebsiella, the genus Proteus, the genus Enterococcus, the genus Citrobacter, the genus Pseudomonas, the genus Bacillus, the genus Serratia, the genus Rahnella, the genus Listeria, the genus Enterobacter, and the genus Salmonella, the genus Moraxella, the genus Aeromonas, the genus Lactobacillus, the genus Micrococcus, the genus Acinetobacter, the genus Campylobacter, the genus Vibrio, the genus Streptococcus, the genus Haemophilus, the genus Mycoplasma, the genus Legionella, the genus Bordetella, the genus Chlamydia, and the genus Neisseria, the genus Yersinia, the genus Erwinia, the genus Hafnia, the genus Morganella, the genus Providencia, the genus Shigella, and the genus Pectobacterium.

Bacteria belonging to the genus Escherichia include Escherichia coli (E. coli, EC) and Escherichia albertii. Preferred among these is Escherichia coli.

Bacteria belonging to the genus Staphylococcus include Staphylococcus aureus (Staphylococcus aureus, S. aureus, SA), S. epidermidis, and S. argenteus. Preferred among these is Staphylococcus aureus.

Bacteria belonging to the genus Klebsiella include Klebsiella pneumoniae (K. pneumoniae, KP), Klebsiella oxytoca (K. oxytoca), and Klebsiella aerogenes (K. aerogenes). Among these, it may be preferred to detect an antigen-antibody reaction with Klebsiella pneumoniae.

Bacteria belonging to the genus Proteus include Proteus milabilis (P.milabilis, PM), Proteus morganii (P. morganii), Proteus vulgaris (P. vulgaris), and Proteus rettgeri (P. rettgeri). Preferred among these is Proteus milabilis.

Bacteria belonging to the genus Enterococcus include Enterococcus faecalis and Enterococcus faecium. Preferred among these is Enterococcus faecalis.

Bacteria belonging to the genus Citrobacter include Citrobacter freundii (C. freundii, CF), Citrobacter amalonaticus (C. amalonaticus), and Citrobacter diversus (C. diversus). Preferred among these is Citrobacter freundii.

Bacteria belonging to the genus Pseudomonas include Pseudomonas aeruginosa (P. aeruginosa, PA), P. fluorescens, P. phosphorescence, and P. putida. Preferred among these is Pseudomonas aeruginosa.

Bacteria belonging to the genus Bacillus include Bacillus subtilis (B. subtilis, BS), Bacillus cereus (B. cereus), Bacillus licheniformis (B. licheniformis), and Bacillus megaterium (B. megaterium).

Bacteria belonging to the genus Serratia include Serratia liquefaciens (S. liquefaciens, SL), Serratia marcescens (S. marcescens), and Serratia fonticola (S. fonticola). Preferred among these is Serratia liquefaciens.

Bacteria belonging to the genus Rahnella include Rahnella aquatilis (R. aquatilis, RA), Rahnella victoriana (R.victoriana), and Rahnella bruchi (R. bruchi).

Bacteria belonging to the genus Listeria include Listeria monocytogenes (L. monocytogenes, LM), Listeria innocua (L. innocua) and Listeria ivanovii (L. ivanovii).

Bacteria belonging to the genus Enterobacter include Enterobacter cloacae (E. cloacae, ECL), Enterobacter aerogenes (E. aerogenes), and Enterobacter sakazakii (E. sakazakii).

Bacteria belonging to the genus Salmonella include Salmonella enteritidis (S. enteritidis, SE), Salmonella infantis (S. infantis), and Salmonella typhimurium (S. typhimurium).

[Bacteriolysis Agents]

The bacteriolysis agent may be any substance conventionally used in the art as long as it has the ability to lyse the target bacteria. Examples of bacteriolysis agents include bacteriolysis enzymes and surfactants. Any one bacteriolysis enzyme and/or surfactant may be used alone, or two or more surfactants and/or two or more bacteriolysis enzymes may be used in combination.

[Bacteriolysis enzyme]

Bacteriolysis enzymes may be any bacteriolysis enzymes used in the art as long as they can lyse the target bacteria. Bacteriolysis enzymes may be selected according to the type of target bacteria. Examples include lysozyme, lysostaphin, pepsin, glucosidase, galactosidase, achromopeptidase, $\beta$-N-acetylglucosaminidase, acetylglucotaminidase, and achromopeptidase. These enzymes may be naturally-occurring enzymes or genetically engineered enzymes. The amount of the bacteriolysis enzyme attached may be determined depending on the desired final concentration and the amount of the sample liquid. As an example, since the bacteriolysis enzyme may be used at a final concentration of 0.01 $\mu$g/mL to 100 mg/mL, the attached amount may be 0.01 $\mu$g to 100 mg, preferably 0.1 $\mu$g to 10 mg, more preferably 1 $\mu$g to 3 mg.

[Surfactant]

The surfactant may be selected according to the type of target bacteria. Examples of surfactants include nonionic surfactants, cationic surfactants, anionic surfactants, and zwitterionic surfactants, which may be used either singly or in combination of any two or more. The amount of the surfactant attached may be determined depending on the desired final concentration and the amount of the sample liquid. As an example, surfactant may be used at a final concentration of from 0.01% to 10%, the attached amount may be 0.1 to 100 mg, preferably 0.1 to 10 mg, more preferably 1 to 10 mg.

Examples of nonionic surfactants include, although are not limited to, ester-ether type, ester type, and ether type surfactants, all of which may preferably be used. More specific examples include polyoxyethylene alkyl ether, polyoxyethylene alkyl phenyl ether, fatty acid sorbitan ester, alkyl polyglucoside, fatty acid diethanol amide, alkyl monoglyceryl ether, and polysorbates (fatty acid sorbitan esters condensed with tens of molecules of ethylene oxide). Especially preferred examples are polysorbates, more specifically polyoxyethylene (20) sorbitan monolaurate, polyoxyethylene (60) sorbitan monostearate, polyoxyethylene (65) sorbitan tristearate, and polyoxyethylene (80) sorbitan monooleate, as well as polyoxyethylene alkyl phenyl ethers, more specifically polyoxyethylene (10) octyl phenyl ether, etc.

Examples of anionic surfactants include carboxylates, sulfonates, and sulfates, all of which may preferably be used. Specific examples include alkyl ether carboxylates, straight chain alkyl benzene sulfonates (LAS), α-olefin sulfonates (AOS), dialkyl sulfosuccinates, formaldehyde condensation products of naphthalene sulfonates, alkyl sulfates (AS), polyoxyethylene alkyl sulfates sulfated by adding ethylene oxide to higher alcohols (AES), and phosphates of higher alcohols or their ethylene oxide adducts. More specific examples include: sodium alkyl sulfates such as sodium dodecyl sulfate and sodium myristyl sulfate; sodium N-acyl sarcosinates such as sodium N-lauroyl sarcosinate and sodium N-myristoyl sarcosinate; N-acyl glutamates such as sodium dodecylbenzene sulfonate, sodium hydrogenated coconut fatty acid monoglyceride monosulfate, sodium lauryl sulfoacetate, and sodium N-palmitoyl glutamate; sodium N-methyl-N-acyl alanine, and sodium α-olefin sulfonate.

Examples of cationic surfactants include amine salts and quaternary ammonium salts, both of which may preferably be used. Specific examples include distearyldimethyl ammonium chloride, benzalkonium chloride, hexadecyltrimethyl ammonium bromide, hexadecyltrimethyl ammonium bromide, myristyltrimethyl ammonium bromide.

Examples of zwitterionic surfactants include, although are not limited to, amino acids (e.g., alkyl amino fatty acid salts), betaines (alkyl betaines), and amine oxides (e.g., alkylamine oxides). More specific examples include dimethyl ammoniopropane sulfonate, dodecyl dimethyl ammoniobutyrate, betaine laurylate, and amide propyl betaine.

[Detection of the target substance to be detected]

The method for detecting a target substance to be detected in a milk sample based on an antigen-antibody reaction using the immunochromatographic device 10 according to this embodiment includes:

immersing the immersing region 9 of the immunochromatographic device 10 in the milk sample; and detecting the second complex captured at the detection region 4 of the immunochromatographic device 10, based on the label of the labeling antibody.

When the immersing region 9 of the immunochromatographic device is immersed in the milk sample, at least a portion of the labeling antibody-attached member 1 is immersed, whereby the labeling antibody elutes into the milk sample. This allows antigen-antibody reactions to occur in the milk sample and ensures a longer reaction time, thus improving detection sensitivity. In addition, when the dipstick-type immunochromatographic device includes the bacteriolysis agent-attached member 2, in addition to the labeling antibody-attached member 1, at least a portion of the bacteriolysis agent-attached member 2 is immersed in the milk sample, whereby the labeling antibody and the bacteriolysis agent elute into the milk sample. The presence of the labeling antibody and the bacteriolysis agent in the milk sample allows the bacteriolysis reaction and antigen-antibody reaction to occur in the milk sample, prolonging the time for both reactions and thus improving detection sensitivity. Therefore, it is important for highly sensitive detection of bacteria that the sample liquid is developed in the immunochromatographic device smoothly, even after several minutes, without causing blockage. The immersion time may be long enough for the second complex to be captured by the capture antibody immobilized on the immunochromatographic device 10. The immersion time may be until the labeling antibody binds to the control region and the labeling is detected. In the capture region, where the antibody for capture is immobilized, the second complex can be formed and detected based on the labeling of the labeling antibody.

The detection method according to this embodiment may also include the step of agitating the detection solution (sample liquid) using the immunochromatographic device 10. Agitation can promote elution of the labeling antibody and the bacteriolysis into the sample liquid and promote bacteriolysis reaction and/or antibody-antigen reaction. After agitation, the immunochromatographic device 10 may be left to stand still, whereby the first complex formed via the antibody-antigen reaction may be, along with the sample liquid, developed on the membrane member for chromatographic development 3 via the sample contact member 5, the first filter member 6-1, and the second filter member 6-2 of the immunochromatographic device 10.

When the immunochromatographic device does not include the bacteriolysis agent-attached member 2, the detection method of target bacteria according to this embodiment may also include the step of adding a bacteriolysis agent to the sample liquid. The bacteriolysis agent may be arranged in the sample liquid retention container 20 of the dipstick-type immunochromatographic device in advance. In this case, the step of placing the sample liquid in the retention container also allows for the bacteriolysis agent to disperse in the sample liquid.

The step of detecting the second complex captured by the detection region 4 of the immunochromatographic device 10 based on the label of the labeling antibody detection can be carried out by any appropriate means depending on the type of the label. When gold colloid is used as the label, the coloration in the detection region can be confirmed with the naked eye. The coloration of the control area where the antigen that can bind to the labeling antibody is immobilized indicates that the labeling antibody has been properly developed on the membrane member for chromatographic development 3. On the other hand, if the filter members are not well adjusted, clogging may occur in the membrane member for chromatographic development 3. This can be judged by the absence of coloration of the control section, or by visual confirmation by the presence or absence of liquid wetting that the chromatographic membrane development of the milk sample has stopped halfway. Specifically, when chromatographic membrane development occurs, the membrane member for chromatographic development 3 and the absorption member 7 change color due to liquid wetting, but when chromatographic membrane development stops, no liquid wetting occurs. If the chromatographic development of the milk sample stops in the middle of the development, the detection performance will be compromised. It is therefore important to adjust the filter members properly such that the chromatographic development of the milk sample can be successfully performed.

All references cited herein are incorporated herein by reference in their entirety.

**EXAMPLES**

The examples of the present invention described below are for illustrative purposes only and do not limit the technical scope of the present invention. The technical scope of the present invention is limited only by the claims. The present invention may be modified, e.g., additions, deletions, and substitutions of the constituent requirements of the present invention may be made, provided that the intent of the present invention is not departed from.

[Example Group I: Immunochromatographic device according to the first embodiment]

*Test 1: Detection of Staphylococcus aureus using immunochromatographic devices

(1) Production of immunochromatographic devices for detecting Staphylococcus aureus

Immunochromatographic devices shown in the cross-sectional schematic views of Figures 1 to 10 were produced according to the following procedure.

(a) Production of ribosome protein L7/L12 antibodies

Monoclonal antibodies against Staphylococcus aureus ribosome protein L7/L12 for the detection of Staphylococcus aureus. According to the method described in Example 5 of WO2000/006603 A, Staphylococcus aureus L7/L12 ribosome protein was obtained, and monoclonal antibodies against the protein were produced. The monoclonal antibodies selected are a combination of two clones (SA-1 and SA-2) that can simultaneously bind to different sites of the L7/L12 ribosomal protein (i.e., that can form a sandwich-type complex with the L7/L12 antigen).

(b) Labeling antibody-attached members

Monoclonal antibody SA-2 was used as a labeling antibody, which forms a first complex with an antigen derived from the target bacterium based on an antigen-antibody reaction. 0.9 mL of gold colloid solution (60 nm particle size) manufactured by BBI International was mixed with 0.1 M potassium phosphate (pH 7.5), and then combined with 0.1 mL of 100 $\mu$g/mL monoclonal antibody SA-2 to be labeled with gold colloids and allowed to stand for 5 minutes at room temperature, to allow this antibody to bind to the surface of the gold colloidal particles. A 10% aqueous solution of bovine serum albumin (BSA) was then added so that the final concentration in the gold colloid solution was 1% to block the remaining surface of the gold colloidal particles with BSA, whereby a solution of gold colloid-labeled monoclonal antibody SA-2 (hereafter referred to as the "labeling antibody") was prepared. This solution was centrifuged (at 15000×rpm for 5 minutes) to precipitate the labeling antibody, and the supernatant solution was removed to obtain the labeling antibody. This labeling antibody was then suspended in 20mM tris hydrochloric acid buffer solution (pH 8.2) containing 0.25 % BSA and 2.5 % sucrose such that the absorbance at 540 nm wavelength becomes 0.5 when diluted 21 times, to thereby obtain a labeling antibody solution. A strip of glass fiber pad of 5 mm × 300 mm was impregnated with 1mL of the labeling antibody solution and dried under reduced pressure at room temperature to prepare a labeling antibody-impregnated member (hereinafter referred to as labeling antibody-impregnated member 1) as the labeling antibody-attached member 1.

(c) Bacteriolysis agent-attached members

Recombinant lysostaphin manufactured by Fujifilm Wako Pure Chemical Corporation was dissolved in 20 mM sodium acetate buffer (pH 4.5) containing 5% sucrose to a concentration of 100 $\mu$g/mL. A strip of glass fiber pad of 10 mm × 300 mm was impregnated with 1 mL of the enzyme solution and air dried at room temperature to prepare a bacteriolysis agent-attached member 2.

(d) Immobilization of capture antibodies in detection regions

A nitrocellulose membrane with a width of 25 mm and a length of 300 mm was prepared as the membrane carrier for chromatographic development 3. Monoclonal antibody SA-1 was used as a capture antibody, which forms a second complex with the first complex based on an antigen-antibody reaction. A solution containing 1.5 mg/mL of monoclonal antibody SA-1 was applied in a line at 1 μL/cm at a position of 10 mm from the end of the chromatographic development start point on the membrane carrier for chromatographic development 3. The membrane carrier was dried at 50°C for 30 minutes, then at room temperature overnight, to thereby form the capture site 4 for the complex formed of the Staphylococcus aureus ribosome protein L7/L12 antigen and the gold colloid-labeled antibody.

(e) Assembly of immunochromatographic devices

In addition to the labeling antibody-attached member 1, the bacteriolysis agent-attached member 2, and the membrane carrier for chromatographic development 3, cotton cloth was prepared as a sample contact member 5, and filter paper was used as an absorption member 7. These members were adhered to a base material 8 (254 μm in thickness, made of polystyrene, with adhesive material for attaching members applied on its surface), and a cover film (water-impermeable material) was used to cover the upper side of the figure (the side opposite the base material 8) except for the immersing region 9. This was cut into pieces of 5 mm in width to prepare immunochromatographic devices as shown in the cross-sectional views of Figures 1 to 10. A 10-mm portion of each immunochromatographic device on the upstream part was used as the immersing region 9.

(f) Preparation of reaction solutions

For the immunochromatographic devices of Figures 1 to 9, 0.1M MOPSO buffer solution (pH7.4) was mixed with 0.1% Tween20 as a surfactant for liquid development to prepare reaction solution for immunochromatography.
For the immunochromatographic device of Figure 10, which lacks any bacteriolysis agent attached, 0.1M MOPSO buffer solution (pH7.4) was mixed with 16 μg/mL (75 units/mL) lysostaphin as a bacteriolysis agent and 0.1% Tween20 as a surfactant for liquid development to prepare reaction solution for immunochromatography.

(g) Sample liquid retention containers

Microtubes with a 2-mL capacity made by Eppendorf were used as sample liquid retention containers 20.

(2) Detection of Staphylococcus aureus by immunochromatography

Sample solutions, i.e., normal saline with Staphylococcus aureus (ATCC25923 strain) at final concentrations of $1\times10^4$ cfu/mL, $1\times10^5$ cfu/mL, $1\times10^6$ cfu/mL, or normal saline with no bacteria, were each dispensed into microtubes 300μL each. The dispensed solution was then combined with 300μL of the reaction solution, and mixed at room temperature. For the immunochromatographic device of Figure 10, the immunochromatographic device was allowed to stand for 30 minutes before immersion.
The above immunochromatographic device was immersed in the mixed solution (sample liquid) from the sample contact member 5 such that the entire immersing region was immersed in the sample liquid. The immunochromatographic devices having the structures of Figures 1 and 2 in the state of being used are shown in Figures 20 and 21, respectively. The sample liquid was then agitated with the immunochromatographic device, allowed to stand at room temperature for 60 minutes to cause development, and then visually determined whether the complex of ribosomal protein L7/L12 antigen captured at the capture site 4 and the labeling antibody labeled with gold colloids was captured. For the immunochromatographic devices of Figures 4 and 10, the time since the sample liquid and the reaction solution was mixed until the complex was visually observed at the capture site 4 was measured and noted in parentheses in the table.

[Table 1-1]

|  | Example I-1 | Example I-2 | Example I-3 | Example I-4 | Example I-5 | Example I-6 |
|---|---|---|---|---|---|---|
| Structure | Figure 1 | Figure 2 | Figure 3 | Figure 4 | Figure 5 | Figure 6 |
| Bacterial conc. 1 e6 cfu/mL | Positive | Positive | Positive | Positive (5 min) | Positive | Positive |
| Bacterial conc. 1 e5 cfu/mL | Positive | Positive | Positive | Positive (15 min) | Positive | Positive |

(continued)

|  | Example I-1 | Example I-2 | Example I-3 | Example I-4 | Example I-5 | Example I-6 |
|---|---|---|---|---|---|---|
| Bacterial conc. 1e4 cfu/mL | Positive | Positive | Positive | Positive (40 min) | Positive | Positive |
| No bacteria | Negative | Negative | Negative | Negative | Negative | Negative |

[Table 1-2]

|  | Comparative Example I-1 | Comparative Example I-2 | Comparative Example I-3 | Comparative Example I-4 |
|---|---|---|---|---|
| Structure | Figure 7 | Figure 8 | Figure 9 | Figure 10 |
| Bacterial conc. 1 e6 cfu/mL | Positive | Positive | Positive | Positive (35 min) |
| Bacterial conc. 1 e5 cfu/mL | Positive | Positive | Positive | Positive (40 min) |
| Bacterial conc. 1e4 cfu/mL | Negative | Negative | Negative | Negative |
| No bacteria | Negative | Negative | Negative | Negative |

With the immunochromatographic devices having the structures shown in Figures 1 to 6, which were configured to immerse at least a portion of the bacteriolysis agent-attached member 2, to which the bacteriolysis enzyme used as a bacteriolysis agent is attached, and the labeling antibody-attached member 1 in the sample liquid, the bacteria were successfully detected even at a concentration of as low as 1e4 cfu/mL. On the other hand, with the immunochromatographic devices having the structures shown in Figures 7 to 10, which are configured not to immerse the labeling antibody-attached member 1 and/or the bacteriolysis agent-attached member 2 into the sample liquid, the bacteria were detected only at a concentration of 1e5 cfu/mL at the lowest. It was also found that the immunochromatographic device of Example I-4, which corresponds to the present invention, was able to detect the antigen to be detected in a shorter time than the immunochromatographic device of Comparative Example I-4, which corresponds to the prior art.

*Test 2: Detection of Escherichia coli by immunochromatography

(1) Production of immunochromatographic devices for detecting Escherichia coli

Immunochromatographic devices shown in the cross-sectional schematic views of Figures 1 to 10 were produced according to the following procedure.

(a) Production of ribosome protein L7/L12 antibodies

Monoclonal antibodies against Escherichia coli ribosome protein L7/L12 for the detection of Escherichia coli. According to the method described in Example 5 of WO2000/006603 A, Escherichia coli L7/L12 ribosome protein was obtained, and monoclonal antibodies against the protein were produced. The monoclonal antibodies selected are a combination of two clones (EC-1 and EC-2) that can simultaneously bind to different sites of the L7/L12 ribosomal protein.

(b) Labeling antibody-attached members

Monoclonal antibody EC-2 was used as a labeling antibody, which forms a first complex with an antigen derived from the target bacterium based on an antigen-antibody reaction. 0.9 mL of gold colloid solution (60 nm particle size) manufactured by BBI International was mixed with 0.1 M potassium phosphate (pH 7.5), and then combined with 0.1 mL of 100 $\mu$g/mL monoclonal antibody EC-2 to be labeled with gold colloids and allowed to stand for 5 minutes at room temperature, to allow this antibody to bind to the surface of the gold colloidal particles. A 10% aqueous solution of bovine serum albumin (BSA) was then added so that the final concentration in the gold colloid solution was 1% to block the remaining surface of the gold colloidal particles with BSA, whereby a solution of gold colloid-labeled monoclonal antibody EC-2 (hereafter referred to as the "labeling antibody") was prepared. This solution was centrifuged (at 15000×rpm for 5 minutes) to precipitate the labeling antibody, and the supernatant solution was removed to obtain the labeling antibody. This labeling antibody was then suspended in 20mM tris hydrochloric acid buffer solution (pH 8.2) containing 0.25 % BSA and 2.5 % sucrose such that the absorbance at 540 nm wavelength becomes 0.5 when diluted 21 times, to thereby obtain a labeling antibody solution. A strip of glass fiber pad of 5 mm × 300 mm was impregnated with 1mL of the labeling antibody solution and dried under reduced pressure at room temperature to prepare the labeling antibody-attached member 1.

(c) Bacteriolysis agent-attached members

Egg white-derived lysozyme from Fujifilm Wako Pure Chemicals was dissolved in 20 mM Tris hydrochloric acid buffer (pH 8.0) containing 5% sucrose to a concentration of 100 mg/mL. A 5 mm × 300 mm strip of glass fiber pad was impregnated with 1 mL of the enzyme solution and air-dried at room temperature to prepare a bacteriolysis agent-attached member 2.

(d) Immobilization of capture antibodies in detection regions

A nitrocellulose membrane with a width of 25 mm and a length of 300 mm was prepared as the membrane carrier for chromatographic development 3. Monoclonal antibody EC-1 was used as a capture antibody, which forms a second complex with the first complex based on an antigen-antibody reaction. A solution containing 1.5 mg/mL of monoclonal antibody EC-1 was applied in a line at 1 μL/cm at a position of 10 mm from the end of the chromatographic development start point on the membrane carrier for chromatographic development 3. The membrane carrier was dried at 50°C for 30 minutes, then at room temperature overnight, to thereby form the capture site 4 for the complex formed of the Escherichia coli ribosome protein L7/L12 antigen and the gold colloid-labeled antibody.

(e) Assembly of immunochromatographic devices

In addition to the labeling antibody-attached member 1, the bacteriolysis agent-attached member 2, and the membrane carrier for chromatographic development 3, cotton cloth was prepared as a sample contact member 5, and filter paper was used as an absorption member 7. These members were adhered to a base material 8 (254 μm in thickness, made of polystyrene, with adhesive material for attaching members applied on its surface), and a cover film (water-impermeable material) was used to cover the upper side of the figure (the side opposite the base material 8) except for the immersing region 9. This was cut into pieces of 5 mm in width to prepare immunochromatographic devices as shown in the cross-sectional views of Figures 1 to 10. A 10-mm portion of each immunochromatographic device on the upstream part was used as the immersing region 9.

(f) Preparation of reaction solutions

For the immunochromatographic devices of Figures 1 to 9, 0.1M MOPSO buffer solution (pH7.4) was mixed with 0.1% Tween20 as a surfactant for liquid development, to prepare reaction solution for immunochromatography.
For the immunochromatographic device of Figure 10, which lacks any bacteriolysis agent attached, 0.1M MOPSO buffer solution (pH7.4) was mixed with 9 mg/mL lysozyme as a bacteriolysis agent and 0.1% Tween20 as a surfactant for liquid development to prepare reaction solution for immunochromatography.

(g) Sample liquid retention containers

Microtubes with a 2-mL capacity made by Eppendorf were used as sample liquid retention containers 20.

(2) Detection of Escherichia coli by immunochromatography

Sample solutions, i.e., normal saline with Escherichia coli (ATCC25922 strain) at final concentrations of $1 \times 10^4$ cfu/mL, $1 \times 10^5$ cfu/mL, $1 \times 10^6$ cfu/mL, or normal saline with no bacteria, were each dispensed into microtubes 300μL each. The dispensed solution was then combined with 300μL of the reaction solution, and mixed at room temperature. For the immunochromatographic device of Figure 10, the immunochromatographic device was allowed to stand for 30 minutes before immersion.
The above immunochromatographic device was immersed in the mixed solution (sample liquid) from the sample contact member 5 such that the entire immersing region was immersed in the sample liquid. The sample liquid was then agitated with the immunochromatographic device, allowed to stand at room temperature for 60 minutes to cause development, and then visually determined whether the complex of ribosomal protein L7/L12 antigen captured at the capture site 4. For the immunochromatographic devices of Figures 4 and 10, the time since the sample liquid and the reaction solution was mixed until the complex was visually observed at the capture site 4 was measured and noted in parentheses in the table.

[Table 2-1]

|  | Example I-7 | Example I-8 | Example I-9 | Example I-10 | Example I-11 | Example I-12 |
|---|---|---|---|---|---|---|
| Structure | Figure 1 | Figure 2 | Figure 3 | Figure 4 | Figure 5 | Figure 6 |
| Bacterial conc. 1 e6 cfu/mL | Positive | Positive | Positive | Positive (10 min) | Positive | Positive |
| Bacterial conc. 1 e5 cfu/mL | Positive | Positive | Positive | Positive (15 min) | Positive | Positive |
| Bacterial conc. 1e4 cfu/mL | Positive | Positive | Positive | Positive (40 min) | Positive | Positive |
| No bacteria | Negative | Negative | Negative | Negative | Negative | Negative |

[Table 2-2]

|  | Comparative Example I-5 | Comparative Example I-6 | Comparative Example I-7 | Comparative Example I-8 |
|---|---|---|---|---|
| Structure | Figure 7 | Figure 8 | Figure 9 | Figure 10 |
| Bacterial conc. 1e6 cfu/mL | Positive | Positive | Positive | Positive (35 min) |
| Bacterial conc. 1e5 cfu/mL | Positive | Positive | Positive | Positive (40 min) |
| Bacterial conc. 1e4 cfu/mL | Negative | Negative | Negative | Negative |
| No bacteria | Negative | Negative | Negative | Negative |

With the immunochromatographic devices having the structures of Figures 1 to 6, which were configured to immerse the bacteriolysis agent-attached member 2, to which the bacteriolysis enzyme was attached as the bacteriolysis agent, and at least a portion of the labeling antibody-attached member 1 into the sample liquid, the bacteria were successfully detected even at a concentration of as low as 1e4 cfu/mL. On the other hand, with the immunochromatographic devices having the structures of Figures 7 to 10, which were configured not to immerse the labeling antibody-attached member 1 and/or the bacteriolysis agent-attached member 2 into the sample liquid, the bacteria were detected only at a concentration of 1e5 cfu/mL at the lowest. It was also found that the immunochromatographic device of Example I-10, which corresponds to the present invention, was able to detect the antigen to be detected in a shorter time than the immunochromatographic device of Comparative Example I-8, which corresponds to the prior art.

*Test 3: Detection of Streptococcus uberis by immunochromatography

(1) Production of immunochromatographic devices for detecting Streptococcus uberis

Immunochromatographic devices shown in the cross-sectional schematic views of Figures 1 to 9 were produced according to the following procedure.

(a) Production of ribosome protein L7/L12 antibodies

Monoclonal antibodies against Streptococcus uberis ribosome protein L7/L12 for the detection of Streptococcus uberis. According to the method described in Example 5 of WO2000/006603 A, Streptococcus uberis L7/L12 ribosome protein was obtained, and monoclonal antibodies against the protein were produced. The monoclonal antibodies selected are a combination of two clones (SU-1 and SU-2) that can simultaneously bind to different sites of the L7/L12 ribosomal protein.

(b) Labeling antibody-attached members

Monoclonal antibody SU-2 was used as a labeling antibody, which forms a first complex with an antigen derived from the target bacterium based on an antigen-antibody reaction. 0.9 mL of gold colloid solution (60 nm particle size) manufactured by BBI International was mixed with 0.1 M potassium phosphate (pH 7.5), and then combined with 0.1 mL of 100 $\mu$g/mL monoclonal antibody SU-2 to be labeled with gold colloids and allowed to stand for 5 minutes at room temperature, to allow this antibody to bind to the surface of the gold colloidal particles. A 10% aqueous solution of bovine serum albumin (BSA) was then added so that the final concentration in the gold colloid solution was 1% to block the remaining surface of the gold colloidal particles with BSA, whereby a solution of gold colloid-labeled monoclonal antibody SU-2 (hereafter referred to as the "labeling antibody") was prepared. This solution was centrifuged (at 15000$\times$rpm for 5 minutes) to precipitate the labeling antibody, and the supernatant solution was removed to obtain the labeling antibody. This labeling antibody was then suspended in 20mM tris hydrochloric acid buffer solution (pH 8.2) containing 0.25 % BSA and 2.5 % sucrose such that the absorbance at 540 nm wavelength becomes 0.5 when diluted 21 times, to thereby obtain a labeling antibody solution. A strip of glass fiber pad of 5 mm $\times$ 300 mm was impregnated with 1mL of the labeling antibody solution and dried under reduced pressure at room temperature to prepare the labeling antibody-attached member 1.

(c) Bacteriolysis agent-attached members

Labiase from Ozeki Co., Ltd. and egg white-derived lysozyme from Fujifilm Wako Pure Chemicals were dissolved in 20 mM Tris hydrochloric acid buffer (pH 8.0) containing 5% sucrose to a concentration of 100 mg/mL. A 5 mm $\times$ 300 mm strip of glass fiber pad was impregnated with 1 mL of the enzyme solution and air-dried at room temperature to prepare a bacteriolysis agent-attached member 2.

(d) Immobilization of capture antibodies in detection regions

A nitrocellulose membrane with a width of 25 mm and a length of 300 mm was prepared as the membrane carrier for chromatographic development 3. Monoclonal antibody SU-1 was used as a capture antibody, which forms a second complex with the first complex based on an antigen-antibody reaction. monoclonal antibody SU-1 1.5 mg/mL was applied in a line at 1 $\mu$L/cm at a position of 10 mm from the end of the chromatographic development start point on the membrane carrier for chromatographic development 3. The membrane carrier was dried at 50°C for 30 minutes, then at room temperature overnight, to thereby form the capture site 4 for the complex formed of the Streptococcus uberis ribosome protein L7/L12 antigen and the gold colloid-labeled antibody.

(e) Assembly of immunochromatographic devices

In addition to the labeling antibody-attached member 1, the bacteriolysis agent-attached member 2, and the membrane carrier for chromatographic development 3, cotton cloth was prepared as a sample contact member 5, and filter paper was used as an absorption member 7. These members were adhered to a base material 8 (254 $\mu$m in thickness, made of polystyrene, with adhesive material for attaching members applied on its surface), and a cover film (water-impermeable material) was used to cover the upper side of the figure (the side opposite the base material 8) except for the immersing region 9. This was cut into pieces of 5 mm in width to prepare immunochromatographic devices as shown in the cross-sectional views of Figures 1 to 9. A 10-mm portion of each immunochromatographic device on the upstream part was used as the immersing region 9.

(f) Preparation of reaction solutions

0.1M MOPSO buffer solution (pH6.3) was mixed with 0.1% Tween20 as a surfactant for liquid development to prepare reaction solution for immunochromatography.

(g) Sample liquid retention containers

Microtubes with a 2-mL capacity made by Eppendorf were used as sample liquid retention containers 20.

(2) Detection of Streptococcus uberis by immunochromatography

Sample solutions, i.e., normal saline with Streptococcus uberis (ATCC19436 strain) at final concentrations of $1 \times 10^4$ cfu/mL, $1 \times 10^5$ cfu/mL, $1 \times 10^6$ cfu/mL, or normal saline with no bacteria, were each dispensed into microtubes 300µL each. The dispensed solution was then combined with 300µL of the reaction solution, and mixed at room temperature. The above immunochromatographic device was immersed in the mixed solution (sample liquid) from the sample contact member 5 such that the entire immersing region was immersed in the sample liquid. The sample liquid was then agitated with the immunochromatographic device, allowed to stand at room temperature for 60 minutes to cause development, and then visually determined whether the complex of ribosomal protein L7/L12 antigen captured at the capture site 4.

Table 3-1]

|  | Example I-13 | Example I-14 | Example I-15 | Example I-16 | Example I-17 | Example I-18 |
|---|---|---|---|---|---|---|
| Structure | Figure 1 | Figure 2 | Figure 3 | Figure 4 | Figure 5 | Figure 6 |
| Bacterial conc. 1e6 cfu/mL | Positive | Positive | Positive | Positive | Positive | Positive |
| Bacterial conc. 1e5 cfu/mL | Positive | Positive | Positive | Positive | Positive | Positive |
| Bacterial conc. 1e4 cfu/mL | Positive | Positive | Positive | Positive | Positive | Positive |
| No bacteria | Negative | Negative | Negative | Negative | Negative | Negative |

[Table 3-2]

|  | Comparative Example I-9 | Comparative Example I-10 | Comparative Example I-11 |
|---|---|---|---|
| Structure | Figure 7 | Figure 8 | Figure 9 |
| Bacterial conc. 1e6 cfu/mL | Positive | Positive | Positive |
| Bacterial conc. 1e5 cfu/mL | Positive | Positive | Positive |
| Bacterial conc. 1e4 cfu/mL | Negative | Negative | Negative |
| No bacteria | Negative | Negative | Negative |

With the immunochromatographic devices having the structures of Figures 1 to 6, which were configured to immerse the bacteriolysis agent-attached member 2, to which the bacteriolysis enzyme was attached as the bacteriolysis agent, and at least a portion of the labeling antibody-attached member 1 into the sample liquid, the bacteria were successfully detected even at a concentration of as low as 1e4 cfu/mL. On the other hand, with the immunochromatographic devices having the structures of Figures 7 to 9, which were configured not to immerse the labeling antibody-attached member 1 and/or the bacteriolysis agent-attached member 2 into the sample liquid, the bacteria were detected only at a concentration of 1e5 cfu/mL at the lowest.

*Test 4: Detection of Staphylococcus aureus in milk by immunochromatography

(1) Production of immunochromatographic devices for detecting Staphylococcus aureus in milk

Immunochromatographic devices shown in the cross-sectional schematic views of Figures 11 to 13 were produced according to the following procedure.

(a) Production of ribosome protein L7/L12 antibodies

The combination of SA-1 and SA-2 selected in Test 1 was used.

(b) Labeling antibody-attached members

The labeling antibody-attached member 1 produced in Test 1 was used.

(c) Bacteriolysis agent-attached members

For Example I-19, the bacteriolysis agent-attached member 2 produced in Test 1 was used.
For Example I-20, recombinant lysostaphin from FujiFilm Wako Pure Chemicals was dissolved in 20 mM sodium acetate buffer (pH 4.5) containing 5% casein to a concentration of 50 μg/mL. A strip of glass fiber pad of 10 mm × 300 mm was impregnated with 2mL of the enzyme solution, and air dried at room temperature to prepare a bacteriolysis agent-attached member 2.

(d) Immobilization of capture antibodies in detection regions

The membrane carrier for chromatographic development 3 and the capture site 4 were produced in the same manner as in Test 1, except that 1% sucrose and 0.15% casein were added to the solution containing 1.5 mg/mL monoclonal antibody SA-1 to be applied to the membrane carrier for chromatographic development.

(e) Assembly of immunochromatographic devices

For Example I-19, in addition to the labeling antibody-attached member 1, the bacteriolysis agent-attached member 2, and the membrane carrier for chromatographic development 3, a 25-mm GF/DVA (a filter from Cytiva: made of glass fiber, 776 μm in thickness, retention particle size = 3.5 μm) was used as a sample contact member 5, a 15-mm GF/AVA (a filter from Cytiva: made of glass fiber, 299 μm in thickness, retention particle size = 1.7 μm) was used as a filter member 6, and filter paper was used as an absorption member 7. These members were adhered to a base material 8 (254 μm in thickness, made of polystyrene, with adhesive material for attaching members applied on its surface), and a cover film (water-impermeable material) was used to cover the upper side of the figure (the side opposite the base material 8) except for the immersing region 9. This was cut into pieces of 5 mm in width to prepare immunochromatographic device as shown in the cross-sectional view of Figure 11. A 10-mm portion of each immunochromatographic device on the upstream part was used as the immersing region 9.
Example I-20, in addition to the labeling antibody-attached member 1, the bacteriolysis agent-attached member 2, and the membrane carrier for chromatographic development 3, a 25-mm GF/DVA (a filter from Cytiva: made of glass fiber, 776 μm in thickness, retention particle size = 3.5 μm) was used as a sample contact member 5, a 15-mm GF/AVA (a filter from Cytiva: made of glass fiber, 299 μm in thickness, retention particle size = 1.7 μm) was used as a filter member 6, and filter paper was used as an absorption member 7. The same procedure as in Example I-19 was followed except that the bacteriolysis agent-attached member 2 was adhered onto the labeling antibody-attached member 1, to prepare immunochromatographic device as shown in the cross-sectional view of Figure 12. A 10-mm portion of each immunochromatographic device on the upstream part was used as the immersing region 9.
For Comparative Example I-12, in addition to the labeling antibody-attached member 1 and the membrane carrier for chromatographic development 3, a 20-mm GF/DVA(a filter from Cytiva: made of glass fiber, 776 μm in thickness, retention particle size = 3.5 μm) was used as a sample contact member 5, and a 15-mm GF/AVA (a filter from Cytiva: made of glass fiber, 299 μm in thickness, retention particle size = 1.7 μm) was used as a filter member 6, and filter paper was used as an absorption member 7. to prepare immunochromatographic device as shown in the cross-sectional view of Figure 13. A 10-mm portion of each immunochromatographic device on the upstream part was used as the immersing region 9.

(f) Preparation of reaction solutions

For Examples 1-19 and 1-20, 0.1M MOPSO buffer solution (pH7.4) was mixed with 1% Tween20 as a surfactant for liquid development to prepare reaction solution for immunochromatography.
For the immunochromatographic device of Comparative Example I-12, which lacks any bacteriolysis agent attached, 0.1M MOPSO buffer solution (pH7.4) was mixed with 16 μg/mL (75units/mL) of lysostaphin as a bacteriolysis agent and 11% Tween20 as a surfactant for liquid development to prepare reaction solution for immunochromatography.

(g) Sample liquid retention containers

Microtubes with a 2-mL capacity made by Eppendorf were used as sample liquid retention containers 20.

(2) Detection of Staphylococcus aureus by immunochromatography

Sample solutions, i.e., milk combined with normal saline with Staphylococcus aureus (ATCC25923 strain) at final concentrations of $1 \times 10^4$ cfu/mL, $1 \times 10^5$ cfu/mL, $1 \times 10^6$ cfu/mL, or milk combined with normal saline with no bacteria, were each dispensed into microtubes 300μL each. The dispensed solution was then combined with 300μL of the reaction solution, and mixed at room temperature. The milk used was a commercial product. For the immunochromatographic device of Figure 13, the immunochromatographic device was allowed to stand for 30 minutes before immersion. The above immunochromatographic device was immersed in the mixed solution (sample liquid) from the sample contact member 5 such that the entire immersing region was immersed in the sample liquid. The sample liquid was then agitated with the immunochromatographic device, allowed to stand at room temperature for 60 minutes for Examples I-19 and I-20 and for 30 minutes for Comparative Example I-12 to cause development, and then visually determined whether the complex of ribosomal protein L7/L12 antigen captured at the capture site 4 and the labeling antibody labeled with gold colloids was captured. For the immunochromatographic devices of Figures 12 and 13, the time since the sample liquid and the reaction solution was mixed until the complex was visually observed at the capture site 4 was measured.

[Table 4]

|  | Example I-19 | Example I-20 | Comparative Example I-12 |
|---|---|---|---|
| Structure | Figure 11 | Figure 12 | Figure 13 |
| Bacterial conc. 1e6 cfu/mL | Positive | Positive (10 min) | Positive (35 min) |
| Bacterial conc. 1e5 cfu/mL | Positive | Positive (15 min) | Positive (40 min) |
| Bacterial conc. 1e4 cfu/mL | Positive | Positive (30 min) | Negative |
| No bacteria | Negative | Negative | Negative |

With the immunochromatographic devices having the structures shown in Figures 11 and 12, the bacteria at a concentration of 1e4 cfu/mL in milk were successfully detected with high sensitivity. It was also found that the immunochromatographic device of Example I-20, which corresponds to the present invention, was able to detect the antigen to be detected in a shorter time than the immunochromatographic device of Comparative Example I-12, which corresponds to the prior art.

*Test 5: Detection of Escherichia coli in milk by immunochromatography

(1) Production of immunochromatographic devices for detecting Escherichia coli in milk

Immunochromatographic devices shown in the cross-sectional schematic views of Figures 10, 14, and 13 were produced according to the following procedure.

(a) Production of ribosome protein L7/L12 antibodies

The combination of EC-1 and EC-2 selected in Test 2 was used.

(b) Labeling antibody-attached members

For Example I-21, the labeling antibody-attached member 1 produced in Test 2 was used.
For Example I-22, monoclonal antibody EC-2 was used as a labeling antibody, which forms a first complex with an antigen derived from the target bacterium based on an antigen-antibody reaction. 0.9 mL of gold colloid solution (60 nm particle size) manufactured by BBI International was mixed with 0.1 M potassium phosphate (pH 7.5), and then combined with 0.1 mL of 100 μg/mL monoclonal antibody EC-2 to be labeled with gold colloids and allowed to stand for 5 minutes at room temperature, to allow this antibody to bind to the surface of the gold colloidal particles. A 10% aqueous solution of bovine serum albumin (BSA) was then added so that the final concentration in the gold colloid solution was 1% to block the remaining surface of the gold colloidal particles with BSA, whereby a solution of gold colloid-labeled monoclonal antibody EC-2 (hereafter referred to as the "labeling antibody") was prepared. This solution was centrifuged (at 15000×rpm for 5 minutes) to precipitate the labeling antibody, and the supernatant solution was removed to obtain the labeling antibody. This labeling antibody was then suspended in 20mM tris hydrochloric acid buffer solution (pH 8.2) containing 0.25 % BSA and 2.5 % sucrose such that the absorbance at 540 nm wavelength becomes 0.5 when diluted 21 times, to thereby obtain a labeling antibody solution. A strip of glass fiber pad of 5 mm × 300 mm was impregnated with 2mL of the labeling antibody solution, and dried under reduced pressure at room temperature to prepare the labeling antibody-attached member 1.

(c) Bacteriolysis agent-attached members

For Examples I-21 and I-22, the bacteriolysis agent-attached member 2 produced in Test 2 was used.

(d) Immobilization of capture antibodies in detection regions

The membrane carrier for chromatographic development 3 and the capture site 4 were produced in the same manner as in Test 1, except that 2% sucrose and 0.1% casein were added to the solution containing 1.5 mg/mL monoclonal antibody EC-1 to be applied to the membrane carrier for chromatographic development.

(e) Assembly of immunochromatographic devices

For Example I-21, in addition to the labeling antibody-attached member 1, the bacteriolysis agent-attached member 2, and the membrane carrier for chromatographic development 3, a 25-mm GF/DVA (a filter from Cytiva: made of glass fiber, 776 $\mu$m in thickness, retention particle size = 3.5 $\mu$m) was used as a sample contact member 5 and a 15-mm GF/AVA (a filter from Cytiva: made of glass fiber, 299 $\mu$m in thickness, retention particle size = 1.7 $\mu$m) was used as a filter member 6, and filter paper was used as an absorption member 7. These members were adhered to a base material 8 (254 $\mu$m in thickness, made of polystyrene, with adhesive material for attaching members applied on its surface), and a cover film (water-impermeable material) was used to cover the upper side of the figure (the side opposite the base material 8) except for the immersing region 9. This was cut into pieces of 5 mm in width to prepare immunochromatographic device as shown in the cross-sectional view of Figure 11. A 10-mm portion of each immunochromatographic device on the upstream part was used as the immersing region 9.

For Example I-22, in addition to the labeling antibody-attached member 1, the bacteriolysis agent-attached member 2, and the membrane carrier for chromatographic development 3, a 25-mm GF/DVA (a filter from Cytiva: made of glass fiber, 776 $\mu$m in thickness, retention particle size = 3.5 $\mu$m) was used as a sample contact member 5 and a 15-mm GF/AVA (a filter from Cytiva: made of glass fiber, 299 $\mu$m in thickness, retention particle size = 1.7 $\mu$m) was used as a filter member 6, and filter paper was used as an absorption member 7. The same procedure as in Example I-19 was followed except that the bacteriolysis agent-attached member 2 was adhered onto the labeling antibody-attached member 1, whereby the immunochromatographic kit shown in the cross-sectional view of Figure 14 was produced. A 10-mm portion of each immunochromatographic device on the upstream part was used as the immersing region 9.

For Comparative Example I-13, in addition to the labeling antibody-attached member 1, the membrane carrier for chromatographic development 3, 20-mm GF/DVA(a filter from Cytiva: made of glass fiber, 776 $\mu$m in thickness, retention particle size = 3.5 $\mu$m)was used as a sample contact member 5 and a 15-mm GF/AVA (a filter from Cytiva: made of glass fiber, 299 $\mu$m in thickness, retention particle size = 1.7 $\mu$m) was used as a filter member 6, and filter paper was used as an absorption member 7, whereby the immunochromatographic device as shown in the cross-sectional view of Figure 13 was produced. A 10-mm portion of each immunochromatographic device on the upstream part was used as the immersing region 9.

(f) Preparation of reaction solutions

For Examples I-21 and I-22, 0.1M MOPSO buffer solution (pH7.4) was mixed with 1% Tween20 as a surfactant for liquid development to prepare reaction solution for immunochromatography.

For the immunochromatographic device of Comparative Example I-13, which lacks any bacteriolysis agent attached, 0.1M MOPSO buffer solution (pH7.4) was mixed with 9 mg/mL of lysozyme as a bacteriolysis agent and 1% Tween20 as a surfactant for liquid development to prepare reaction solution for immunochromatography.

(g) Sample liquid retention containers

Microtubes with a 2-mL capacity made by Eppendorf were used as sample liquid retention containers 20.

(2) Detection of Escherichia coli in milk by immunochromatography

Sample solutions, i.e., milk combined with normal saline with Escherichia coli (ATCC25922 strain) at final

concentrations of $1 \times 10^4$ cfu/mL, $1 \times 10^5$ cfu/mL, $1 \times 10^6$ cfu/mL, or milk combined with normal saline with no bacteria, were each dispensed into microtubes 300μL each. The dispensed solution was then combined with 300μL of the reaction solution, and mixed at room temperature. The milk used was a commercial product. For the immunochromatographic device of Figure 13, the immunochromatographic device was allowed to stand for 30 minutes before immersion. The above immunochromatographic device was immersed in the mixed solution (sample liquid) from the sample contact member 5 such that the entire immersing region was immersed in the sample liquid. The sample liquid was then agitated with the immunochromatographic device, and allowed to stand at room temperature for 60 minutes for Examples I-21 and I-22, and for 30 minutes for Comparative Example I-13 to cause development, and then visually determined whether the complex of ribosomal protein L7/L12 antigen captured at the capture site 4 and the labeling antibody labeled with gold colloids was captured. For Figure 14 and the immunochromatographic device of Figure 13, the time since the sample liquid and the reaction solution was mixed until the complex was visually observed at the capture site 4 was measured.

[Table 5]

|  | Example I-21 | Example I-22 | Comparative Example I-13 |
|---|---|---|---|
| Structure | Figure 11 | Figure 14 | Figure 13 |
| Bacterial conc. 1e6 cfu/ml | Positive | Positive (10 min) | Positive (35 min) |
| Bacterial conc. 1e5 cfu/ml | Positive | Positive (25 min) | Positive (40 min) |
| Bacterial conc. 1e4 cfu/ml | Positive | Positive (50 min) | Negative |
| No bacteria | Negative | Negative | Negative |

With the immunochromatographic devices having the structures shown in Figures 11 and 14, the bacteria at a concentration of 1e4 cfu/mL in milk were successfully detected with high sensitivity. It was also found that the immunochromatographic device of Example I-22, which corresponds to the present invention, was able to detect the antigen to be detected in a shorter time than the immunochromatographic device of Comparative Example I-13, which corresponds to the prior art.

*Test 6: Detection of Staphylococcus aureus, Escherichia coli, and Streptococcus uberis in milk by immunochromatography

(1) Production of immunochromatographic devices for detecting Staphylococcus aureus in milk

For Example I-23, the members produced for Example I-19 in Test 4 were used to assemble the immunochromatographic device shown in the cross-sectional view of Figure 15. For Example I-24, the immunochromatographic device assembled for Example I-19 of Test 4 was used. For Example I-25, the immunochromatographic device assembled for Example I-20 of Test 4 was used. The reaction solutions and the sample retention containers were the same as those produced and used in Test 4.

(2) Detection of Staphylococcus aureus by immunochromatography

Sample solutions, i.e., milk combined with normal saline with Staphylococcus aureus (ATCC25923 strain) at final concentrations of $1 \times 10^4$ cfu/mL, or milk combined with normal saline with no bacteria, were each dispensed into microtubes 300μL each. The dispensed solution was then combined with 300μL of the reaction solution, and mixed at room temperature. The milk used was a commercial product. The above immunochromatographic device was immersed in the mixed solution (sample liquid) from the sample contact member 5 such that the entire immersing region was immersed in the sample liquid. Then, two conditions were prepared for each of Examples I-23, I-24, and I-25; under the first condition, the sample liquid was agitated with the immunochromatographic device, while under the second condition, the sample liquid was not agitated. The sample was allowed to stand at room temperature for 60 minutes to cause development, and then visually determined whether the complex of ribosomal protein L7/L12 antigen captured at the capture site 4 and the labeling antibody labeled with gold colloids was captured.

[Table 6-1]

| *Detection of Staphylococcus aureus in milk | | | |
|---|---|---|---|
| | Example I-23 | Example I-24 | Example I-25 |
| Structure | Figure 15 | Figure 11 | Figure 12 |
| Bacterial conc. 1e4 cfu/mL w/ agitation | Positive | Positive | Positive |
| Bacterial conc. 1e4 cfu/mL w/o agitation | Negative | Positive | Positive |
| No bacteria w/ agitation | Negative | Negative | Negative |
| No bacteria w/o agitation | Negative | Negative | Negative |

(3) Production of immunochromatographic devices for detecting Streptococcus uberis in milk

Immunochromatographic devices shown in the cross-sectional schematic views of Figures 16, 17, and 18 were produced according to the following procedure.

(a) Production of ribosome protein L7/L12 antibodies

The combination of SU-1 and SU-2, which was selected in Test 3, was used.

(b) Labeling antibody-attached members

For Example I-26, the labeling antibody-attached member 1 produced in Test 3 was used.
The labeling antibody-attached member 1 to be used for Examples I-27 and I-28 was produced as follows. Monoclonal antibody SU-2 was used as a labeling antibody, which forms a first complex with an antigen derived from the target bacterium based on an antigen-antibody reaction. 0.9 mL of gold colloid solution (60 nm particle size) manufactured by BBI International was mixed with 0.1 M potassium phosphate (pH 7.5), and then combined with 0.1 mL of 100 $\mu$g/mL monoclonal antibody SU-2 to be labeled with gold colloids and allowed to stand for 5 minutes at room temperature, to allow this antibody to bind to the surface of the gold colloidal particles. A 10% aqueous solution of bovine serum albumin (BSA) was then added so that the final concentration in the gold colloid solution was 1% to block the remaining surface of the gold colloidal particles with BSA, whereby a solution of gold colloid-labeled monoclonal antibody SU-2 (hereafter referred to as the "labeling antibody") was prepared. This solution was centrifuged (at 15000$\times$rpm for 5 minutes) to precipitate the labeling antibody, and the supernatant solution was removed to obtain the labeling antibody. This labeling antibody was then suspended in 20mM tris hydrochloric acid buffer solution (pH 8.2) containing 0.25 % BSA and 2.5 % sucrose such that the absorbance at 540 nm wavelength becomes 0.5 when diluted 21 times, to thereby obtain a labeling antibody solution. A strip of glass fiber pad of 5 mm $\times$ 300 mm was impregnated with 2mL of the labeling antibody solution, and then dried at room temperature under reduced pressure to thereby produce the labeling antibody-attached member 1-2.

(c) Bacteriolysis agent-attached members

Labiase from Ozeki Co., Ltd. was dissolved in McIlvaine buffer solution (pH4.0) containing 5% sucrose to a concentration of 30 mg/mL. A 5 mm $\times$ 300 mm strip of glass fiber pad was impregnated with 1mL of the enzyme solution, and then dried in air at room temperature to produce the bacteriolysis enzyme-attached member 2-1.
Lysozyme from Creative Enzymes was dissolved in 20 mM Tris hydrochloric acid buffer solution (pH8.0) containing 5% of sucrose to a concentration of 50 mg/mL. A 5 mm $\times$ 300 mm strip of glass fiber pad was impregnated with 1mL of the enzyme solution, and then dried in air at room temperature to produce the bacteriolysis enzyme-attached member 2-2.

(d) Immobilization of capture antibodies in detection regions

A nitrocellulose membrane with a width of 25 mm and a length of 300 mm was prepared as the membrane carrier for chromatographic development 3. Monoclonal antibody SU-1 was used as a capture antibody, which forms a second complex with the first complex based on an antigen-antibody reaction. A solution containing 1% sucrose, 0.1% casein, and 1.5 mg/mL of monoclonal antibody SU-1 was applied in a line at 1 $\mu$L/cm at a position of 10 mm from the end of the chromatographic development start point on the membrane carrier for chromatographic development 3. The membrane carrier was dried at 50°C for 30 minutes, then at room temperature overnight, to thereby form the capture site 4 for the complex formed of the Streptococcus uberis ribosome protein L7/L12 antigen and the gold colloid-labeled antibody.

(e) Assembly of immunochromatographic devices

For Example I-26, in addition to the labeling antibody-attached member 1, the bacteriolysis agent-attached members 2-1 and 2-2, and the membrane carrier for chromatographic development 3, a 32-mm GF/DVA (a filter from Cytiva: made of glass fiber, 776 $\mu$m in thickness, retention particle size = 3.5 $\mu$m) was used as a sample contact member 5, a 16-mm GF/AVA (a filter from Cytiva: made of glass fiber, 299 $\mu$m in thickness, retention particle size = 1.7 $\mu$m) was used as a filter member 6, and filter paper was used as an absorption member 7. These members were adhered to a base material 8 (254 $\mu$m in thickness, made of polystyrene, with adhesive material for attaching members applied on its surface), and a cover film (water-impermeable material) was used to cover the upper side of the figure (the side opposite the base material 8) except for the immersing region 9. This was cut into pieces of 5 mm in width to prepare the immunochromatographic device as shown in the cross-sectional view of Figure 16. A 10-mm portion of each immunochromatographic device on the upstream part was used as the immersing region 9.

For Example I-27, the same procedure as in Example I-26 was followed except that the labeling antibody-attached member 1-2 was used, whereby the immunochromatographic device as shown in the cross-sectional view of Figure 17 was produced.

For Example I-28, the same procedure as in Example I-26 was followed except that the labeling antibody-attached member 1-2 was used, whereby the immunochromatographic device as shown in the cross-sectional view of Figure 18 was produced.

A 10-mm portion of each immunochromatographic device produced in Examples I-26, I-27, and I-28 on the upstream part was used as the immersing region 9.

(f) Preparation of reaction solutions

0.1M MOPSO buffer solution (pH6.3) was mixed with 1% Tween20 as a surfactant for liquid development to prepare reaction solution for immunochromatography.

(g) Sample liquid retention containers

Microtubes with a 2-mL capacity made by Eppendorf were used as sample liquid retention containers 20.

(4) Detection of Streptococcus uberis in milk by immunochromatography

Sample solutions, i.e., normal saline with Streptococcus uberis (ATCC19436 strain) at final concentrations of $1 \times 10^4$ cfu/mL, or normal saline with no bacteria, were each dispensed into microtubes 300$\mu$L each. The dispensed solution was then combined with 300$\mu$L of the reaction solution, and mixed at room temperature. The above immunochromatographic device was immersed in the mixed solution (sample liquid) from the sample contact member 5 such that the entire immersing region was immersed in the sample liquid. Then, two conditions were prepared for each of Examples I-26, I-27, and I-28; under the first condition, the sample liquid was agitated with the immunochromatographic device, while under the second condition, the sample liquid was not agitated. The sample was allowed to stand at room temperature for 60 minutes to cause development, and then visually determined whether the complex of ribosomal protein L7/L12 antigen captured at the capture site 4 and the labeling antibody labeled with gold colloids was captured.

[Table 6-2]

| *Detection of Streptococcus uberis in milk | | | |
|---|---|---|---|
| | Example I-26 | Example I-27 | Example I-28 |
| Structure | Figure 16 | Figure 17 | Figure 18 |
| Bacterial conc. 5e4 cfu/mL w/ agitation | Positive | Positive | Positive |
| Bacterial conc. 5e4 cfu/mL w/o agitation | Negative | Positive | Positive |
| No bacteria w/ agitation | Negative | Negative | Negative |
| No bacteria w/o agitation | Negative | Negative | Negative |

(1) Production of immunochromatographic devices for detecting Escherichia coli in milk

For Example I-29, the members produced in Example I-21 of Test 5 were used to assemble the immunochromatographic device shown in the cross-sectional view of Figure 15. For Example I-30, the members produced in Example I-22 of Test 5 were used to assemble the immunochromatographic device shown in the cross-sectional view of Figure 19. The reaction solutions and the sample retention containers were the same as prepared and used in Test 5.

(2) Detection of Escherichia coli in milk by immunochromatography

Sample solutions, i.e., milk combined with normal saline with Escherichia coli (ATCC25922 strain) at final concentrations of $1 \times 10^4$ cfu/mL, or milk combined with normal saline with no bacteria, were each dispensed into microtubes 300μL each. The dispensed solution was then combined with 300μL of the reaction solution, and mixed at room temperature. The milk used was a commercial product. The above immunochromatographic device was immersed in the mixed solution (sample liquid) from the sample contact member 5 such that the entire immersing region was immersed in the sample liquid. Then, two conditions were prepared for each of Examples I-29 and I-30; under the first condition, the sample liquid was agitated with the immunochromatographic device, while under the second condition, the sample liquid was not agitated. The sample was allowed to stand at room temperature for 60 minutes to cause development, and then visually determined whether the complex of ribosomal protein L7/L12 antigen captured at the capture site 4 and the labeling antibody labeled with gold colloids was captured.

[Table 6-3]

| * Detection of Escherichia coli in milk | | |
|---|---|---|
| | Example I-29 | Example I-30 |
| Structure | Figure 15 | Figure 19 |
| Bacterial conc. 1e4 cfu/mL w/ agitation | Positive | Positive |
| Bacterial conc. 1e4 cfu/mL w/o agitation | Negative | Positive |
| No bacteria w/ agitation | Negative | Negative |
| No bacteria w/o agitation | Negative | Negative |

When the labeling antibody-attached member 1 and the bacteriolysis agent-attached member 2 are positioned so that they are in contact or overlap when projected in the direction perpendicular to the chromatographic development direction, the detection performance can be maintained regardless of the agitation conditions during the test. Specifically, in order to obtain higher detection sensitivity, at least a portion of the labeling antibody-attached member 1 and at least a portion of the bacteriolysis agent-attached member 2 may preferably be arranged in close proximity. More specifically, when the side of the immersing region or the sample contact member of the chromatographic device is referred to as upstream and the side of the detection region is referred to as downstream, and the direction from upstream to downstream is referred to as the chromatographic development direction, the labeling antibody-attached member 1 and the bacteriolysis agent-attached member 2 may preferably be arranged such that their positions when projected in the direction perpendicular to the chromatographic development direction are in contact or overlap with each other (that is, the labeling antibody-attached member 1 and the bacteriolysis agent-attached member 2 contain the same position at least partially in the chromatographic development direction). The "overlap" arrangement herein is not limited to cases where the labeling antibody-attached member 1 and the bacteriolysis agent-attached member 2 are in contact with or overlap each other on the same side of the chromatographic system, but also includes cases where, for example, one of these members is placed on the back side of the chromatographic device so that they are separated from each other. This is presumably because the labeling antibody and the lysing agent are eluted from the labeling antibody-attached member 1 and the bacteriolysis agent-attached member 2, respectively, when they are in close proximity to each other in the ample liquid, whereby the lysis and antigen-antibody reactions occur more efficiently.

[Example Group II: Immunochromatographic device according to the second embodiment]

*Test 1: Detection of Staphylococcus aureus by immunochromatography

(1) Production of immunochromatographic devices for detecting Staphylococcus aureus in milk

An immunochromatographic device shown in the cross-sectional schematic view of Figure 22 was produced according to the following procedure.

(a) Production of ribosome protein L7/L12 antibodies

Monoclonal antibodies against Staphylococcus aureus ribosome protein L7/L12 for the detection of Staphylococcus aureus. According to the method described in Example 5 of WO2000/006603 A, Staphylococcus aureus L7/L12 ribosome protein was obtained, and monoclonal antibodies against the protein were produced. The monoclonal antibodies selected are a combination of two clones (SA-1 and SA-2) that can simultaneously bind to different sites of the L7/L12 ribosomal protein (i.e., that can form a sandwich-type complex with the L7/L12 antigen).

(b) Labeling antibody-attached members

Monoclonal antibody SA-2 was used as a labeling antibody, which forms a first complex with an antigen derived from the target bacterium based on an antigen-antibody reaction. 0.9 mL of gold colloid solution (60 nm particle size) manufactured by BBI International was mixed with 0.1 M potassium phosphate (pH 7.5), and then combined with 0.1 mL of 100 $\mu$g/mL monoclonal antibody SA-2 to be labeled with gold colloids and allowed to stand for 5 minutes at room temperature, to allow this antibody to bind to the surface of the gold colloidal particles. A 10% aqueous solution of bovine serum albumin (BSA) was then added so that the final concentration in the gold colloid solution was 1% to block the remaining surface of the gold colloidal particles with BSA, whereby a solution of gold colloid-labeled monoclonal antibody SA-2 (hereafter referred to as the "labeling antibody") was prepared. This solution was centrifuged (at 15000×rpm for 5 minutes) to precipitate the labeling antibody, and the supernatant solution was removed to obtain the labeling antibody. This labeling antibody was then suspended in 20mM tris hydrochloric acid buffer solution (pH 8.2) containing 0.25 % BSA and 2.5 % sucrose such that the absorbance at 540 nm wavelength becomes 0.5 when diluted 21 times, to thereby obtain a labeling antibody solution. A strip of glass fiber pad of 5 mm × 300 mm was impregnated with 1mL of the labeling antibody solution and dried under reduced pressure at room temperature to prepare the labeling antibody-attached member 1.

(c) Bacteriolysis agent-attached members

Recombinant lysostaphin from FujiFilm Wako Pure Chemicals was dissolved into 20 mM sodium acetate buffer solution (pH4.5) to a concentration of 100 $\mu$g/mL. A 5 mm × 300 mm strip of glass fiber pad was impregnated with 1 mL of the enzyme solution and air-dried at room temperature to prepare a bacteriolysis agent-attached member 2.

(d) Immobilization of capture antibodies in detection regions

A nitrocellulose membrane with a width of 25 mm and a length of 300 mm was prepared as the membrane carrier for chromatographic development 3. Monoclonal antibody SA-1 was used as a capture antibody, which forms a second complex with the first complex based on an antigen-antibody reaction. A aqueous solution containing 3.0 mg/mL of monoclonal antibody SA-1, 25 mM sodium phosphate, 1% sucrose, and 0.15% casein, pH6.2, was applied at a position 10 mm from the end of the chromatographic development start point on the membrane carrier for chromatographic development 3 in a line with a concentration of 1$\mu$L/cm, and dried under conditions shown in Table 7, to thereby form the capture site 4 for the complex formed of the Staphylococcus aureus ribosome protein L7/L12 antigen and the gold colloid-labeled antibody.

(e) Assembly of immunochromatographic devices

In addition to the labeling antibody-attached member 1, the bacteriolysis agent-attached member 2, and the membrane carrier for chromatographic development 3, a 25-mm GF/DVA (a filter from Cytiva: made of glass fiber, 776 $\mu$m in thickness, retention particle size = 3.5 $\mu$m) was used as a sample contact member 5 and a 15-mm GF/AVA (a filter from Cytiva: made of glass fiber, 299 $\mu$m in thickness, retention particle size = 1.7 $\mu$m) was used as a filter member 6, and filter paper was used as an absorption member 7. These members were adhered to a base material 8 (254 $\mu$m

in thickness, made of polystyrene, with adhesive material for attaching members applied on its surface), and a cover film (water-impermeable material) was used as a cover member 11 to cover the upper side of the figure (the side opposite the base material 8) except for the immersing region 9. This was cut into pieces of 5 mm in width, whereby the immunochromatographic device shown in the cross-sectional view of Figure 22 was produced. A 10-mm portion of each immunochromatographic device on the upstream part was used as the immersing region 9.

(f) Accelerated heating test

The immunochromatographic devices were divided into Group A and Group B. Group A was kept under low humidity at room temperature for one week. Group B was kept under 50°C for one week after being aluminum-pouched with desiccants in order to determine the change in performance during storage at room temperature. According to the present inventors' study, one week of storage at 50°C under these conditions is equivalent to approximately three to six months at room temperature.

(g) Preparation of reaction solutions

0.16M MOPSO buffer solution (pH7.4) was mixed with 1.6% Tween20 and 0.16% Zwittergent 3-12 to prepare reaction solution for immunochromatography.

(h) Sample liquid retention containers

Microtubes with a 2-mL capacity made by Eppendorf were used as sample liquid retention containers 20.

(2) Detection of Staphylococcus aureus in milk by immunochromatography

Sample solutions, i.e., commercial milk combined with normal saline with Staphylococcus aureus (ATCC25923 strain) at final concentrations of $2\times10^3$ cfu/mL, $4\times10^3$ cfu/mL, or commercial milk combined with normal saline, were each dispensed into microtubes 300μL each. The dispensed solution was then combined with 300μL of the reaction solution, and mixed at room temperature. Each of the immunochromatographic devices divided into two groups in the accelerated heating test of section (f) above was immersed in the mixed solution (sample liquid) from the sample contact member 5 such that the immersing region is completely immersed in the sample liquid. The immunochromatographic device of Figure 22 in the state of being used is shown in Figure 23.

The sample liquid was then agitated with the immunochromatographic device, allowed to stand at room temperature for 60 minutes to cause development, and then visually determined whether the complex of ribosomal protein L7/L12 antigen captured at the capture site 4 and the labeling antibody labeled with gold colloids was captured.

Table 7

| | Example II-1 | Example II-2 | Reference Example II-1 | Reference Example II-2 |
|---|---|---|---|---|
| Drying temp. (°C) | 60 | | 50 | |
| Drying time (hours) | 17 | | 0.5 | |
| Accelerated heating test | Group A (room temp.) | Group B (w/ heating) | Group A (room temp.) | Group B (w/ heating) |
| Bacterial conc. 4e3 cfu/mL | Positive | Positive | Positive | Positive |
| Bacterial conc. 2e3 cfu/mL | Positive | Positive | Negative | Positive |
| No bacteria | Negative | Negative | Negative | Negative |

With the immunochromatographic devices produced by applying the capture antibody to the membrane carrier for chromatographic development 3 followed by drying at 60°C for 17 hours, the bacteria were successfully detected even at a concentration of as low as 2e3 cfu/mL regardless of the presence or absence of the accelerated heating test. On the other hand, with the immunochromatographic devices produced by applying the capture antibody to the membrane carrier for chromatographic development 3 followed by drying at 50°C for 0.5 hour, the bacteria were successfully detected only at the concentration of 4e3 cfu/mL at the lowest in the absence of the accelerated heating test. Based on these results, it is expected that under the condition of drying at 50°C for 0.5 hours, the detection performance would increase by a factor of 2 after long-term storage at room temperature after production.

*Test 2: Detection of Escherichia coli in milk by immunochromatography

(1) Production of immunochromatographic devices for detecting Escherichia coli in milk

An immunochromatographic device shown in the cross-sectional schematic view of Figure 22 was produced according to the following procedure.

(a) Production of ribosome protein L7/L12 antibodies

Monoclonal antibodies against Escherichia coli ribosome protein L7/L12 for the detection of Escherichia coli. According to the method described in Example 5 of WO2000/006603 A, Escherichia coli L7/L12 ribosome protein was obtained, and monoclonal antibodies against the protein were produced. The monoclonal antibodies selected are a combination of two clones (EC-1 and EC-2) that can simultaneously bind to different sites of the L7/L12 ribosomal protein.

(b) Labeling antibody-attached members

Monoclonal antibody EC-2 was used as a labeling antibody, which forms a first complex with an antigen derived from the target bacterium based on an antigen-antibody reaction. 0.9 mL of gold colloid solution (60 nm particle size) manufactured by BBI International was mixed with 0.1 M potassium phosphate (pH 7.5), and then combined with 0.1 mL of 100 $\mu$g/mL monoclonal antibody EC-2 to be labeled with gold colloids and allowed to stand for 5 minutes at room temperature, to allow this antibody to bind to the surface of the gold colloidal particles. A 10% aqueous solution of bovine serum albumin (BSA) was then added so that the final concentration in the gold colloid solution was 1% to block the remaining surface of the gold colloidal particles with BSA, whereby a solution of gold colloid-labeled monoclonal antibody EC-2 (hereafter referred to as the "labeling antibody") was prepared. This solution was centrifuged (at 15000×rpm for 5 minutes) to precipitate the labeling antibody, and the supernatant solution was removed to obtain the labeling antibody. This labeling antibody was then suspended in 20mM tris hydrochloric acid buffer solution (pH 8.2) containing 0.25 % BSA and 2.5 % sucrose such that the absorbance at 540 nm wavelength becomes 0.5 when diluted 21 times, to thereby obtain a labeling antibody solution. A strip of glass fiber pad of 5 mm × 300 mm was impregnated with 1mL of the labeling antibody solution and dried under reduced pressure at room temperature to prepare the labeling antibody-attached member 1.

(c) Bacteriolysis agent-attached members

Egg white-derived lysozyme from Fujifilm Wako Pure Chemicals was dissolved in 20 mM Tris hydrochloric acid buffer solution (pH8.0) to a concentration of 10 mg/mL. A 5 mm × 300 mm strip of glass fiber pad was impregnated with 1 mL of the enzyme solution and air-dried at room temperature to prepare a bacteriolysis agent-attached member 2.

(d) Immobilization of capture antibodies in detection regions

A nitrocellulose membrane with a width of 25 mm and a length of 300 mm was prepared as the membrane carrier for chromatographic development 3. Monoclonal antibody EC-1 was used as a capture antibody, which forms a second complex with the first complex based on an antigen-antibody reaction. An aqueous solution containing 1.0 mg/mL of monoclonal antibody EC-1, 10 mM CAPSO, 2% sucrose, and 0.1% casein, pH 10.5, was applied at a position 10 mm from the end of the chromatographic development start point on the membrane carrier for chromatographic development 3 in a line with a concentration of 1$\mu$L/cm, and dried under conditions shown in Table 8, to thereby form the capture site 4 for the complex formed of the Escherichia coli ribosome protein L7/L12 antigen and the gold colloid-labeled antibody.

(e) Assembly of immunochromatographic devices

In addition to the labeling antibody-attached member 1, the bacteriolysis agent-attached member 2, and the membrane carrier for chromatographic development 3, a 25-mm GF/DVA (a filter from Cytiva: made of glass fiber, 776 $\mu$m in thickness, retention particle size = 3.5 $\mu$m) was used as a sample contact member 5 and a 15-mm GF/AVA (a filter from Cytiva: made of glass fiber, 299 $\mu$m in thickness, retention particle size = 1.7 $\mu$m) was used as a filter member 6, and filter paper was used as an absorption member 7. These members were adhered to a base material 8 (254 $\mu$m

in thickness, made of polystyrene, with adhesive material for attaching members applied on its surface), and a cover film (water-impermeable material) was used as a cover member 11 to cover the upper side of the figure (the side opposite the base material 8) except for the immersing region 9. This was cut into pieces of 5 mm in width to thereby produce the immunochromatographic device shown in the cross-sectional view of Figure 22. A 10-mm portion of each immunochromatographic device on the upstream part was used as the immersing region 9.

(f) Accelerated heating test

The immunochromatographic devices were divided into Group A and Group B. Group A was kept under low humidity at room temperature for one week. Group B was kept under 50°C for one week after being aluminum-pouched with desiccants in order to determine the change in performance during storage at room temperature.

(g) Preparation of reaction solutions

0.08M MOPSO buffer solution (pH7.4) was mixed with 1.8% TritonX100, 0.48% sodium N-dodecanoylsarcosinate, and 0.32M sodium chloride to prepare reaction solution for immunochromatography.

(h) Sample liquid retention containers

Microtubes with a 2-mL capacity made by Eppendorf were used as sample liquid retention containers 20.

(2) Detection of Escherichia coli in milk by immunochromatography

Sample solutions, i.e., commercial milk combined with normal saline with Escherichia coli (ATCC25922 strain) at final concentrations of $5 \times 10^4$ cfu/mL or $1 \times 10^5$ cfu/mL, or milk combined with normal saline, were each dispensed into microtubes 300μL each. The dispensed solution was then combined with 300μL of the reaction solution, and mixed at room temperature. Each of the immunochromatographic devices divided into two groups in the accelerated heating test of section (f) above was immersed in the mixed solution (sample liquid) from the sample contact member 5 such that the immersing region is completely immersed in the sample liquid. The sample liquid was then agitated with the immunochromatographic device, allowed to stand at room temperature for 60 minutes to cause development, and then visually determined whether the complex of ribosomal protein L7/L12 antigen captured at the capture site 4.

[Table 8]

|  | Example II-3 | Example II-4 | Reference Example II-3 | Reference Example II-4 |
|---|---|---|---|---|
| Drying temp. (°C) | 60 | | 50 | |
| Drying time (hours) | 17 | | 0.5 | |
| Accelerated heating test | Group A (room temp.) | Group B (w/ heating) | Group A (room temp.) | Group B (w/ heating) |
| Bacterial conc. 1e5 cfu/mL | Positive | Positive | Positive | Positive |
| Bacterial conc. 5e4 cfu/mL | Positive | Positive | Negative | Positive |
| No bacteria | Negative | Negative | Negative | Negative |

With the immunochromatographic devices produced by applying the capture antibody to the membrane carrier for chromatographic development 3 followed by drying at 60°C for 17 hours, the bacteria were successfully detected even at a concentration of as low as 5e4 cfu/mL regardless of the presence or absence of the accelerated heating test. On the other hand, with the immunochromatographic devices produced by applying the capture antibody to the membrane carrier for chromatographic development 3 followed by drying at 50°C for 0.5 hour, the bacteria were successfully detected only at the concentration of 1e5 cfu/mL at the lowest in the absence of the accelerated heating test. Based on these results, it is expected that under the condition of drying at 50°C for 0.5 hours, the detection performance would increase by a factor of 2 after long-term storage at room temperature after production.

*Test 3: Detection of Streptococcus uberis in milk by immunochromatography

(1) Production of immunochromatographic devices for detecting Streptococcus uberis in milk

An immunochromatographic device shown in the cross-sectional schematic view of Figure 22 was produced according to the following procedure.

(a) Production of ribosome protein L7/L12 antibodies

Monoclonal antibodies against Streptococcus uberis ribosome protein L7/L12 for the detection of Streptococcus uberis. According to the method described in Example 5 of WO2000/006603 A, Streptococcus uberis L7/L12 ribosome protein was obtained, and monoclonal antibodies against the protein were produced. The monoclonal antibodies selected are a combination of two clones (SU-1 and SU-2) that can simultaneously bind to different sites of the L7/L12 ribosomal protein.

(b) Labeling antibody-attached members

Monoclonal antibody SU-2 was used as a labeling antibody, which forms a first complex with an antigen derived from the target bacterium based on an antigen-antibody reaction. 0.9 mL of gold colloid solution (60 nm particle size) manufactured by BBI International was mixed with 0.1 M potassium phosphate (pH 7.5), and then combined with 0.1 mL of 100 $\mu$g/mL monoclonal antibody SU-2 to be labeled with gold colloids and allowed to stand for 5 minutes at room temperature, to allow this antibody to bind to the surface of the gold colloidal particles. 10 mass % aqueous solution of bovine serum albumin (BSA) was then added such that the final concentration in the gold colloid solution was 1 mass %, to block the remaining surface of the gold colloidal particles with BSA, whereby a solution of gold colloid-labeled monoclonal antibody SU-2 (hereafter referred to as the "labeling antibody") was prepared. This solution was centrifuged (at 15000×rpm for 5 minutes) to precipitate the labeling antibody, and the supernatant solution was removed to obtain the labeling antibody. This labeling antibody was dissolved into 20 mM Tris hydrochloric acid buffer solution (pH8.2) containing 0.25 mass %BSA and 2.5 mass % sucrose such that the absorbance at 540 nm wavelength becomes 0.5 when diluted 21 times, to thereby obtain a labeling antibody solution. A strip of glass fiber pad of 5 mm × 300 mm was impregnated with 1mL of the labeling antibody solution and dried under reduced pressure at room temperature to prepare the labeling antibody-attached member 1.

(c) Bacteriolysis agent-attached members

Labiase from Ozeki Co., Ltd. and egg white-derived lysozyme from Fujifilm Wako Pure Chemicals were dissolved into citric acid -phosphoric acid buffer solution (pH4.0) to a concentration of 100 mg/mL. A 5 mm × 300 mm strip of glass fiber pad was impregnated with 1 mL of the enzyme solution and air-dried at room temperature to prepare a bacteriolysis agent-attached member 2.

(d) Immobilization of capture antibodies in detection regions

A nitrocellulose membrane with a width of 25 mm and a length of 300 mm was prepared as the membrane carrier for chromatographic development 3. Monoclonal antibody SU-1 was used as a capture antibody, which forms a second complex with the first complex based on an antigen-antibody reaction. An aqueous solution containing 1.0 mg/mL of monoclonal antibody SU-1, 10 mM potassium phosphate, and 1 mass % sucrose, pH 8, was applied at a position 10 mm from the end of the chromatographic development start point on the membrane carrier for chromatographic development 3 in a line with a concentration of 1$\mu$L/cm, and dried under conditions shown in Table 9, to thereby form the capture site 4 for the complex formed of the Escherichia coli ribosome protein L7/L12 antigen and the gold colloid-labeled antibody.

(e) Assembly of immunochromatographic devices

In addition to the labeling antibody-attached member 1, the bacteriolysis agent-attached member 2, and the membrane carrier for chromatographic development 3, a 25-mm GF/DVA (a filter from Cytiva: made of glass fiber, 776 $\mu$m in thickness, retention particle size = 3.5 $\mu$m) was used as a sample contact member 5 and a 15-mm GF/AVA (a filter from Cytiva: made of glass fiber, 299 $\mu$m in thickness, retention particle size = 1.7 $\mu$m) was used as a filter member 6, and filter paper was used as an absorption member 7. These members were adhered to a base material 8 (254 $\mu$m in thickness, made of polystyrene, with adhesive material for attaching members applied on its surface), and a cover film (water-impermeable material) was used as a cover member 11 to cover the upper side of the figure (the side opposite the base material 8) except for the immersing region 9. This was cut into pieces of 5 mm in width to thereby produce the immunochromatographic device in the cross-sectional view of Figure 22. A 10-mm portion of each immunochromatographic device on the upstream part was used as the immersing region 9.

(f) Accelerated heating stability test

The immunochromatographic devices were divided into Group A and Group B. Group A was kept under low humidity at room temperature for one week. Group B was kept under 50°C for one week after being aluminum-pouched with desiccants in order to determine the change in performance during storage at room temperature.

(g) Preparation of reaction solutions

0.16M MOPSO buffer solution (pH6.3) was mixed with 1.8 mass % of TritonX100, 1.2 mass % of Tween20, 0.48 mass % of sodium N-dodecanoylsarcosinate, 0.12M sodium chloride, and 0.001M EGTA to prepare reaction solution for immunochromatography.

(h) Sample liquid retention containers

Microtubes with a 2-mL capacity made by Eppendorf were used as sample liquid retention containers 20.

(2) Detection of Streptococcus uberis in milk by immunochromatography

Sample solutions, i.e., commercial milk combined with normal saline with Streptococcus uberis (ATCC19436 strain) at final concentrations of $2\times10^4$ cfu/mL or $4\times10^4$ cfu/mL, or commercial milk combined with normal saline, were each dispensed into microtubes 300μL each. The dispensed solution was then combined with 300μL of the reaction solution, and mixed at room temperature.

Each of the immunochromatographic devices divided into two groups in the accelerated heating test of section (f) above was immersed in the mixed solution (sample liquid) from the sample contact member 5 such that the immersing region is completely immersed in the sample liquid. The sample liquid was then agitated with the immunochromatographic device, allowed to stand at room temperature for 60 minutes to cause development, and then visually determined whether the complex of ribosomal protein L7/L12 antigen captured at the capture site 4.

[Table 9]

| | Example II-5 | Example II-6 | Reference Example II-5 | Reference Example II-6 |
|---|---|---|---|---|
| Drying temp. (°C) | 60 | | 50 | |
| Drying time (hours) | 17 | | 0.5 | |
| Accelerated heating test | Group A (room temp.) | Group B (w/ heating) | Group A (room temp.) | Group B (w/ heating) |
| Bacterial conc. 4e4 cfu/mL | Positive | Positive | Positive | Positive |
| Bacterial conc. 2e4 cfu/mL | Positive | Positive | Negative | Positive |
| No bacteria | Negative | Negative | Negative | Negative |

With the immunochromatographic devices produced by applying the capture antibody to the membrane carrier for chromatographic development 3 followed by drying at 60°C for 17 hours, the bacteria were successfully detected even at a concentration of as low as 2e4 cfu/mL regardless of the presence or absence of the accelerated heating test. On the other hand, with the immunochromatographic devices produced by applying the capture antibody to the membrane carrier for chromatographic development 3 followed by drying at 50°C for 0.5 hour, the bacteria were successfully detected only at the concentration of 4e4 cfu/mL at the lowest in the absence of the accelerated heating test. Based on these results, it is expected that under the condition of drying at 50°C for 0.5 hours, the detection performance would increase by a factor of 2 after long-term storage at room temperature after production.

*Test 4: Comparison of drying conditions after application of capture antibodies

(1) Production of immunochromatographic devices for detecting Escherichia coli

An immunochromatographic device shown in the cross-sectional schematic view of Figure 22 was produced according to the following procedure.

(a) Production of ribosome protein L7/L12 antibodies

The combination of EC-1 and EC-2, which was selected in Test 2, was used.

(b) Labeling antibody-attached members

The labeling antibody-attached member 1 produced in Test 2 was used.

(c) Bacteriolysis agent-attached members

The bacteriolysis agent-attached member 2 produced in Test 2 was used.

(d) Immobilization of capture antibodies in detection regions

A nitrocellulose membrane with a width of 25 mm and a length of 300 mm was prepared as the membrane carrier for chromatographic development 3. Monoclonal antibody EC-1 was used as a capture antibody, which forms a second complex with the first complex based on an antigen-antibody reaction. An aqueous solution containing 1.0 mg/mL of monoclonal antibody EC-1, 10 mM CAPSO, 2 mass % sucrose, 0.1 mass % casein, pH10.5, was applied at a position 10 mm from the end of the chromatographic development start point on the membrane carrier for chromatographic development 3 in a line with a concentration of 1 $\mu$L/cm, and dried under conditions shown in Table 10, to thereby form the capture site 4 for the complex formed of the Escherichia coli ribosome protein L7/L12 antigen and the gold colloid-labeled antibody.

(e) Assembly of immunochromatographic devices

In addition to the labeling antibody-attached member 1, the bacteriolysis agent-attached member 2, and the membrane carrier for chromatographic development 3, a 25-mm GF/DVA (a filter from Cytiva: made of glass fiber, 776 $\mu$m in thickness, retention particle size = 3.5 $\mu$m) was used as a sample contact member 5 and a 15-mm GF/AVA (a filter from Cytiva: made of glass fiber, 299 $\mu$m in thickness, retention particle size = 1.7 $\mu$m) was used as a filter member 6, and filter paper was used as an absorption member 7. These members were adhered to a base material 8 (254 $\mu$m in thickness, made of polystyrene, with adhesive material for attaching members applied on its surface), and a cover film (water-impermeable material) was used as a cover member 11 to cover the upper side of the figure (the side opposite the base material 8) except for the immersing region 9. This was cut into pieces of 5 mm in width to thereby produce the immunochromatographic device shown in the cross-sectional view of Figure 22. A 10-mm portion of each immunochromatographic device on the upstream part was used as the immersing region 9.

(f) Storage of kits

The immunochromatographic devices were stored under low humidity at room temperature for one week.

(g) Preparation of reaction solutions

The reaction solutions produced in Test 2 were used.

(h) Sample liquid retention containers

Microtubes with a 2-mL capacity made by Eppendorf were used as sample liquid retention containers 20.

(2) Detection of Escherichia coli in milk by immunochromatography

Sample solutions, i.e., commercial milk combined with normal saline with Escherichia coli (ATCC25922 strain) at final concentrations of $5\times10^4$ cfu/mL, $1\times10^5$ cfu/mL or commercial milk combined with normal saline, were each dispensed into microtubes 300μL each. The dispensed solution was then combined with 300μL of the reaction solution, and mixed at room temperature. Each of the immunochromatographic devices was immersed in the mixed solution from the sample contact member 5 such that the immersing region is completely immersed in the sample liquid, and allowed to stand at room temperature for 60 minutes to cause development, and then visually determined whether the complex of ribosomal protein L7/L12 antigen captured at the capture site 4 and the labeling antibody labeled with gold colloids was captured.

[Table 10]

|  | Reference Example II-7 | Example II-7 | Example II-8 | Example II-9 | Example II-10 |
|---|---|---|---|---|---|
| Drying temp. (°C) | 60 | | | | |
| Drying time (hours) | 0.5 | 6 | 13 | 17 | 24 |
| Bacterial conc. 1e5 cfu/mL | Positive | Positive | Positive | Positive | Positive |
| Bacterial conc. 5e4 cfu/mL | Negative | Positive | Positive | Positive | Positive |
| No bacteria | Negative | Negative | Negative | Negative | Negative |

With the immunochromatographic devices produced by applying the capture antibody to the membrane carrier for chromatographic development 3 followed by drying at 60°C for 6 hours, the bacteria were successfully detected even at a concentration of as low as 5e4 cfu/mL even in the absence of the accelerated heating test, as was the case with Example II-3 of Test 2. On the other hand, with the immunochromatographic devices produced by applying the capture antibody to the membrane carrier for chromatographic development 3 followed by drying at 60°C for 0.5 hour, the bacteria were successfully detected only at the concentration of 1e5 cfu/mL at the lowest. Based on these results, it is expected that the condition of drying at 60°C for 6 hours or more can suppress the change in detection performance during long-term storage at room temperature after production.

[Example Group III: Immunochromatographic device according to the third embodiment]

An immunochromatographic device shown in the cross-sectional schematic view of Figure 25 was produced according to the following procedure.

(a) Production of ribosome protein L7/L12 antibodies

Monoclonal antibodies against Staphylococcus aureus ribosome protein L7/L12 were used for labeling with gold colloids. According to the method described in Example 5 of WO2000/006603 A, Staphylococcus aureus L7/L12 ribosome protein was obtained, and monoclonal antibodies against the protein were produced. The monoclonal antibodies selected are a combination of two clones (SA-1 and SA-2) that can simultaneously bind to different sites of the L7/L12 ribosomal protein.

(b) Labeling antibody-attached members

Monoclonal antibody SA-2 was used as a labeling antibody, which forms a first complex with an antigen derived from the target bacterium based on an antigen-antibody reaction. 0.9 mL of gold colloid solution (60 nm particle size) manufactured by BBI International was mixed with 0.1 M potassium phosphate (pH 7.5), and then combined with 100 μg/mL monoclonal antibody SA-2 for gold colloid labeling and allowed to stand at room temperature for 5 minutes, to allow this antibody to bind to the surface of the gold colloidal particles. A 10% aqueous solution of bovine serum albumin (BSA) was then added so that the final concentration in the gold colloid solution was 1% to block the remaining surface of the gold colloidal particles with BSA, whereby a solution of gold colloid-labeled monoclonal antibody SA-2 (hereafter referred to as the "labeling antibody") was prepared. This solution was centrifuged (at 15000×rpm for 5 minutes) to precipitate the labeling antibody, and the supernatant solution was removed to obtain the labeling antibody. This labeling antibody was then suspended in 20mm Tris hydrochloric acid buffer solution (pH8.2) containing 0.25%BSA and 2.5% sucrose such that the absorbance at 540 nm wavelength becomes 0.5 when diluted 21 times, to thereby obtain a labeling antibody solution, to thereby obtain a labeling antibody solution. A strip of glass fiber pad of 5 mm × 300 mm was impregnated with 1mL of the labeling antibody solution and dried under reduced pressure at room temperature to prepare the labeling antibody-attached member 1.

(c) Bacteriolysis agent-attached members

Recombinant lysostaphin from FujiFilm Wako Pure Chemicals was dissolved in 20 mM sodium acetate buffer solution (pH4.5) to a concentration of 100 μg/mL. A 5 mm × 300 mm strip of glass fiber pad was impregnated with 1 mL of the enzyme solution and air-dried at room temperature to prepare a bacteriolysis agent-attached member 2.

(d) Immobilization of capture antibodies in detection regions

A nitrocellulose membrane with a width of 25 mm and a length of 300 mm was used as a membrane member for chromatographic development 3 of chromatographic medium. Monoclonal antibody SA-1 was used as a capture antibody, which forms a second complex with the first complex based on an antigen-antibody reaction. A solution containing 1.5 mg/mL of monoclonal antibody SA-1 was applied at a position 10 mm from the end of the chromatographic development start point on the membrane carrier for chromatographic development 3 in a line with a concentration of 1μL/cm, and dried at 50°C for 30 minutes, to thereby form the capture site 4 for the complex formed of the Staphylococcus aureus ribosome protein L7/L12 antigen and the gold colloid-labeled antibody.

(e) Assembly of immunochromatographic devices

In addition to the labeling antibody-attached member 1 (length: 5mm), the bacteriolysis agent-attached member 2 (length: 5mm), and the membrane member for chromatographic development 3 (CN95 from Sartorius, length: 25mm), filter paper (17CHR from Cytiva, length: 25mm) was used as the absorption member 7, a GF/DVA (a filter from Cytiva: made of glass fiber, 776 μm in thickness, retention particle size: 3.5 μm) was used as the first filter member 6-1, and a GF/AVA (a filter from Cytiva: made of glass fiber, 299 μm in thickness, retention particle size: 1.7 μm) was used as the second filter member 6-2. These members were adhered onto AR Care 9020 (254 μm in thickness, made of polystyrene, with adhesive material for attaching members applied on its surface) as the base material 8, and cut into pieces of 5 mm in width. The lengths of the first filter and the second filter were varied as indicated in Table 11 below. The immunochromatographic devices of Examples III-1, III-5, and III-6 are schematically illustrated with dimensions (Figures 26 (A), (B), (C)).

[Table 11]

|  | Reference Example III-1 | Example III-1 | Example III-2 | Example III-3 | Example III-4 | Example III-5 | Example III-6 |
|---|---|---|---|---|---|---|---|
| First filter member 6-1 (GF/DVA) | 20mm | 20mm | 20mm | 20mm | 23mm | 26mm | 26mm |
| Second filter member 6-2 (GF/AVA) | 14mm | 15mm | 18mm | 21mm | 15mm | 15mm | 15mm |
| Overlap distance X | 2mm | 3mm | 6mm | 9mm | 6mm | 9mm | 9mm |
| Total amount of absorbed water Y | 0.08g | 0.08g | 0.08g | 0.08g | 0.08g | 0.08g | 0.08g |
| X/Y mm/g | 25 | 37.5 | 75 | 112.5 | 75 | 112.5 | 112.5 |
| Failure rate | 50% | 35% | 25% | 15% | 15% | 0% | 0% |

(f) Milk sample

Milk sample used was a 1:1 mixture of raw milk (not heat pasteurized or homogenized) collected from lactating Holstein cows and a 0.1 M MOPSO solution (pH 7.4).

(g) Sample liquid retention containers

Microtubes with a 2-mL capacity made by Eppendorf were used as sample liquid retention containers 20. 10 immunochromatographic devices were produced, and a chromatographic development experiment was conducted by dispensing 600 μL of milk sample into each sample holding container 20 to determine the percentage of poor chromatographic development. The overlap distance X between the first filter member and the second filter member was determined by measuring the length in the longitudinal direction of the area where the first and second filter members overlap when the immunochromatographic device is observed from the longitudinal side. The total water absorption Y (g) was measured as follows. A sample liquid was developed using the immunochromatographic device. After sufficient time had elapsed and the weight of the entire device had become stable, the filter member, the labeling enzyme-attached member, and the bacteriolysis agent-attached member were removed from the device, to leave only the base material, the membrane member for chromatographic development, and the absorption member. The weight of the remaining members was measured as W2 (g). Likewise, the filter member, the labeling enzyme-attached member, and the bacteriolysis agent-attached member were removed from the unused immunochromatographic device before the development of sample liquid, to leave only the base material, the membrane member for chromatographic development, and the absorption member. The weight of the remaining members was measured as W1 (g). The value calculated as W2 - W1 corresponds to the volume of chromatographically developed liquid, i.e., the total amount of water absorbed by the membrane member for chromatographic development and the absorption member, Y (g).

The chromatographic development failure rate was as high as 50% when X/Y was less than 30, but when X/Y was 30 or more, the failure rate was 40% or less; when X/Y was 70 or more, the failure rate was 25% or less; and when X/Y was 100 or more, the failure rate was 15% or less. Provided that X/Y was the same, the failure rate tended to be lower when the first filter member was longer. Examples III-5 and III-6 (X/Y = 112.5 mm/g) did not cause a single failure, with a failure rate of 0%.

**EXPLANATION OF SYMBOLS**

| | |
|---|---|
| 1 | Labeling antibody-attached member |
| 2, 2-1, 2-2 | Bacteriolysis agent-attached member |
| 3 | Membrane carrier for chromatographic development |
| 4 | Capture site/detection region |
| 5 | Sample contact member |
| 6, 6-1, 6-2 | Filter member |
| 7 | Absorption member |
| 8 | Base material |
| 9 | Immersing region |
| 10 | Immunochromatographic device |
| 11 | Cover member |
| 20 | Sample fluid retention container/milk sample retention container |
| 100 | Overlap between the first filter member and the second filter member |

**Claims**

1. A method for detecting a target bacterium in a sample liquid based on an antigen-antibody reaction using an immunochromatographic device, comprising the steps of:

   1) immersing at least a portion of a labeling antibody-attached member, to which a labeling antibody is attached, in the sample liquid to thereby cause at least a portion of an antigen-antibody reaction between an antigen derived from the target bacterium and the labeling antibody in the sample liquid to form a first complex; and
   2) causing an antigen-antibody reaction between a capture antibody immobilized on the immunochromatographic device and the first complex to form a second complex, thereby detecting the bacterium based on the label of the labeling antibody.

2. The method according to claim 1, wherein together with the at least portion of the labeling antibody-attached member, at least a portion of a bacteriolysis agent-attached member, to which a bacteriolysis agent is attached, is immersed in the sample liquid.

3. The method according to claim 2, wherein the at least portion of the labeling antibody-attached member and the at least portion of the bacteriolysis agent-attached member are immersed in the sample liquid at the same time.

4. The method according to any one of claims 1 to 3, further comprising agitating the sample liquid after the immersion and before the detection.

5. The method according to any one of claims 1 to 4, wherein the antigen derived from the target bacterium is an intracellular antigen.

6. The method according to claim 5, wherein the intracellular antigen is a ribosome protein.

7. The method according to claim 6, wherein the ribosome protein is a L7/L12 protein.

8. The method according to any one of claims 1 to 7, wherein after the immersion, at least a portion of the labeling antibody attached to the labeling antibody-attached member and the bacteriolysis agent attached to the bacteriolysis agent-attached member dissolves in the sample liquid.

9. An immunochromatographic device for detecting a target bacterium in a sample liquid based on an antigen-antibody reaction detection, comprising:

   (a) a labeling antibody-attached member, to which is attached a labeling antibody, which forms a first complex with an antigen derived from the target bacterium based on an antigen-antibody reaction; and
   (b) a chromatographic development member, which contains an immersing region, which is to be immersed in the sample liquid, and a detection region, on which is immobilized a capture antibody, which forms a second complex with the first complex based on an antigen-antibody reaction,

      wherein the immunochromatographic device is configured such that when it is used, the at least portion of the labeling antibody-attached member and the immersing region of the chromatographic development member are both immersed in the sample liquid, and
      wherein the detection of the target bacterium in the sample liquid by the immunochromatographic device is carried out by detecting the second complex captured in the detection region of the chromatographic development member based on the label of the labeling antibody detection.

10. The immunochromatographic device according to claim 9, which is configured such that the at least portion of the labeling antibody-attached member is included in the immersing region.

11. The immunochromatographic device according to claim 9 or 10, further comprising:
    (c) a bacteriolysis agent-attached member, to which a bacteriolysis agent is attached,
    wherein the immunochromatographic device is configured such that when it is used, at least a portion of the bacteriolysis agent-attached member is immersed in the sample liquid.

12. The immunochromatographic device according to claim 11, wherein the bacteriolysis agent-attached member and the labeling antibody-attached member are formed as separate members.

13. The immunochromatographic device according to claim 11, wherein the bacteriolysis agent-attached member and the labeling antibody-attached member are formed as a single member.

14. The immunochromatographic device according to any one of claims 11 to 13, which is configured such that at least a portion of the bacteriolysis agent-attached member is included in the immersing region.

15. The immunochromatographic device according to any one of claims 11 to 14, wherein either or both of the bacteriolysis agent-attached member and the labeling antibody-attached member are provided separately from the chromatographic development member.

16. The immunochromatographic device according to any one of claims 11 to 15, further comprising:
    (d) a sample contact member,
    wherein the immunochromatographic device is configured such that when it is used, at least a portion of the sample contact member is immersed in the sample liquid.

# Fig. 1

# Fig. 2

# Fig. 3

# Fig. 4

# Fig. 5

# Fig. 6

Fig. 7

Fig. 8

Fig. 9

Fig.10

Fig.11

Fig.12

# Fig. 13

# Fig. 14

# Fig. 15

Fig. 16

Fig. 17

Fig. 18

Fig. 19

# Fig. 20

Sample liquid level

Kit of Figure 1
being used for
detection

# Fig. 21

10

20

Sample liquid level

9

Kit of Figure 2
being used for
detection

Fig. 22

# Fig. 23

10

20

9

Sample liquid level

Fig. 24

Fig. 25A

Fig. 25B

Fig. 26A

Fig. 26B

Fig. 26C

# Fig. 27

## INTERNATIONAL SEARCH REPORT

| | International application No. |
|---|---|
| | **PCT/JP2022/043411** |

**A. CLASSIFICATION OF SUBJECT MATTER**

*G01N 33/543*(2006.01)i; *G01N 33/569*(2006.01)i
FI: G01N33/543 521; G01N33/569 B

According to International Patent Classification (IPC) or to both national classification and IPC

**B. FIELDS SEARCHED**

Minimum documentation searched (classification system followed by classification symbols)

G01N33/543; G01N33/569

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 3436269 B2 (WAKO PURE CHEMICAL INDUSTRIES, LTD) 11 August 2003 (2003-08-11)<br>claims, p. 3, left column, line 37 to right column, line 41, p. 7, left column, lines 6-14, p. 7, right column, lines 25-30, p. 9, left column, lines 20-40 | 1, 4-7, 9-10 |
| A | JP 2005-214670 A (DENKA SEIKEN CO LTD) 11 August 2005 (2005-08-11)<br>entire text, all drawings | 1-16 |
| A | JP 2002-181815 A (GODO SHUSEI CO LTD) 26 June 2002 (2002-06-26)<br>entire text, all drawings | 1-16 |
| A | JP 2007-046959 A (BL KK) 22 February 2007 (2007-02-22)<br>entire text, all drawings | 1-16 |
| A | 大島光宏　ほか, 唾液潜血試験におけるモノクローナル抗体を用いたヘモグロビン検出試験紙の有用性—ペルオキシダーゼ試験紙, 日本歯周病学会会誌, 28 June 1997<br>entire text, all drawings, (OHSHIMA, Mitsuhiro et al. An Immunological Test Using Anti-human Hemoglobin Monoclonal Antibody for Detection of Occult Blood in Saliva. Nihon Shishubyo Gakkai Kaishi.) | 1-16 |

☑ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **22 December 2022** | **17 January 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2022/043411**

C. DOCUMENTS CONSIDERED TO BE RELEVANT

| Category* | Citation of document, with indication, where appropriate, of the relevant passages | Relevant to claim No. |
|---|---|---|
| A | WO 2017/213227 A1 (DENKA SEIKEN CO LTD) 14 December 2017 (2017-12-14) entire text, all drawings | 1-16 |
| A | WO 2020/111223 A1 (ASAHI CHEMICAL IND) 04 June 2020 (2020-06-04) entire text, all drawings | 1-16 |

Form PCT/ISA/210 (second sheet) (January 2015)

**INTERNATIONAL SEARCH REPORT**
Information on patent family members

International application No.

**PCT/JP2022/043411**

| Patent document cited in search report | | | Publication date (day/month/year) | Patent family member(s) | | | Publication date (day/month/year) |
|---|---|---|---|---|---|---|---|
| JP | 3436269 | B2 | 11 August 2003 | (Family: none) | | | |
| JP | 2005-214670 | A | 11 August 2005 | US entire text, all drawings EP | 2008/0193953 1712913 | A1 A1 | |
| JP | 2002-181815 | A | 26 June 2002 | (Family: none) | | | |
| JP | 2007-046959 | A | 22 February 2007 | (Family: none) | | | |
| WO | 2017/213227 | A1 | 14 December 2017 | US entire text, all drawings EP CN KR | 2019/0145864 3470841 109313185 10-2019-0017826 | A1 A1 A A | |
| WO | 2020/111223 | A1 | 04 June 2020 | US entire text, all drawings EP | 2022/0042990 3889609 | A1 A1 | |

Form PCT/ISA/210 (patent family annex) (January 2015)

**REFERENCES CITED IN THE DESCRIPTION**

**Patent documents cited in the description**

- JP 6325895 B
- JP 6280567 B
- JP 6314156 B
- JP 6370807 B
- JP 6081457 B
- JP 5693938 B
- JP 63014156 B
- WO 2000006603 A